# EUROPEAN PATENT APPLICATION

(11) **EP 4 517 642 A1**
(43) Date of publication of application: **05.03.2025**
(21) Application number: 22940306.8
(22) Date of filing: 05.08.2022
(51) Int. Cl.: G06Q 50/22

(54) **INFORMATION PROCESSING SYSTEM, INFORMATION PROCESSING DEVICE, AND INFORMATION PROCESSING METHOD**

(30) Priority: 25.04.2022 JP 2022071410
(71) Applicant: PARAMOUNT BED CO., LTD., Tokyo 136-8670 (JP)
(72) Inventor: ISHIKAWA, Takashi, Tokyo 136-8670 (JP); NAKAMURA, Yuuki, Tokyo 136-8670 (JP)
(74) Representative: Novagraaf International SA
(86) International application number: PCT/JP2022/030137
(87) International publication number: WO 2023/210035

(57) **Abstract**

An information processing system includes: a device including an application that executes processing corresponding to tacit knowledge of a skilled worker; and a server system that transmits a data frame including a media access control (MAC) header, a frame body, and a trailer, in a data link layer of communication with the device, in which the server system transmits the data frame in which the frame body includes a fixed-length region including a first region that stores ability information, a second region that stores scene information, and a third region that stores device type information, and the device determines whether the application is active or inactive based on at least one of the ability information, the scene information, and the device type information.

## Description

### Technical Field

The present invention relates to an information processing system, an information processing apparatus, an information processing method, and the like. This application claims priority from Japanese Patent Application No. 2022-071410, filed on April 25, 2022, the contents of which are incorporated herein by reference.

### Background Art

Systems have been known that are used in scenes where care givers provide care assistance to care receivers. Patent Literature 1 discloses a method of disposing a sensor in a living space and generating provision information on a state of an inhabitant, living in the living space, based on time variation in detection information acquired by the sensor.

### Citation List

### Patent Literature

PTL 1: JP2021-18760A

### Summary of Invention

### Technical Problem

The present invention provides an information processing system, an information processing apparatus, an information processing method, and the like that appropriately support care assistance provided to a care receiver by a care giver.

### Solution to Problem

One aspect of this disclosure relates to an information processing system that includes: a device including an application that executes processing corresponding to tacit knowledge of a skilled worker; and a server system that transmits a data frame including a media access control (MAC) header, a frame body, and a trailer, in a data link layer of communication with the device, in which the server system transmits the data frame in which the frame body includes a fixed-length region including a first region that stores ability information indicating an activity ability of a care receiver, a second region that stores scene information specifying a care assistance scene with respect to the care receiver, and a third region that stores device type information specifying a type of a combined device to be used with the device, and the device determines whether the application is active or inactive based on at least one of the ability information, the scene information, and the device type information.

Another aspect of this disclosure relates to an information processing apparatus that includes: a communicator that transmits a data frame including a media access control (MAC) header, a frame body, and a trailer, in a data link layer of communication with a device including an application that executes processing corresponding to tacit knowledge of a skilled worker; and a communication processing unit that controls the communicator, in which the communicator transmits the data frame in which the frame body includes a fixed-length region including a first region that stores ability information indicating an activity ability of a care receiver, a second region that stores scene information specifying a care assistance scene with respect to the care receiver, and a third region that stores device type information specifying a type of a combined device to be used with the device, as information for determining whether the application is active or inactive, to the device.

Still another aspect of this disclosure relates to an information processing method in an information processing system that includes a device including an application that executes processing corresponding to tacit knowledge of a skilled worker and a server system that transmits a data frame including a media access control (MAC) header, a frame body, and a trailer, in a data link layer of communication with the device, the information processing method includes: transmitting the data frame in which the frame body includes a fixed-length region including a first region that stores ability information indicating an activity ability of a care receiver, a second region that stores scene information specifying a care assistance scene with respect to the care receiver, and a third region that stores device type information specifying a type of a combined device to be used with the device, from the server system to the device; and determining whether the application is active or inactive based on at least one of the ability information, the scene information, and the device type information.

### Brief Description of Drawings

[FIG. 1] FIG. 1 is a diagram illustrating a configuration example of an information processing system.
[FIG. 2] FIG. 2 is a diagram illustrating a relation example among devices and tacit knowledge.
[FIG. 3] FIG. 3 is a diagram illustrating a configuration example of a server system.
[FIG. 4] FIG. 4 is a diagram illustrating a configuration example of a device.
[FIG. 5] FIG. 5 is a diagram illustrating a relation example of an ability of a care receiver and assumed risks.
[FIG. 6] FIG. 6 is a sequence diagram illustrating processing in the information processing system.
[FIG. 7] FIG. 7 is a sequence diagram illustrating processing in the information processing system.
[FIG. 8] FIG. 8 is a diagram illustrating a specific example of a device related to a falling-down risk.
[FIG. 9] FIG. 9 is a diagram illustrating a specific example of a device related to a falling-down risk.
[FIG. 10] FIG. 10 is a diagram illustrating a specific example of a device related to a fall risk.
[FIG. 11] FIG. 11 is a diagram illustrating a specific example of a device related to a fall risk.
[FIG. 12] FIG. 12 is a diagram illustrating a specific example of a device related to an aspiration risk.
[FIG. 13] FIG. 13 is a diagram illustrating a specific example of a device related to a bedsore risk.
[FIG. 14] FIG. 14 is a diagram illustrating a specific example of a device related to a bedsore risk.
[FIG. 15A] FIG. 15A illustrates a screen example that is used for bed position adjustment.
[FIG. 15B] FIG. 15B illustrates a screen example that is used for bed position adjustment.
[FIG. 16] FIG. 16 illustrates a screen example that is used for end-of-life care.
[FIG. 17] FIG. 17 is a diagram illustrating an example of operation modes for a device in accordance with abilities.
[FIG. 18] FIG. 18 is a sequence diagram illustrating processing in the information processing system.
[FIG. 19] FIG. 19 is a flowchart illustrating determination processing of an operation mode in the device.
[FIG. 20] FIG. 20 is a flowchart illustrating determination processing of an operation mode in the device.
[FIG. 21] FIG. 21 is a diagram illustrating a cooperation example between a swallowing and choking detection device and another device.
[FIG. 22] FIG. 22 is a flowchart illustrating determination processing of an operation mode in the device.
[FIG. 23] FIG. 23 is a diagram illustrating a specific example of a control target device.
[FIG. 24] FIG. 24 is a diagram illustrating a specific example of a control target device.
[FIG. 25] FIG. 25 is a diagram illustrating a configuration example of the information processing system in a case where serverless communication is performed.
[FIG. 26] FIG. 26 is a diagram illustrating a configuration example of a communication processing unit and a communicator.
[FIG. 27] FIG. 27 is a diagram illustrating a configuration example of a MAC frame.
[FIG. 28A] FIG. 28A is a diagram illustrating a configuration example of a frame body.
[FIG. 28B] FIG. 28B is a diagram illustrating a relation example of data type IDs and contents.
[FIG. 29A] FIG. 29A is a diagram illustrating a configuration example of a frame body.
[FIG. 29B] FIG. 29B is a diagram illustrating a relation example of data type IDs and contents.
[FIG. 30] FIG. 30 is a diagram illustrating an example of parameters in accordance with access categories.
[FIG. 31] FIG. 31 is a diagram illustrating an allocation example of the access category.
[FIG. 32] FIG. 32 is a diagram illustrating a configuration example of the information processing system in at-home care.
[FIG. 33] FIG. 33 illustrates an example of a screen to be displayed on a terminal device of a care giver.
[FIG. 34] FIG. 34 illustrates an example of a screen to be displayed on the terminal device of the care giver.
[FIG. 35] FIG. 35 illustrates an example of a screen to be displayed on the terminal device of the care giver.
[FIG. 36A] FIG. 36A illustrates an example of a screen to be displayed on a terminal device of a care manager.
[FIG. 36B] FIG. 36B illustrates an example of a screen to be displayed on the terminal device of the care manager.
[FIG. 36C] FIG. 36C illustrates an example of a screen to be displayed on the terminal device of the care manager.

### Description of Embodiments

Hereinafter, the present embodiments will be described with reference to the drawings. As for the drawings, the same reference numerals are given to the same or similar elements, and redundant descriptions are omitted. Note that, the present embodiments to be described below do not unduly limit the scope of the claims. Moreover, all the configurations to be described in the present embodiments do not need to be essential features in the disclosure.

### 1. System Configuration Example

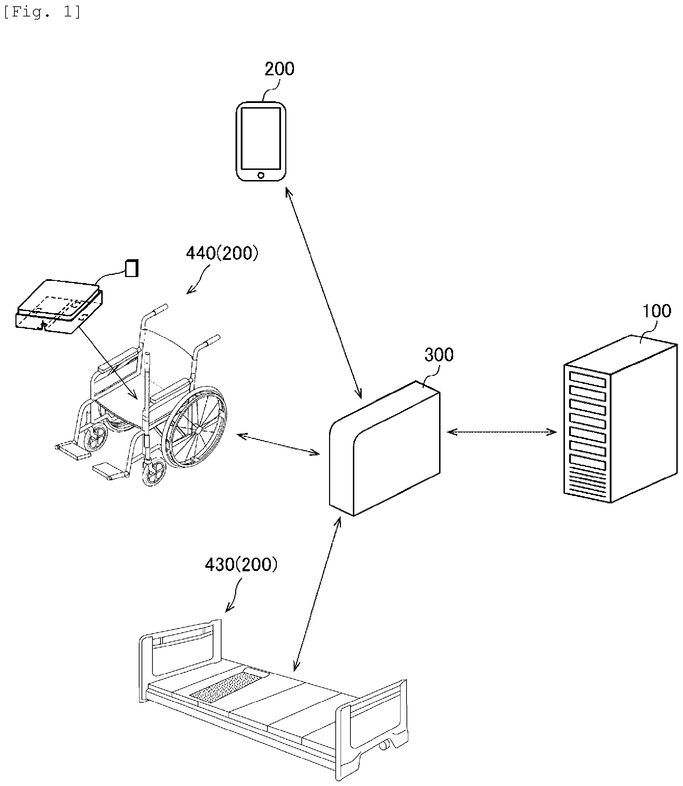

FIG. 1 is a diagram illustrating a configuration example of an information processing system 10 according to this embodiment. As for work that is performed in accordance with "intuitions" and "tacit knowledge" of care givers, for example, in medical facilities and care facilities, the information processing system 10 according to the embodiment provides, by digitalizing the "intuition" and the "tacit knowledge", an instruction to a care giver so as to perform appropriate care assistance independent of the degree of proficiency.

Note that, the care giver herein may be a care staff in a care facility, or may be a nurse or a practical nurse in a medical facility such as a hospital. In other words, the care assistance in the embodiment includes various actions that support a care receiver, may include care, or may include an activity related to medical care such as an injection. Moreover, the care receiver herein is a person who receives care assistance by the care giver, may be a resident in a care facility, or may be a patient who is hospitalized or visits a hospital.

Moreover, the care assistance in the embodiment may be performed at home. For example, the care receiver in the embodiment may be a care recipient who receives at-home care, or may be a patient who receives at-home medical care. Moreover, the care giver may be a family of the care recipient, the patient, or the like, or may be a home-visit care helper or the like.

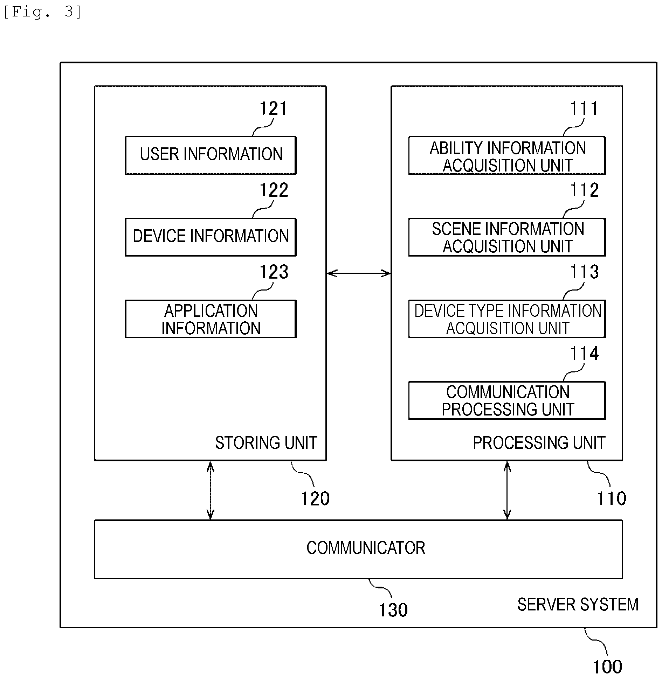

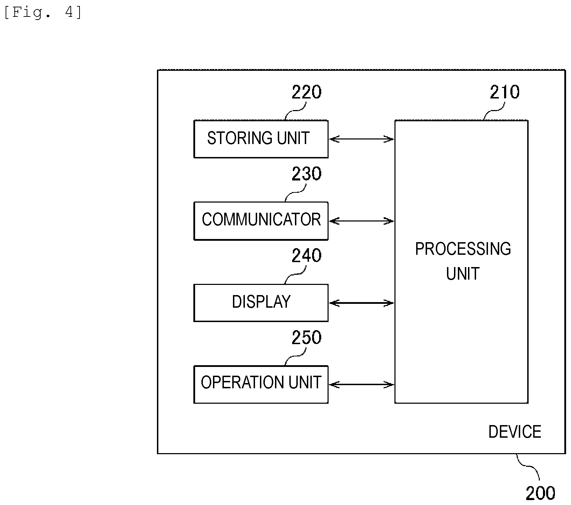

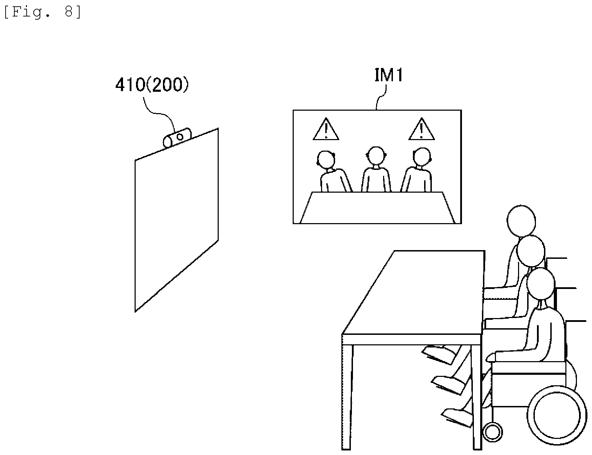

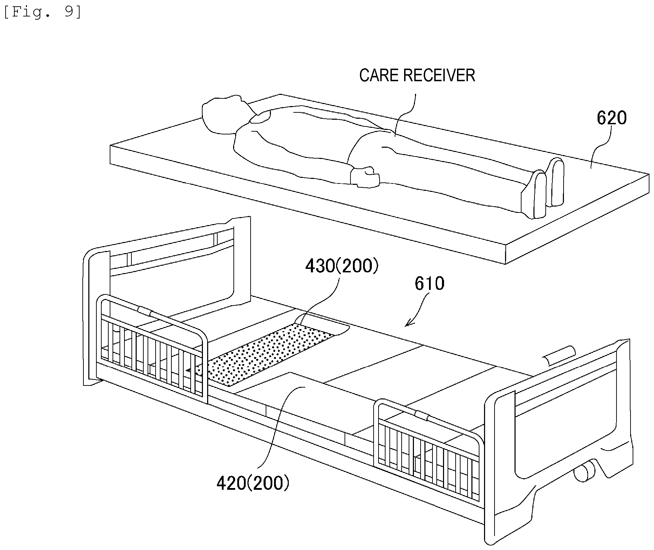

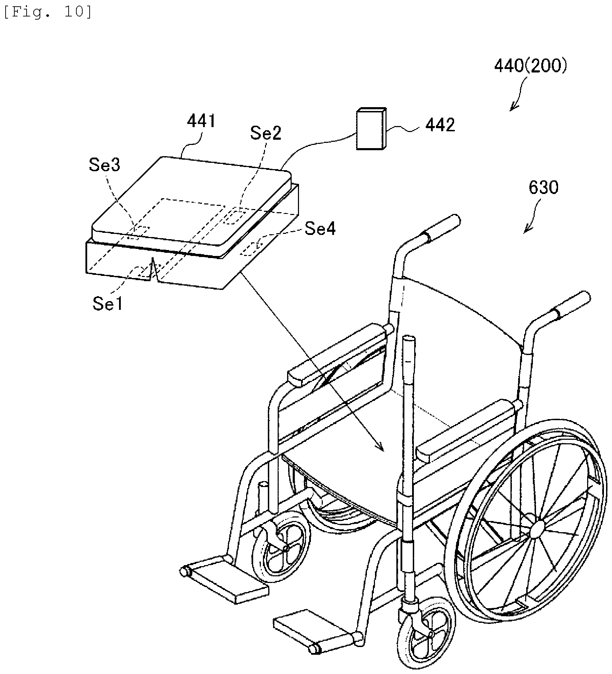

The information processing system 10 illustrated in FIG. 1 includes a server system 100, a device 200, and a gateway 300. The configuration of the information processing system 10 is not limited to that in FIG. 1, but various modifications such as omitting a part thereof, adding another configuration, and the like can be made. For example, FIG. 1 exemplifies, as the device 200, a tablet type terminal device such as a smartphone, a seat surface sensor 440 (which is described later using FIG. 10) that is disposed to a wheelchair 630, and a detection device 430 (which is described later using FIG. 9) that is placed to a bed 610, but the number and the types of the devices 200 are not limited to these. For example, the information processing system 10 may include various kinds of devices 200, which are described later using FIGs. 8 to 14. Moreover, the information processing system 10 is not prevented from including devices other than the devices 200 illustrated in FIGs. 8 to 14. In the following, if a plurality of devices 200 do not need to be distinguished from each other, the devices 200 are simply expressed as the device 200. Moreover, modifications such as the omission and addition of the configuration can be made similarly in FIGs. 3 and 4 and other drawings, which are described later.

The information processing apparatus in the embodiment corresponds to the server system 100, for example. Further, a method in the embodiment is not limited thereto, but the processing of the information processing apparatus can be executed by the distribution processing using the server system 100 and other apparatuses. For example, the information processing apparatus in the embodiment may include the server system 100 and the device 200. Hereinafter, an example in which the information processing apparatus is the server system 100 will be described.

The server system 100 is connected to the device 200 via a network, for example. For example, the server system 100 is connected to the gateway 300 via a public communication network such as the Internet, and the gateway 300 is connected to the device 200 using Local Area Network (LAN) and the like. For example, the gateway 300 may be an access point (AP) that performs communication in conformity with the standard of IEEE802. 11, and the device 200 may be a station (STA) that performs communication in conformity with the standard of IEEE802. 11. Various modifications for the communication method between the respective devices can be made.

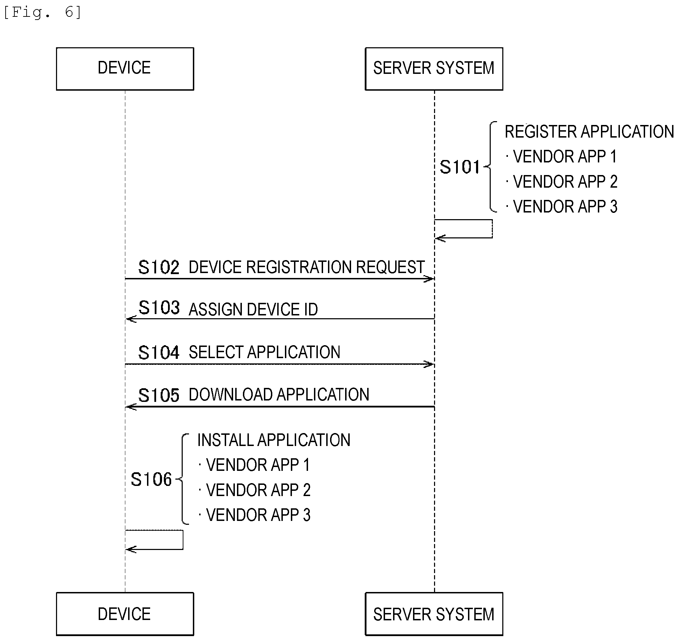

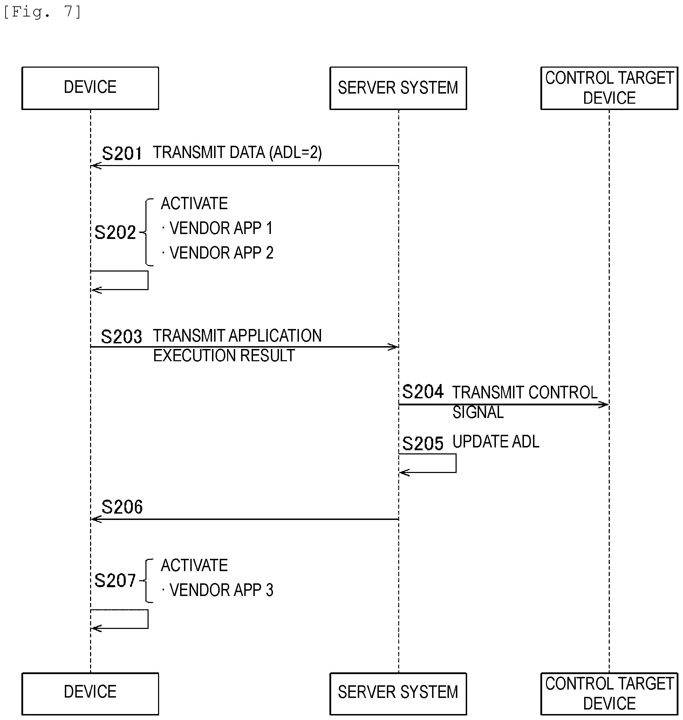

The server system 100 may be one server, or may include a plurality of servers. The server system 100 may include a database server and an application server, for example. The database server stores various kinds of data, which is described later using FIG. 3. The application server performs processing, which is described later using FIG. 6 and FIG. 7 and other drawings. The plurality of servers herein may be physical servers or may be virtual servers. Moreover, in a case where a virtual server is used, the virtual server may be provided in one physical server, or the virtual servers may be disposed in a distributed manner to a plurality of physical servers. As in the foregoing, various kinds of modifications can be made for the specific configuration of the server system 100 in the embodiment.

The device 200 includes various kinds of sensors, for example, and performs processing based on data (hereinafter, expressed as sensing data) sensed by the sensor. The abovementioned digitalization of the tacit knowledge of the skilled worker may be executed by a vendor of the device 200. For example, it is assumed that a skilled worker is provided with tacit knowledge for making a forward displacement/lateral displacement determination based on a posture of a care receiver on a wheelchair. In this case, sensing data corresponding to the posture of the care receiver to be detected by the seat surface sensor 440 is collected, and an application for making a forward displacement/lateral displacement determination is created based on the sensing data, whereby it is possible to digitalize the abovementioned tacit knowledge. For example, the vendor provides the seat surface sensor 440 and the abovementioned application. As a result, a person (for example, a new staff) who is not a skilled worker can use the forward displacement/lateral displacement determination and the like equivalent to those by the skilled worker.

Moreover, tacit knowledge to be digitalized in the single device 200 is not limited to one. For example, tacit knowledge to be digitalized using the seat surface sensor 440 is not limited to the forward displacement/lateral displacement determination, but may include tacit knowledge for making a determination of a fall possibility, and tacit knowledge in which both of the forward displacement/lateral displacement determination and the fall possibility are combined. The forward displacement/lateral displacement determination corresponds to the determination as to whether the posture of the care receiver is right or wrong, and the determination of the fall possibility corresponds to the determination of slipping off from the seat surface. Moreover, in the forward displacement/lateral displacement determination as well, a plurality of tacit knowledge having different determination references for determining which extent of the displacement as forward displacement or lateral displacement, and different determination processing contents, and the like can be present. Therefore, each of the devices 200 can execute the processing corresponding to one or a plurality of tacit knowledge, and may switch whether the processing corresponding to each tacit knowledge is executed. For example, a vendor may mount tacit knowledge as application software (hereinafter, also simply referred to as an application), and may register the application to the server system 100. Each of the devices 200 downloads and installs an application having an execution possibility, among the registered applications, thereby executing processing corresponding to the tacit knowledge.

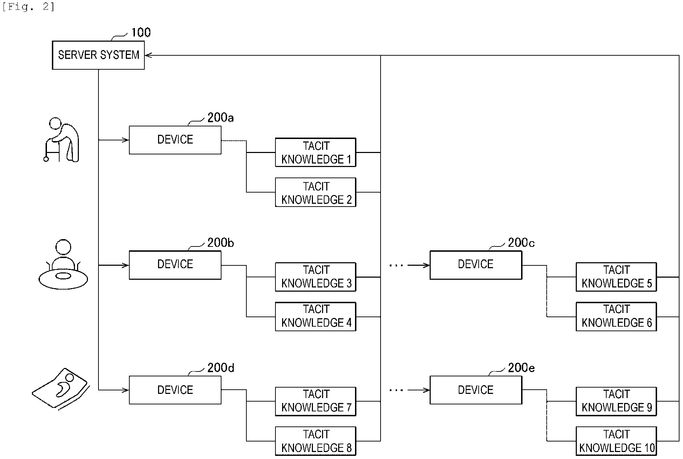

FIG. 2 is a diagram illustrating a relation example between the device 200 and tacit knowledge (applications). FIG. 2 illustrates five examples of devices 200a to 200e, as the device 200 to be connected to the server system 100. In the example of FIG. 2, tacit knowledge 1 and tacit knowledge 2 as two tacit knowledge are associated with the device 200a. For example, the device 200a is in a state where applications of the tacit knowledge 1 and the tacit knowledge 2 are already installed. The device 200a may be a device related to a falling-down risk, for example, which is described later. Specifically, the device 200a may be the seat surface sensor 440, the tacit knowledge 1 may be tacit knowledge related to the forward displacement/lateral displacement determination, and the tacit knowledge 2 may be tacit knowledge related to the fall possibility determination. A processing result corresponding to the tacit knowledge 1 and the tacit knowledge 2 is transmitted to the server system 100, for example.

The device 200b and the device 200c are devices related to an aspiration risk, for example, which is described later, and each of which is associated with a plurality of tacit knowledge in order to respond to the aspiration risk and the like. The device 200d and the device 200e are devices related to a bedsore risk, for example, which is described later, and each of which is associated with a plurality of tacit knowledge in order to respond to the bedsore risk and the like. In this way, it is possible to digitalize a variety of tacit knowledge using various kinds of devices. Two tacit knowledge with respect to one device 200 is exemplified herein, but the number of tacit knowledge to be associated with one device 200 is not limited to this.

With the method in the embodiment, it is possible to switch tacit knowledge to be used depending on the situation. For example, all of the tacit knowledge 1 to the tacit knowledge 10 illustrated in FIG. 2 do not need to be used, but the use and disuse are switched if necessary. Switching tacit knowledge may be implemented by switching the device 200 to be used. For example, in a case where a target is a care receiver having a high falling-down risk but having a low aspiration risk and a low bedsore risk, the device 200a is used, but the devices 200b-200e are not used. In this way, at least one of the tacit knowledge 1 and the tacit knowledge 2 is used, and the other tacit knowledge is not used, so that it is possible to appropriately use tacit knowledge that is highly necessary for the care receiver. Moreover, in a case where the aspiration risk of the care receiver has increased, it is possible to switch tacit knowledge to be used using the device 200b or the device 200c. Moreover, in a case where the aspiration risk is responded as well, a case where only the device 200b is used, a case where only the device 200c is used, a case where both of the devices 200b and 200c are used, and other cases may be switched. In this way, for example, in a case where the aspiration risk is high, necessary tacit knowledge, among the tacit knowledge 3 to the tacit knowledge 6 in FIG. 2, can be used. The same applies to a case where the bedsore risk has increased, it is possible to switch tacit knowledge to be used using the device 200d or the device 200e. Moreover, in a case where the falling-down risk has decreased, such switching that the use of the device 200a is stopped is also possible.

Moreover, switching of tacit knowledge may be implemented, while maintaining a device 200 to be used, by switching tacit knowledge to be used in the device 200. For example, in the device 200a, a case where only the tacit knowledge 1 is used, a case where only the tacit knowledge 2 is used, a case where both of the tacit knowledge 1 and the tacit knowledge 2 are used, and other cases may be switched. This processing can be implemented by controlling an active/inactive state of an application corresponding to each tacit knowledge, for example.

Switching of tacit knowledge may be executed using ability information on a care receiver. The ability information herein is information indicating an activity ability of the care receiver, and is information that is obtained as a result that the device 200 has performed the processing using a some sort of tacit knowledge, for example. The ability information may be, for example, information related to the level of a falling-down risk, an aspiration risk, and a bedsore risk. Details of the ability information will be described in detail later.

For example, in a case where a processing result of tacit knowledge has been obtained in the given device 200, processing of switching tacit knowledge to be used in the same device 200 may be performed. For example, based on a processing result of the tacit knowledge 1 in the device 200a of FIG. 2, an active/inactive state of an application corresponding to the tacit knowledge 1 and an application corresponding to the tacit knowledge 2 may be switched.

Moreover, in a case where a processing result of the tacit knowledge has been obtained in the given device 200, the processing result may have an influence on another device 200. For example, based on a processing result of the tacit knowledge 1 in the device 200a, tacit knowledge to be used in the device 200b is switched. For example, switching of from a state where the device 200b is not used to a state where the device 200b is used may be performed. Alternatively, an active/inactive state of tacit knowledge 3 and tacit knowledge 4 associated with the device 200b may be individually switched.

Moreover, switching of tacit knowledge is not limited to that based on the ability information, but may be executed based on a care assistance scene of a care receiver, a combined use situation of a plurality of devices 200, and the like. Details of information to be used in switching of tacit knowledge, and details of processing of switching tacit knowledge will be described later.

Note that, it is possible to flexibly change a correspondence relationship between the device 200 and tacit knowledge. For example, based on sensing data acquired using the plurality of devices 200, processing corresponding to one tacit knowledge may be executed. For example, based on sensing data acquired by the device 200a and sensing data acquired by the device 200b, processing corresponding to the tacit knowledge 1 may be executed. In this way, since the types of sensing data to be used in the processing can be increased, the improvement and the like in the processing accuracy are possible. In the abovementioned example, the processing corresponding to the tacit knowledge 1 may be performed in the device 200a, may be performed in the device 200b, or may be implemented by distribution processing of the device 200a and the device 200b. In addition, the processing corresponding to the tacit knowledge is not limited to that to be executed by the device 200 that acquires sensing data. For example, based on sensing data acquired by the device 200a and sensing data acquired by the device 200b, processing corresponding to the tacit knowledge 1 may be executed in the device 200 different from either one or both of the device 200a and the device 200b. For example, in FIG. 1, based on sensing data acquired by the seat surface sensor 440 or sensing data acquired by the detection device 430, the device 200, which is a smartphone, may execute processing corresponding to the tacit knowledge.

Note that, although the example in which a vendor of the device 200 digitalizes tacit knowledge has been described in the foregoing, the embodiment is not limited thereto. For example, a care giver who uses the device 200 is not prevented from digitalizing own tacit knowledge. For example the care giver may create an application corresponding to the tacit knowledge, and register the application to the server system 100. In this way, it is possible to promote the digitalization and use of the tacit knowledge.

FIG. 3 is a block diagram illustrating a detailed configuration example of the server system 100. The server system 100 includes, for example, a processing unit 110, a storing unit 120, and a communicator 130.

The processing unit 110 in the embodiment is implemented by hardware described below. The hardware can include at least one of a circuit for processing digital signals and a circuit for processing analog signals. For example, the hardware may be implemented by one or a plurality of circuit devices mounted to a circuit substrate and/or one or a plurality of circuit elements. One or a plurality of circuit devices are, for example, an integrated circuit (IC) and a field-programmable gate array (FPGA). One or a plurality of circuit elements are, for example, a resistance and a capacitor.

Moreover, the processing unit 110 may be implemented by processors described below. The server system 100 in the embodiment includes a memory that stores information, and a processor that operates based on the information stored in the memory. The information is, for example, a program and various kinds of data. The memory may be the storing unit 120, or may be another memory. The processor includes hardware. As the processors, various kinds of processors including a central processing unit (CPU), a graphics processing unit (GPU), a digital signal processor (DSP), and the like can be used. The memory may be a semiconductor memory such as a static random access memory (SRAM), a dynamic random access memory (DRAM), and a flash memory, may be a register, may be a magnetic storage device such as a hard disk device (HDD: hard disk drive), and may be an optical storage device such as an optical disc device. For example, the memory stores an instruction readable by a computer, and the processor executes the instruction, thereby implementing a function of the processing unit 110 as processing. The instruction herein may be an instruction in an instruction set constituting the program, or may be an instruction to instruct the hardware circuit of the processor to operate.

The processing unit 110 includes, for example, an ability information acquisition unit 111, a scene information acquisition unit 112, a device type information acquisition unit 113, and a communication processing unit 114.

The ability information acquisition unit 111 performs processing of acquiring ability information indicating an activity ability of a care receiver. For example, the ability information acquisition unit 111 acquires sensing data from the device 200. The sensing data herein may be, for example, log data in a predetermined period. The ability information acquisition unit 111 obtains, based on the log data, a time-series change in a state of the care receiver, and estimates ability information based on the change. The ability information herein may be indicator information related to activities of daily living (ADL). Various kinds of methods for evaluating ADL, for example, Barthel Index, are known, and are widely applicable in the embodiment. Moreover, as for ability information, indicators of nine states may be used, which are disclosed as a Clinical Frailty Scale in "Frailty and the potential kidney transplant recipient: time for a more holistic assessment?", Henry H. L. Wu et al. For example, the ability information acquisition unit 111 in the embodiment determines to which stage in the nine stages the care receiver belongs based on the sensing data.

Note that, ability information may be obtained in the device 200 and the like. The ability information acquisition unit 111 may execute processing of acquiring ability information via the communicator 130 from the device 200 and the like.

The scene information acquisition unit 112 determines a scene in which care assistance to a care receiver is performed. The scene information herein may be information specifying a type of care assistance to be executed, such as meal care assistance, excretion care assistance, movement and transfer care assistance, and the like. Moreover, the scene information may be information related to a care giver, such as the number of care givers who execute care assistance to a care receiver, the degree of proficiency, and the like. Moreover, the scene information may be information related to a care receiver, such as an attribute and the like of the care receiver. For example, the scene information acquisition unit 112 performs processing of obtaining scene information based on a user input, a schedule of a care giver, and the like.

The device type information acquisition unit 113 is information specifying the type of the device 200 that operates with the target device 200. The device type herein indicates a rough classification, such as a wheelchair and a bed, and may be information in which vendors are not distinguished. For example, a wheelchair of a first vendor and a wheelchair of a second vendor different from the first vendor have the same device type. For example the device type information acquisition unit 113 may perform processing of specifying another device 200 that is used by a care receiver (alternatively, a care giver who provides care assistance to the care receiver) who uses the target device 200, and acquiring information indicating the type of the another device 200 as device type information.

The communication processing unit 114 controls communication that uses the communicator 130. For example, the communication processing unit 114 executes processing of creating data to be transmitted, such as a MAC frame in a data link layer, and the like. Moreover, the communication processing unit 114 may perform processing of interpreting a frame structure with respect to data received by the communicator 130, extracting necessary data, and outputting the necessary data to an upper-level layer of an application or the like.

The storing unit 120 is a work area of the processing unit 110, and stores various kinds of information. The storing unit 120 can be implemented by various kinds of memories, and the memory may be a semiconductor memory such as SRAM, DRAM, ROM, and a flash memory, may be a register, may be a magnetic storage device, and may be an optical storage device.

The storing unit 120 may store user information 121, device information 122, and application information 123.

In the method in the embodiment, for example, tacit knowledge is registered to the server system 100 as an application. Each user who uses the tacit knowledge may register the device 200 to the system, and then download a necessary application to the device 200 to use the application.

The user information 121 includes information, such as a user ID and a user name, uniquely specifying a user of the information processing system 10, and a device ID or the like that is information uniquely specifying the device 200 that is used by the user.

The device information 122 is information related to the device 200, and includes a device ID, a device type ID indicating a type of the device 200, a vendor, an application ID of an installed application, and the like. The application ID is information uniquely specifying an application.

The application information 123 is information related to an application, and includes an application ID, an application name, a creator, and the like. Moreover, the application information may include information specifying a specific processing content of the application. The information specifying a processing content may be a source code of a program or may be an executable file. Moreover, in a case where the application corresponds to a trained model, the application information may include information on a structure of the trained model. For example, in a case where the trained model is a neural network (hereinafter, expressed as NN), the structure of the trained model includes the number of layers in NN, the number of nodes included in each layer, a connection relation between the nodes, a weight, an activating function, and the like .

Using the user information 121 makes it possible to appropriately manage a user who uses the information processing system 10. Moreover, referring to the device information 122 makes it possible to check details of the device 200 that is used by each user. In addition, referring to the application information 123 makes it possible to check details of each application registered to the server system 100.

The communicator 130 is an interface for performing communication via the network, and includes, in a case where the server system 100 performs wireless communication, for example, an antenna, a radio frequency (RF) circuit, and a base band circuit. The server system 100 may perform wired communication, and the communicator 130 in that case may include a communication interface such as an Ethernet connector, a control circuit of the communication interface, and the like. The communicator 130 operates in accordance with the control by the communication processing unit 114. Note that, the communicator 130 is not prevented from including a processor for communication control, which is different from the communication processing unit 114. The communicator 130 may perform communication in accordance with a scheme defined in IEEE802. 11 and IEEE802. 3, for example. Various kinds of modifications can be made for a specific communication scheme.

FIG. 4 is a block diagram illustrating a detailed configuration example of the device 200. The device 200 includes, for example a processing unit 210, a storing unit 220, a communicator 230, a display 240, and an operation unit 250. As is described later using FIGs. 8 to 14, in the method in the embodiment, the devices 200 of various kinds of forms can be used. The configuration of each device 200 is not limited to that in FIG. 4, but modifications such as omitting a part of the configuration, adding another configuration, and the like can be made. For example, the device 200 may include various kinds of sensors in accordance with the device 200, including a motion sensor such as an acceleration sensor and a gyroscope sensor, an imaging sensor, a pressure sensor, a global positioning system (GPS) sensor, and the like.

The processing unit 210 is implemented by hardware including at least one of a circuit for processing digital signals and a circuit for processing analog signals. The processing unit 210 may be implemented by processors. As the processors, various kinds of processors including CPU, GPU, DSP, and the like can be used. The processor executes an instruction stored in the memory of the device 200, thereby implementing the function of the processing unit 210 as processing.

The storing unit 220 is a work area of the processing unit 210, and is implemented by various kinds of memories including SRAM, DRAM, ROM, and the like.

The communicator 230 is an interface for performing communication via the network, and includes, for example, an antenna, an RF circuit, and a base band circuit. The communicator 230 performs communication with the server system 100 via the network, for example. The communicator 230 may execute wireless communication in conformity with the standard of IEEE802. 11, for example with the gateway 300, and perform communication with the server system 100 via the gateway 300.

The display 240 is an interface that displays various kinds of information, and may be a liquid crystal display, may be an organic EL display, or may be a display using another scheme. The operation unit 250 is an interface that receives a user operation. The operation unit 250 may be a button or the like that is provided in the device 200. Moreover, the display 240 and the operation unit 250 may be integrally configured as a touch panel.

The device 200 may also include a configuration not illustrated in FIG. 4 such as a light emitting unit, a vibration unit, a sound input unit, and a sound output unit. The light emitting unit is, for example, a light emitting diode (LED), and makes a notification by light emission. The vibration unit is, for example, a motor, and makes a notification by vibration. The sound input unit is a microphone, for example. The sound output unit is a speaker, for example, and makes a notification by sound.

### 2. Device Control Based on Ability Information

The information processing system 10 in the embodiment includes the server system 100 and the device 200 as illustrated in FIG. 1. Moreover, the device 200 may operate in any one among a plurality of operation modes. The operation mode in the embodiment may be determined based on a combination of tacit knowledge (applications) to be used. For example, as mentioned above, the device 200, to which a plurality of applications corresponding to different tacit knowledge can be installed, switches an active/inactive state of each of the plurality of applications. In this way, it is possible to appropriately switch the tacit knowledge to be used in accordance with a situation.

Particularly, the server system 100 (the ability information acquisition unit 111) may obtain ability information indicating an activity ability of a care receiver based on the sensing data transmitted from the device. Moreover, the server system 100 transmits the obtained ability information to the device 200. The device 200 determines in which operation mode among a plurality of operation modes the device 200 operates based on the received ability information. There is a possibility that a desired operation of the device 200 changes because care assistance to be conducted changes depending on a change in the ability information of the care receiver, but with the method in the embodiment, it is possible to appropriately switch the operation mode in accordance with the ability information. For example, the device 200 can appropriately switch an active/inactive state (use/disuse of tacit knowledge) of the application in accordance with the ability information. Hereinafter, the processing based on the ability information will be described.

### 2.1 Overview

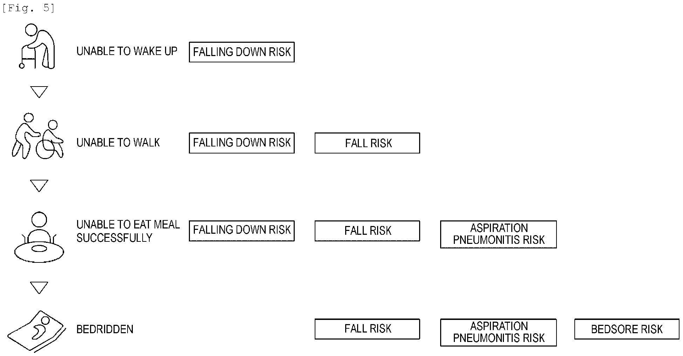

FIG. 5 is a diagram illustrating a relation example of between ability information on a care receiver and assumed risks. For example, in a case where the ability of a care receiver is sufficiently high, the care receiver can execute a daily motion without care assistance by another person, so that a risk in daily living is not high. However, when lowering of the ability is started, for example, it becomes difficult for the care receiver to firstly perform a wake-up motion. The wake-up motion indicates a standing-up motion and a sitting motion. In this case, it becomes difficult to balance in the movement starting motion including the wake-up motion, so that a falling-down risk of the care receiver increases. The movement starting motion indicates a motion of starting movement from a state where the movement is small (a static state in a narrow sense). Meanwhile, less trouble is assumed in daily motions other than the start of movement at this stage. For example, a care receiver can hold a sitting position state for a long period of time, walk using a wheeled walker or the like after standing up, and eat a meal freely to some extent, for example.

When the ability is further lowered, for example, the care receiver becomes difficult to walk, and movement care assistance using a wheelchair and the like becomes necessary. In this case, a high falling-down risk state in the start of movement is similar to the abovementioned example, and in addition an ability of holding a sitting position state is lowered, so that a fall risk needs to be considered. For example, a care receiver at this stage may lose a balance even in a state where the care receiver sits on a bed or a wheelchair, and fall down from a mattress of the bed or a seat surface of the wheelchair.

When the ability is further lowered, for example, the care receiver cannot eat a meal successfully. For example, a swallowing ability is lowered, so that an aspiration risk becomes high. The aspiration risk indicates that a possibility of the occurrence of aspiration pneumonitis becomes high, for example. It can be considered that the care receiver can move the body at this stage, so that the abovementioned state where the falling-down risk and the fall risk are high is maintained, and in addition a risk of aspiration pneumonitis needs to be considered.

When the ability is further lowered, for example, the care receiver is shifted to a bedridden state where care assistance becomes necessary in large part of the daily motions. In this case as well, there is a possibility of diaper changing on the bed and the movement by the wheelchair or the like, so that a fall risk is high. Moreover, unless special circumstances, such as gastric fistula, a meal is continuously taken by mouth, so that the aspiration pneumonitis risk is also high. In addition, the care receiver stays on the bed in an extremely long period of time, and is not easy to spontaneously roll over, so that the bedsore risk also becomes high. Meanwhile, in a bedridden case, it is not assumed that the care receiver starts a movement by himself/herself, so that the falling-down risk at the start of movement becomes low.

As illustrated in FIG. 5, an assumed risk is different in accordance with the ability of a care receiver, so that care assistance to be performed by a care giver changes. Accordingly, in a case where tacit knowledge digitalized using the device 200 is used, tacit knowledge to be used also changes in accordance with the ability. In that regard, the operation mode of the device 200 can be set in accordance with the ability information in the embodiment, so that it is possible to execute processing in accordance with the ability change. For example, in a case where care assistance is provided for about ten care receivers as a unit in a care facility or the like, it can be considered that the care receivers having different abilities are mixed in the unit, but with the method in the embodiment, a care giver does not need to manually set necessary care assistance for each care receiver. In other words, even in a case where a care giver is in charge of a plurality of care receivers, it is possible to set appropriate operation modes without increasing burden of the care giver.

Note that, the description has been made in FIG. 5 using an example in which the ability is lowered downward from the top, but the direction of the ability change is not limited thereto. For example, the ability is recovered by the cure of the illness, the remission, the implementation of rehabilitation, and the like in some cases. The method in the embodiment sets the operation mode in accordance with the ability, and can flexibly respond to a case where the ability is recovered. Moreover, four stages of "UNABLE TO WAKE UP", "UNABLE TO WALK", "UNABLE TO EAT MEAL SUCCESSFULLY", and "BEDRIDDEN" are exemplified in FIG. 5, the stage of the ability indicated by the ability information is not limited thereto, but a part of the stages may be omitted or another stage may be added. Moreover, a state may be considered where the care receiver can walk and a falling-down risk is thus low, but an aspiration risk becomes high because the swallowing ability has decreased. In other words, the abovementioned four stages are not limited to those that change in the abovementioned order, but a more complicated combination may be considered.

FIG. 6 is a sequence diagram illustrating the operations of the server system 100 and the device 200, and is a diagram illustrating preprocessing that is executed before the device 200 executes processing corresponding to the tacit knowledge.

Firstly, at Step S101, the server system 100 performs in advance processing of receiving the registration of an application. For example, as mentioned above, each application corresponds to tacit knowledge, and is created by a vendor or the like of the device 200. A creator of an application performs a login to the information processing system 10 in the embodiment using an arbitrary terminal device (a PC, a smartphone, or the like), for example, and then performs processing of registering the application to the server system 100 using a vendor screen, which is not illustrated, that is displayed on a display of the terminal device. The processing unit 110 of the server system 100 stores information related to the registered application in the storing unit 120 as the application information 123. Herein, an example in which a vendor app 1 to a vendor app 3, which are applications created by the vendors, are registered is illustrated.

At Step S102, based on an operation by a user who uses the device 200, a registration request of the device 200 is transmitted to the server system 100. The user herein may be a care giver who uses tacit knowledge, or may be an administrator or the like of the care facility. For example, the user executes the processing at Step S102 when introducing a new device 200 into the own environment. For example, a user performs a login to the information processing system 10 using theoperation unit of the device 200 or an operation unit of a terminal device to be connected to the device 200, and then performs processing of registering the device 200 to the server system 100 using a user screen, which is not illustrated. The registration request includes, for example, a user ID specifying a user, and information on the device 200 such as a vendor and a model.

At Step S103, the processing unit 110 of the server system 100 executes the processing based on the registration request. For example, the processing unit 110 assigns a device ID uniquely specifying the device 200 to the target device 200, and transmits the device ID to the device 200. Moreover, the processing unit 110 may execute processing of associating a login user with the device 200 about which the registration request has been made. For example, the processing unit 110 may perform processing of adding a device ID of the device 200 about which the registration request has been made to the user information 121 of the login user. Moreover, the processing unit 110 may store the device ID of the device 200 about which the registration request has been made in association with a device type ID and the like of the device 200, in the device information 122. The device type ID can be specified based on, for example, the information, such as a vendor and a model, that is included in the registration request. With the processing in the foregoing, the device 200 to be newly introduced is registered to the information processing system 10.

Next, at Step S104, based on an operation by the user who uses the device 200, an application to be used by the device 200 is selected. For example, in a case where the registered device 200 has accessed the server system 100, the server system 100 may reply in a screen of a list of applications available in the device 200. The user performs an user operation of selecting an application to be used from the list-displayed applications. Herein, an example in which applications including the vendor app 1 to the vendor app 3 registered by the processing illustrated at Step S101 are list-displayed, and the user has performed the selection operation of the vendor app 1 to the vendor app 3 is considered.

At Step S105, the server system 100 permits download of the selected application, and the device 200 executes the download of the selected application. Moreover, the server system 100 may perform processing of associating the device 200 with the application downloaded to the device 200. For example, the processing unit 110 executes processing of adding application IDs corresponding to the vendor app 1 to the vendor app 3, to the device information 122 related to the device 200.

At Step S106, the device 200 executes processing of installing the downloaded vendor app 1 to vendor app 3. This enables the device 200 to operate in any of a plurality of operation modes. For example, the device 200 may switch an active/inactive state of each of the vendor app 1 to the vendor app 3. In this case, the device 200 can select 2³=8 operation modes. Note that, in the embodiment, a state where all the vendor app 1 to the vendor app 3 are inactive is considered as one operation mode. Moreover, a relation between the application and the operation mode is not limited thereto. For example, a plurality of applications may operate exclusively. In the abovementioned example, the device 200 may be able to set four operation modes of a mode in which all the vendor apps are inactive, a mode in which only the vendor app 1 is active, a mode in which only the vendor app 2 is active, and a mode in which only the vendor app 3 is active.

FIG. 7 is a sequence diagram illustrating operations of the server system 100 and the device 200, and is a diagram illustrating an example in which the operation mode of the device 200 changes based on ability information on a care receiver.

Firstly, at Step S201, the server system 100 performs processing of transmitting data including ability information to the device 200. FIG. 7 illustrates the example in which data having an ADL indicator value of 2 is transmitted.

At Step S202, the device 200 controls an active/inactive state of the installed vendor app based on the acquired ability information. For example, the storing unit 220 of the device 200 may store information in which ability information is associated with an operation mode. The processing unit 210 of the device 200 performs processing of obtaining an operation mode based on the information and ability information acquired from the server system 100. For example, the storing unit 220 may store table data in which an indicator value of ADL is associated with an active/inactive state of each application. The processing unit 210 extracts a record coincident with the received indicator value of ADL in the table data, thereby determining an active/inactive state of each application. Herein, an operation mode in which the vendor apps 1 and 2 become active, and the vendor app 3 becomes inactive, among the vendor app 1 to the vendor app 3, is set. Further, the processing of determining an operation mode based on the ability information is not limited to the abovementioned example, but various kinds of modifications are executable.

After the processing at Step S202, the device 200 executes processing in accordance with the vendor app 1, and processing in accordance with the vendor app 2. Specifically, the processing unit 210 of the device 200 acquires sensing data using a sensor, and executes the processing defined by the application using the sensing data as an input, thereby obtaining a processing result. At Step S203, the device 200 transmits the processing result to the server system 100. The processing result herein corresponds to a result of determination executed using the tacit knowledge of the skilled worker. Moreover, the information herein to be transmitted to the server system 100 is not limited to the processing result, but may include information such as a log of the sensing data.

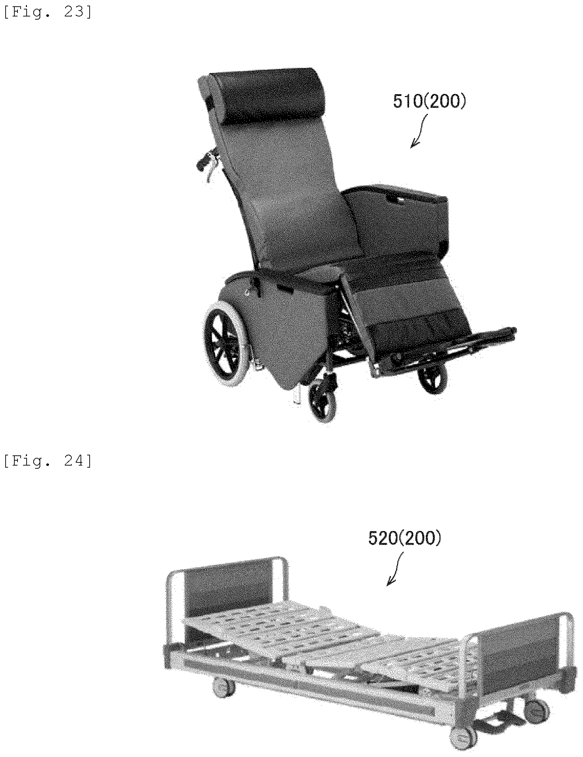

At Step S204, the server system 100 executes control based on the processing result received from the device 200. For example, the processing unit 110 may perform processing of specifying a control target device, and transmitting a control signal for operating the control target device. The control target device herein may be a reclining wheelchair 510, which is described later using FIG. 23 or a care bed 520, which is described later using FIG. 24. The control signal in this case may be a signal instructing a change in an angle of a back surface part of the reclining wheelchair 510 or a section angle of the care bed 520. Moreover, the control signal may be a signal instructing the control target device to execute a notification. For example, the control target device is a device including a notification unit such as a display and a light emitting unit, and the control signal is a signal instructing the control target device to execute the notification using display of an image, light emitting, and the like.

Moreover, the processing unit 110 of the server system 100 or the processing unit 210 of the device 200 may perform the processing of determining a control target device and a control content based on the processing result in the device 200. In the latter case, at Step S203, in addition to the processing result, information specifying a control target device and a control content may be transmitted. Moreover, the device 200 or the server system 100 may only specify a control target device, and the determination of a specific control content may be executed in the control target device. In this case, at each of Steps S203 and S204, the processing of transmitting the processing result is executed. In addition, various kinds of modifications are executable for a control target device, a control signal, and the like. For example, the control target device may be the device 200. Moreover, at Step S204, the server system 100 may perform processing of storing a log of sensing data transmitted from the device 200, in the storing unit 120.

Moreover, at Step S205, the server system 100 executes processing of updating the ability information on the care receiver. For example, the ability information acquisition unit 111 may obtain the ability information based on the log of the abovementioned sensing data. For example, the storing unit 120 of the server system 100 may store information for associating sensing data with ability information. The processing unit 110 executes processing of obtaining ability information based on the information, and the sensing data transmitted from the device 200. Herein, the information for associating sensing data with ability information may be a trained model. The training data for generating a trained model herein is, for example, data in which with respect to sensing data related to a care receiver, ability information on the care receiver determined by a person (for example, a medical doctor or a skilled care giver) having expert knowledge is assigned as ground truth data. The ground truth data may be an indicator value indicating an ability, or may be a set of information indicating the presence or absence and the level difference of an individual ability (a sitting position hold ability, a swallowing ability, and the like, which are described later), as mentioned above. The processing unit 110 inputs sensing data into a trained model, thereby obtaining ability information. Alternatively, information to be stored in the storing unit 120 may be reference data that is sensing data in which a correspondence relationship with the ability information is known. The processing unit 110 may determine a similarity between the acquired sensing data and the reference data, and obtain ability information based on the similarity. The reference data herein may be sensing data acquired from a care receiver having a high ability, for example. In this case, in a case where the similarity with the reference data is high, a high ability value is determined, and in a case where the similarity is low, a low ability value is determined. Moreover, reference data may be another information such as sensing data acquired from a care receiver having a low ability. Moreover, the processing unit 110 may use the present ability information in the processing. For example, the processing unit 110 may obtain a changing amount of ability information based on the sensing data, and may obtain updated ability information based on the changing amount and the present ability information. A specific example of the processing of obtaining ability information is described later. Moreover, in the processing at Step S205, information other than the sensing data, such as a report to be input by the care giver and a medical examination result by a medical doctor, and the like, may be used.

At Step S206, the server system 100 performs processing of transmitting data including the updated ability information, to the device 200. FIG. 7 illustrates the example in which data having an ADL indicator value of 3 is transmitted.

At Step S207, the device 200 controls an active/inactive state of the installed vendor app based on the acquired ability information. For example, as mentioned above, the device 200 determines an active/inactive state of each vendor app based on table data. In the example of FIG. 7, the vendor apps 1 and 2 are maintained in the active state, and the vendor app 3 is changed from the inactive state to the active state. Accordingly, at and after Step S207, the device 200 is shifted in a state of operating in an operation mode in which all the vendor app 1 to the vendor app 3 become active. Operations at and after Step S207 are similar to those at Steps S203-S206, for example.

Note that, the method in the embodiment is not limited to that to be applied to the information processing system 10 including the server system 100 and the device 200, but may be applied to an information processing apparatus. The information processing apparatus herein is the server system 100 in a narrow sense. The information processing apparatus operates in any of a plurality of operation modes, and includes a communicator (which corresponds to the communicator 130 in FIG. 3) that performs communication with the device 200 to be used in care assistance for a care receiver, and a processing unit (which corresponds to the processing unit 110 in FIG. 3, the ability information acquisition unit 111 in a narrow sense) that performs processing of obtaining ability information indicating an activity ability of the care receiver based on the sensing data transmitted from the device 200. Moreover, the processing unit of the information processing apparatus performs processing of transmitting ability information to the device 200 via the communicator, as information indicating in which operation mode among a plurality of operation modes the device 200 operates. In this way, it is possible to estimate transition of ability information on the care receiver based on the information collected from the device 200, and causes the device 200 to operate in accordance with the ability information.

Part or all of the processing that is performed by the information processing system in the embodiment may be implemented by a program. The processing that is performed by the information processing system is processing that includes at least one of processing that is performed by the processing unit 110 of the server system 100 and processing that is performed by the processing unit 210 of the device 200, for example. In addition, a part or all of the processing that is performed by the information processing apparatus in the embodiment may be implemented by a program.

A program according to the embodiment can be stored in a non-temporary information storage device (information storage medium) that is a medium readable by a computer, for example. The information storage device can be implemented by an optical disc, a memory card, an HDD, or a semiconductor memory, for example. The semiconductor memory is an ROM, for example. The processing unit 110 and the like perform various kinds of processing in the embodiment based on the program stored in the information storage device. In other words, the information storage device stores the program for causing a computer to function as the processing unit 110 and the like. The computer is an apparatus that is provided with an input device, a processing unit, a storing unit, and an output unit. Specifically, the program according to the embodiment is a program for causing the computer to execute the respective steps described above using FIGs. 6 and 7 and other drawings.

Moreover, the method in the embodiment can be applied to an information processing method in the information processing system 10 that operates in any of a plurality of operation modes, and includes the device 200 that is used in care assistance for a care receiver, and the server system 100 that is connected to the device 200 via the network. The information processing method includes a step of obtaining, based on sensing data acquired by the device 200, ability information indicating an activity ability of a care receiver, and a step of determining in which operation mode among a plurality of operation modes the device 200 operates based on the obtained ability information.

Hereinafter, a specific device 200 and processing that is executed in the device 200 will be described using the respective stages illustrated in FIG. 5 as an example.

### 2.2 Unable to Wake Up

### <Examples of Device And Motion: Falling-Down risk Determination>

Firstly, the device 200 for responding to a falling-down risk at the start of movement in a state where the difficulty of the wake-up motion has occurs will be described. FIGs. 8 and 9 each illustrate an example of the device 200 that is used for falling-down risk determination at the start of movement.

FIG. 8 is a diagram illustrating an example of an imaging device 410 that images care receivers, and an example of an output image IM1 from the imaging device 410. The imaging device 410 includes an image sensor that outputs a taken image as sensing data, in addition to the respective configurations illustrated in FIG. 4. The imaging device 410 may be disposed in a place where large number of persons work together, such as a living room, a hole, or the like in the care facility. In the example of FIG. 8, the imaging device 410 is disposed to an upper portion of a television device.

The processing unit 210 of the imaging device 410 may perform processing of detecting a movement start of a person based on the taken image. The processing unit 210 operates, for example, in accordance with an application to be installed on the imaging device 410, thereby acquiring a taken image as input data, and executing processing of detecting a person from the taken image and processing of determining the presence or absence of a movement start by the detected person.

For example, the imaging device 410 performs face recognition processing of recognizing a face of a person based on the taken image. For example, the storing unit 220 of the imaging device 410 stores a face image of a person serving as a detection target, and the processing unit 210 may perform the face recognition processing based on the matching processing that uses the face image as a template. Moreover, various kinds of methods for the face recognition processing are known, and are widely applicable in the embodiment. For example, if a state where the motion of the detected face region is equal to or less than a given threshold has continued for a certain period of time, the imaging device 410 sets a position of the face region in the state as a reference position. Moreover, the imaging device 410 may set a detection region at a position distant from the reference position by a predetermined distance, and determine that the start of movement has occurred if the face region has reached the detection region. For example, in a case where standing-up motion has been performed, it is assumed that the position of the face moves relatively upward, so that the abovementioned detection region may be a region to be set upward from the reference position by a predetermined distance. In this case, in a cases where the position of the face region on the image has moved in the upper direction relative to the reference position by equal to or longer than a predetermined distance, a movement start is detected. Note that, the detection region herein is a line-shaped region, for example, but a region having another shape may be set.

Moreover, the imaging device 410 can specify a target care receiver by the face recognition processing. Therefore, the imaging device 410 may perform the detection of a movement start with respect to a care receiver having an ability indicated by the ability information equal to or less than a predetermined threshold (which corresponds to being unable to perform a wake-up motion), and omit the detection of a movement start with respect to a care receiver having an ability higher than the threshold.

Moreover, the detection processing of a movement start is not limited to the abovementioned method. For example, the imaging device 410 may perform skeleton tracking processing based on the taken image. Note that, for the method of skeleton tracking based on an image, various methods such as OpenPose disclosed in "Realtime Multi-Person 2D Pose Estimation using Part Affinity Fields" (https://arxiv.org/pdf/1611.08050.pdf) by Zhe Cao et al., have been known, and these methods can be widely employed in this embodiment.

Moreover, OpenPose described above discloses the method of performing the skeleton tracking for each of multiple persons taken in an image and displaying its result. In the example of FIG. 8, the image sensor outputs a taken image including three care receivers, and the imaging device 410 determines the presence or absence of a movement start with respect to each of the three care receivers.

For example, there is a possibility that a care receiver who is difficult to wake up due to the lowering in the ability falls down even only when taking a standing-up posture. Accordingly, the imaging device 410 may determine whether the care receiver is taking the standing-up posture using the skeleton tracking. For example, if the imaging device 410 has determined that the care receiver leans forward from the sitting posture with his/her hands placed on his/her knees, the seat surface of a chair, and the like, the imaging device 410 determines that the care receiver is taking the standing-up posture and notifies a care giver of the falling-down risk. For example, if the imaging device 410 has detected from the skeleton tracking result that a distance between the position of his/her hands and the position of his/her knees is equal to or less than a predetermined distance, and the position of shoulders moves downward by equal to or more than a predetermined length, the imaging device 410 may determine that the care receiver takes a standing-up posture.

Alternatively, while sectioning data to be processed into windows on a several-seconds basis, the imaging device 410 may determine that a posture change such as standing up occurs if the position of a specific portion such as the head or neck moves in each window by a predetermined threshold or more. Note that, the portion whose movement is to be detected may be other than the head and neck. In addition, the movement direction may be vertical, horizontal, or diagonal. Further, a threshold used for detection may be changed in accordance with the portion to be detected. Alternatively, the imaging device 410 may obtain a region including feature points detected by skeleton tracking with respect to a care receiver in a static state, and determine that a movement starting motion such as standing-up has occurred if the feature points equal to or more than the predetermined number have got out of the region. In addition, various kinds of modifications are executable for the method of the movement start detection processing using the imaging device 410.

IM1 in FIG. 8 is an example of an output image from the imaging device 410. The imaging device 410 may superimposed display a some sort of a display object on the taken image. In the example of FIG. 8, an object including a "!" mark is displayed in association with a care receiver whose movement start has been detected. In this way, it is possible to clearly notify a care giver of the care receiver whose movement start has been detected. For example, at Step S203 in FIG. 7, the imaging device 410 transmits the output image IM1 to the server system 100. At Step S204, the server system 100 outputs the output image IM1 to a smartphone or the like that is used by the care giver. Further, the output from the imaging device 410 may be information specifying a care receiver whose movement start has been detected (for example, an ID of the care receiver), and various kinds of modifications are executable in a specific mode. For example, the example in which a care receiver whose movement start has been detected is notified is illustrated, but information for stopping the movement by a care receiver may be output. For example, the imaging device 410 may specify a care receiver who has started the movement, and output voice data, moving image data, and the like of a family, and the like of the care receiver. Especially, in a case of a dementia patient, a response to calling is dull, but the dementia patient remembers a face and voice of the family and the like in many cases, and those outputs are effective for stopping the movement. The movement of the care receiver is stopped in this way, so that it is possible to gain time before a care giver interposes. Note that, the example in which the voice data or the moving image data of the family is output is described later in relation to the device control based on scene information.

FIG. 9 is a diagram illustrating an example of a bed side sensor 420 and the detection device 430 that are disposed to sections of the bed 610. The bed side sensor 420 and the detection device 430 are each a sheet-shaped or plate-shaped device 200 that is provided between the sections of the bed 610 and a mattress 620, for example, as illustrated in FIG. 9. Note that, FIG. 9 illustrates both of the bed side sensor 420 and the detection device 430, but only either one may be used. Moreover, as will be described hereinafter, the bed side sensor 420 and the detection device 430 are common in that both include pressure sensors, so that the bed side sensor 420 may also serve as the detection device 430 or the detection device 430 may also serve as the bed side sensor 420. In addition, various kinds of modifications are executable in a specific mode.

The bed side sensor 420 includes a pressure sensor that outputs a pressure value as sensing data, and is disposed to the sections at the side that a care giver uses for going up to and get out from the bed. In the example of FIG. 9, the care giver uses a near side of the bed 610 for going up and getting out. At this time, as illustrated in FIG. 9, fall prevention fences may be disposed at the near side of the bed 610, and the bed side sensor 420 may be disposed in a position at which the fence is not provided. In this way, a user who goes up to and gets out from the bed 610 temporarily performs a sitting motion on the bed side sensor 420.

The processing unit 210 of the bed side sensor 420 operates in accordance with, for example, an application to be installed on the bed side sensor 420, thereby acquiring a pressure value as input data, and executing processing of determining a movement by the care receiver on the bed 610 from the pressure value.

For example, when the care receiver stands up from the bed 610, it is assumed that the care receiver is shifted from a state of taking a lying position on the bed to a state of taking a sitting position on the bedside (hereinafter, expressed as an edge sitting position), and further puts his/her hand on the knee or the section surface to apply a force thereto, thereby executing a standing-up motion . The lying position, the edge sitting position, and the standing-up motion are in descending order of the pressure value to be detected by the bed side sensor 420. For example, the bed side sensor 420 may determine that a movement start has been detected in a case where the bed side sensor 420 has detected a change from the edge sitting position to the standing-up motion based on the comparison processing between a pressure value and a given threshold. Alternatively, from the viewpoint of detecting a standing-up motion at an earlier stage, the bed side sensor 420 may determine that a movement start has been detected in a case where the bed side sensor 420 has detected a change from the lying position to the edge sitting position based on the comparison processing between a pressure value and a given threshold.

Alternatively, if the standing-up motion is continued, the buttocks of the care receiver float up from the section surface, so that a pressure value to be output from the pressure sensor largely decreases. Therefore, in a case where based on a time-series change in the pressure value, the pressure value has increased to be equal to or more than a first threshold, and then has decreased to be equal to or less than a second threshold smaller than the first threshold, the processing unit 210 may determine that the standing-up motion has been performed. In addition, various kinds of modifications are executable for the specific processing content of the movement start determination.

In a case where the movement start by the care receiver has been detected, the bed side sensor 420 transmits information indicating the fact to the server system 100. The server system 100 transmits the information to a smartphone or the like that is used by a care giver, for example, and the notification processing is executed in the smartphone or the like. In this way, it is possible to clearly notify a care giver of the care receiver whose movement start has been detected.

Moreover, the detection device 430 illustrated in FIG. 9 is the device 200 that senses information related to sleep in a care receiver. The detection device 430 includes a pressure sensor that outputs a pressure value.

The detection device 430 detects, when a user goes to bed, body vibration (body movement, vibration) of the user via the mattress 620. Based on the body vibration detected by the detection device 430, information related to a respiratory rate, a heartbeat rate, an amount of activity, a posture, awake/sleep, and bed departure/bed presence is obtained. Moreover, the detection device 430 may determine non REM sleep and REM sleep, and a determination of a sleep depth. For example, the periodicity of the body movement is analyzed, and a respiratory rate and a heartbeat rate may be calculated from the peak frequency. The analysis of the periodicity is a Fourier transform, for example. The respiratory rate is the frequency of breathing per unit time. The heartbeat rate is the frequency of heartbeats per unit time. The unit time is one minute, for example. Moreover, body vibration is detected per sampling unit time, and the frequency of the detected body vibration may be calculated as an amount of activity. Moreover, the pressure value to be detected decreases in the bed departure of the user compared with in the bed presence, so that a determination of bed departure/bed presence is possible based on the pressure value and a time-series change in the pressure value.

For example, the processing unit 210 of the detection device 430 may determine that a movement start has been detected based on a determination result of bed departure/bed presence, in a case where a care receiver is shifted from the bed presence to the bed departure.

In a case where the movement start by the care receiver has been detected, the detection device 430 transmits information indicating the fact to the server system 100. The server system 100 transmits the information to a smartphone or the like that is used by a care giver, for example, and the notification processing is executed in the smartphone or the like. In this way, it is possible to clearly notify a care giver of the care receiver whose movement start has been detected.

For example, each of the devices 200 illustrated in FIGs. 8 and 9 is set to an operation mode 0 indicating an inactive state in a case where an ability of a care receiver that is indicated by the ability information is equal to or more than a predetermined ability, and is set to an operation mode 1 indicating an active state in a case where the ability is less than the predetermined ability. The ability less than the predetermined ability indicates herein that the care receiver is unable to perform the wake-up motion. In this way, the abovementioned determination operation of the movement start can be executed in an appropriate situation. As a result, in a case where a care receiver having a high falling-down risk is present, a falling-down risk of the care receiver can be appropriately reduced.

### <Update Ability Information>

Moreover, as illustrated at Step S205 in FIG. 7, the ability information acquisition unit 111 of the server system 100 may perform processing of updating ability information based on the sensing data. For example, the ability information acquisition unit 111 determines a change in a state related to waking-up based on the sensing data.

For example, the ability information acquisition unit 111 may determine a way of standing-up based on the sensing data. When a care receiver stands up, as mentioned above, the care receiver executes a motion of placing his/her hands on the section surface or the like from the edge sitting position, further applying the body weight to his/her legs, and straightening up his/her back while straightening up his/her knees. At this time, in a case where the body weight movement to the legs is not sufficient, the center of gravity is relatively deviated backward, and the care receiver is in a state of flopping down into the section surface. Moreover, in a case where the body weight movement to the legs is excessive, the center of gravity is deviated forward, and thus the care receiver may fall down in the front. Moreover, if the care receiver sits too forward in the edge sitting position, there is also a possibility that his/her buttocks may be fell from the section surface.

Therefore, the ability information acquisition unit 111 may determine, based on the log of the skeleton tracking by the imaging device 410 or the log of the pressure value from the bed side sensor 420 or the detection device 430, whether the body movement of the care receiver at the time of standing-up is appropriate. For example, a higher ability is determined as the movement at the time of standing-up is closer to a normal state, and a lower ability is determined as the deviation of the center of gravity, the position of the buttocks in the edge sitting position, and the like are shifted more relative to the normal state.

Moreover, the ability information acquisition unit 111 may obtain ability information based on the number of times of standing-up motions executed within a predetermined period. For example, a higher ability is determined as the number of times of standing-up is more, and a lower ability is determined as the number of times is less.

Moreover, the ability information acquisition unit 111 may obtain ability information based on an elapsed time from when a care receiver takes the edge sitting position to when the care receiver stands up. The lying position, the edge sitting position, and the standing position can be determined based on a positional relationship of feature points in the skeleton tracking or a time-series change in the pressure value. For example, a higher ability is determined as an elapsed time from when a care receiver takes the edge sitting position to when the care receiver stands up is shorter, and a lower ability is determined as the elapsed time is longer.

Moreover, the ability information acquisition unit 111 may obtain ability information based on the amount of activity in the bed 610. The amount of activity is detected by the detection device 430, for example, as mentioned above. Moreover, the amount of activity may be obtained based on a result by the skeleton tracking or an output from the bed side sensor 420. For example, a higher ability is determined as the amount of activity is more, and a lower ability is determined as the amount of activity is less.

In the method in the embodiment, the determination of ability information may be executed using any one among the abovementioned elements, or two or more elements may be combined. Moreover, as mentioned above, ability information may be obtained based on a combination of sensing data and data other than the sensing data. With the method in the embodiment, it is possible to reduce a falling-down risk of a care receiver who cannot perform a wake-up motion, and appropriately determine a change in ability information on the care receiver.

### 2.3 Unable to Walk

### <Examples of Device And Motion: Fall Risk>

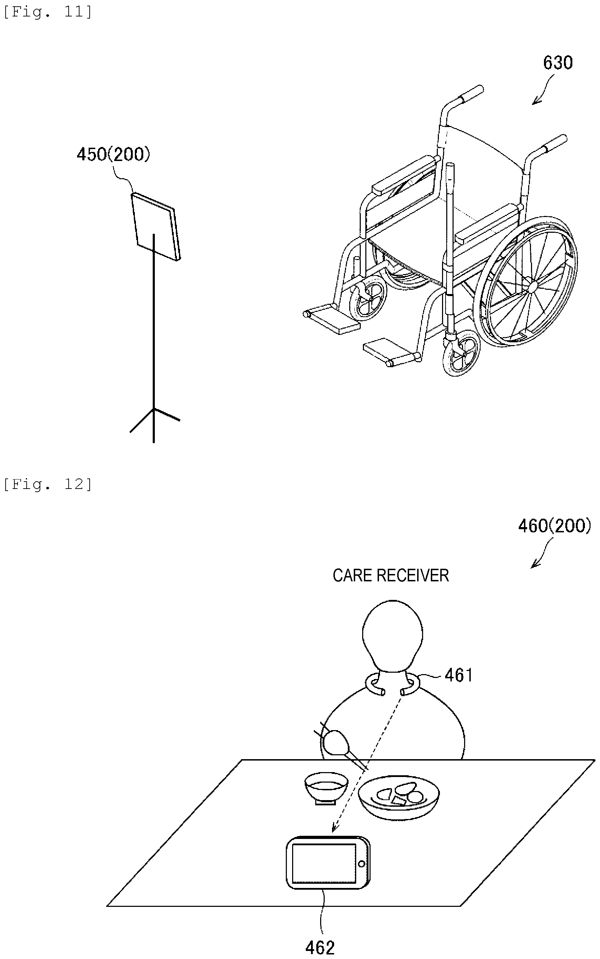

Firstly, the device 200 for responding to a fall risk from the wheelchair 630 or the like in a state where the difficulty of walking has occurs will be described. FIGs. 10 and 11 each illustrate an example of the device 200 that is used for fall risk determination.

FIG. 10 is a diagram illustrating the seat surface sensor 440 serving as the device 200 that is disposed on a seat surface of the wheelchair 630. The seat surface sensor 440 includes a pressure sensor that outputs a pressure value, and determines, based on the pressure value, a posture (hereinafter, also described as a seated posture) of the care receiver when being seated on the wheelchair 630 is which one of a plurality of postures including normal, forward displacement, lateral displacement, and other postures. The forward displacement indicates a state where the center of gravity of a user is shifted in the front direction compared with the normal state, and the lateral displacement indicates a state where the center of gravity of the user is shifted in either one of left and right directions compared with the normal state. Both of the forward displacement and the lateral displacement correspond to a state where a risk of slipping off is relatively high. Note that, the seat surface sensor 440 may be a sensor device that is disposed to a normal chair, or may be a sensor device that determines a posture of a user who is seated on the bed or the like. Moreover, the seat surface sensor 440 may perform the determination of a fall possibility to determine the presence or absence of a possibility that a care receiver falls from the seat surface.

In the example in FIG. 10, four pressure sensors Se1 to Se4 are disposed on a back surface side of a cushion 441 that is disposed to the seat surface of the wheelchair 630. The pressure sensor Se1 is a sensor that is disposed in front, the pressure sensor Se2 is a sensor that is disposed rearward, the pressure sensor Se3 is a sensor that is disposed rightward, and the pressure sensor Se4 is a sensor that is disposed leftward. Note that, the front, rear, left, and right directions herein indicate directions seen from a care receiver in a state where the care receiver is seated on the wheelchair 630.

As illustrated in FIG. 10, the pressure sensors Se1 to Se4 are connected to a control box 442. The control box 442 includes a processor that controls the pressure sensors Se1 to Se4, and a memory that serves as a work area of the processor, in an inside thereof. For example, the processor corresponds to the processing unit 210, and the memory corresponds to the storing unit 220. The processor detects a pressure value by causing the pressure sensors Se1 to Se4 to operate.

A care receiver seated on the wheelchair 630 may feel pain on his/her buttocks and displace the position of his/her buttocks. For example, forward displacement indicates a state where his/her buttocks are displaced forward and lateral displacement indicates a state where his/her buttocks are displaced laterally, relative to the normal state. In addition, there may be a case where the forward displacement and the lateral displacement occur at the same time and his/her center of gravity is displaced obliquely. As illustrated in FIG. 10, by using the pressure sensors disposed on the cushion 441, it is possible to detect a change in the position of the buttocks appropriately, and thus detect the forward displacement and the lateral displacement precisely.

For example, it is assumed that the timing when a care receiver transfers to the wheelchair 630 and takes a normal posture is set as an initial state. In the initial state, since the care receiver sits deeply on the seat surface of the wheelchair 630, the value of the pressure sensor Se2 located rearward is supposed to be relatively large. On the other hand, if the forward displacement occurs, the position of his/her buttocks moves forward, and thus the value of the pressure sensor Se1 located forward increases. For example the processor of the control box 442 may determine that the forward displacement has occurred in a case where the value of the pressure sensor Se1 has increased by equal to or more than a predetermined value compared with that in the initial state. It is determined that a care receiver is on the wheelchair 630 if the value of the pressure sensor Se1 exceeds a given threshold, and the occurrence of forward displacement may be determined only based on a change in the value of the pressure sensor Se2, without comparing with the pressure sensor Se1. Alternatively, instead of using the value of the pressure sensor Se1 by itself, processing may be performed using the relationship between the value of the pressure sensor Se2 and the value of the pressure sensor Se1. For example, a difference between voltage values which are outputs from the pressure sensor Se2 and the pressure sensor Se1 may be used, the ratio of the voltage values may be used, or the rate of change in the difference or ratio relative to that in the initial state may be used.

Likewise, if the lateral displacement occurs, the position of his/her buttocks moves leftward or rightward, and thus the value of the pressure sensor Se4 increases in the case of the leftward displacement and the value of the pressure sensor Se3 increases in the case of the rightward displacement. Accordingly, the processor may determine that the leftward displacement occurs if the value of the pressure sensor Se4 increases by a predetermined amount or more compared with that in the initial state, and may determine that the rightward displacement occurs if the value of the pressure sensor Se3 increases by a predetermined amount or more compared with that in the initial state. Alternatively, the processor may determine rightward displacement and leftward displacement using a relationship between the values of the pressure sensor Se4 and the pressure sensor Se3. Similar to the example of the forward displacement, a difference between voltage values which are outputs from the pressure sensor Se4 and the pressure sensor Se3 may be used, the ratio of the voltage values may be used, or the rate of change in the difference or ratio relative to that in the initial state may be used.

In a case where forward displacement, lateral displacement, and the like have been detected, the seat surface sensor 440 transmits information indicating the fact to the server system 100. The server system 100 transmits the information to a smartphone or the like that is used by a care giver, for example, and the notification processing is executed in the smartphone or the like. Moreover, the control box 442 may include a light emitting unit or the like, and the notification to the care giver may be performed using the light emitting unit. In this way, a change in the sitting posture on the wheelchair 630 or the like can be clearly notified to the care giver, so that it is possible to prevent the care receiver from falling down.

FIG. 11 is a diagram illustrating a terminal device 450 that is the device 200 for adjustment support of a wheelchair position. The wheelchair position is information related to a position and a posture of a care receiver in the wheelchair 630. The wheelchair position may indicate the abovementioned sitting posture, or may indicate information including the arrangement of a cushion and the like. As illustrated in FIG. 11, for the wheelchair position adjustment, the terminal device 450 that includes a camera and is fixed at a height such that the camera can take an image of at least the upper body of a care receiver who is seated on the wheelchair 630 may be used. Note that, the terminal device 450 may be capable of taking an image in a larger range of the care receiver, and may take an image of the care receiver from the top to knees or may take an image of the whole body of the care receiver, for example. For example, the terminal device 450 is disposed at a predetermined position of a care facility, and a care giver performs the wheelchair position adjustment after moving the care receiver to the wheelchair 630 and moving him/her to the front of the terminal device 450.

The terminal device 450 includes a display that displays a comparison result between an image taken by the camera and labeled training data. The labeled training data herein is, for example, image data obtained such that a care giver having a high degree of proficiency takes an image of a care receiver in a state of being seated on the wheelchair 630 in an appropriate posture. For example, the labeled training data is registered in advance using the terminal device 450 and the like. Moreover, the labeled training data may be registered in the storing unit 120 of the server system 100. Moreover, the labeled training data is not limited to image data itself indicating an appropriate posture, but may be data to which additional information is added. The additional information herein may be information indicating a point that is considered to be important by a care giver having a high degree of proficiency. Moreover, the labeled training data may be a result by the skeleton tracking.

The terminal device 450 (the processing unit 210) may output an image in which labeled training data subjected to transmission processing is superimposed and displayed on an actual taken image. Moreover, the terminal device 450 may determine whether a position of a care receiver in the wheelchair 630 is appropriate based on the comparison processing between the labeled training data and the actual taken image, and output a determination result thereof. Moreover, in a case where the position of the care receiver is inappropriate, the terminal device 450 may present a point to be corrected. The point to be corrected is, for example, a site where a difference between the labeled training data and the actual taken image is equal to or more than a predetermined value.

For example, each of the devices 200 illustrated in FIGs. 10 and 11 is set to an operation mode 0 indicating an inactive state in a case where an ability of a care receiver that is indicated by the ability information is equal to or more than a predetermined ability, and is set to an operation mode 1 indicating an active state in a case where the ability is less than the predetermined ability. The ability less than the predetermined ability indicates herein that the care receiver is unable to walk. In this way, the abovementioned position determination in the wheelchair 630 or the like can be executed in an appropriate situation. As a result, in a case where a care receiver having a high fall risk from the wheelchair 630 or the bed 610 is present, the fall risk of the care receiver can be appropriately reduced.

### <Motion of Device Related to Falling-Down Risk>

Moreover, as illustrated in FIG. 5, even a care receiver who is unable to walk but is not bedridden has a high falling-down risk because there is a possibility that the care receiver performs a movement starting motion such as standing-up. The movement start detection by each of the devices 200 illustrated in FIGs. 8 and 9 is preferably continued even for a care receiver who is unable to walk.

At that time, in a state where a care receiver is unable to wake up but able to walk and a state where a care receiver is unable to walk, the ability is lower and a falling-down risk is higher in the latter in some cases. Therefore, each of the devices 200 illustrated in FIGs. 8 and 9 may operate in the operation mode 1 in a state where a care receiver is unable to wake up but able to walk, and operate in an operation mode 2 different from the operation mode 1 in a state where a care receiver is unable to walk. For example, in a case where the detection device 430 is used as the device 200, the processing unit 210 of the detection device 430 may detect a movement start in the operation mode 1 based on the determination result of bed departure/bed presence, and detect a movement start in the operation mode 2 based on the determination result of awake/sleep. For example, in the operation mode 2, the processing unit 210 determines that a possibility of the movement start is present in a state where the sleep state is shifted to the awake state. In this way, the movement start can be detected at an earlier stage in the operation mode 2 than the operation mode 1, so that it is possible to further reduce a falling-down risk.

### <Update Ability Information>

Moreover, as illustrated at Step S205 in FIG. 7, the ability information acquisition unit 111 of the server system 100 may perform processing of updating ability information based on the sensing data. For example, the ability information acquisition unit 111 determines a change in a sitting posture holding ability that is an ability maintaining a sitting posture state based on the sensing data.

For example, the ability information acquisition unit 111 may determine the number of times of forward displacement or lateral displacement based on the sensing data. For example, a higher sitting posture holding ability is determined as the number of times of forward displacement or lateral displacement is less, and a lower sitting posture holding ability is determined as the number of times is less.

Moreover, the ability information acquisition unit 111 may obtain a sitting posture holding ability based on the time during when the posture can be held (hereinafter, expressed as posture holding time). The posture holding time is the length of a period from a start point that is the timing when a care receiver has taken a given reference posture, for example, to an end point that is the timing when the posture of the care receiver has changed by equal to or more than a predetermined amount relative to the reference posture. The posture holding time may be, for example, the time from when a care giver has corrected the posture of the care receiver using the terminal device 450 to when forward displacement or lateral displacement is determined by the seat surface sensor 440. For example, a higher sitting posture holding ability is determined as the posture holding time is longer, and a lower sitting posture holding ability is determined as the posture holding time is shorter. Moreover, a similar posture can be maintained if the sitting posture holding ability is high, so that a state where the pressure is equally applied to each of the pressure sensors is continued. On the other hand, when the sitting posture holding ability is decreased, the body is frequently inclined or the posture is corrected even if neither forward displacement nor lateral displacement has been determined. As a result, the timing when the pressure value decreases (decline in pressure) easily occurs. Therefore, the ability information acquisition unit 111 may estimate a sitting posture holding ability based on the number of times and the frequency, the degree of the decrease in the value, the direction of the decline (the value of which of the pressure sensors Selto Se4 has decreased), and the like, of such decline in pressure.

In the method in the embodiment, the determination of ability information may be executed using any one among the abovementioned elements, or two or more elements may be combined. Moreover, as mentioned above, ability information may be obtained based on a combination of sensing data and data other than the sensing data. With the method in the embodiment, it is possible to reduce a fall risk of a care receiver who is unable to walk, and appropriately determine a change in ability information on the care receiver.

### 2.4 Unable to Eat Meal Successfully

### <Examples of Device And Motion: Aspiration Risk>

Next, the device 200 that becomes an operation state in a state where a care receiver has been unable to eat a meal successfully, in order to respond to an aspiration risk (aspiration pneumonitis risk in a narrow sense) will be described. FIG. 12 illustrates an example of the device 200 that is used for aspiration risk determination in the meal.

FIG. 12 is a diagram illustrating an example of a swallowing and choking detection device 460 serving as the device 200 that is used in a meal scene. As illustrated in FIG. 12, the swallowing and choking detection device 460 includes a throat microphone 461 that is attached around a neck of a care receiver, and a terminal device 462 including a camera. Note that, another device having a camera may be used instead of the terminal device 462. The throat microphone 461 outputs audio data due to swallowing, coughing, and the like by a care receiver. The camera of the terminal device 462 outputs a taken image of a meal state of the care receiver. For example, the terminal device 462 is a smartphone or the like that is placed on a table where the care receiver is taking a meal. The throat microphone 461 is connected to the terminal device 462 using Bluetooth (registered trademark) and the like, and the terminal device 462 is connected to the server system 100 via the gateway 300. Further, both of the throat microphone 461 and the terminal device 462 may be connectable to the gateway 300, and various kinds of modifications are executable for a specific connection aspect.

For example, a processor included in the swallowing and choking detection device 460 acquires voice data from the throat microphone 461, and a taken image taken using the camera. The processor herein corresponds to the processing unit 210, and may be a processor included in the terminal device 462, for example.

The processor determines choking and swallowing of the care receiver based on the voice data from the throat microphone 461. A device of detecting swallowing using a microphone attached around a neck is described in U.S. Patent Application No. 16/276768, filed on 15 February, 2019, "Swallowing action measurement device and swallowing action support system", for example. This patent application is fully incorporated in the specification of the present application by reference. The processor can detect the frequency of choking, choking time (occurrence time, continuous time, and the like), and whether swallowing has been made, based on the voice data.

In addition, as illustrated in FIG. 12, for example, the camera of the terminal device 462 can detect the mouth and eyes of a care receiver, and chopsticks, a spoon, and the like to be used by the care receiver by taking images of the care receiver in the front direction. Note that, various kinds of methods of detecting these parts of the face, and the objects based on the image processing are known, and the publicly known methods are widely applicable in the embodiment.

For example, based on images taken by the camera, the processor can determine whether the mouth of the care receiver is open, whether food is spilling out of the mouth of the care receiver, and whether the care receiver is chewing food. In addition, based on the images taken by the camera, the processor can determine whether the eyes of the care receiver are open. Further, based on the images taken by the camera, the processor can determine whether the chopsticks, the spoon, and the like are near dishes, whether the care receiver can hold them, and whether the care receiver is spilling food.

In the method in the embodiment, based on these information, a situation related to swallowing and choking by a care receiver is estimated. For example, the processor may perform processing based on detection results of choking and swallowing, and an opening and closing determination result of the mouth of the care receiver.

For example, the processor may determine whether choking occurs frequently based on the number of times and the time of choking, and may output a determination result. For example, the processor may determine that choking occurs frequently if the number of times of choking per unit time exceeds a threshold. In this way, the situation related to the choking can be automatically determined.

For example, the processor may obtain swallowing time from when a care receiver opens his/her mouth to when he/she swallows, based on a detection result of the swallowing, and an opening and closing determination result of the mouth of the care receiver. In this way, even if it is found that the number of times of swallowing is reduced, for example, a specific situation can be determined such as a situation where a care receiver does not even perform an action of putting food into his/her mouth or a situtiona where the care receiver has put food into his/her mouth but does not swallow it. For example, the processor may start counting up with a timer when it is found based on images taken by the terminal device 462 that a care receiver transitions from a state of closing his/her mouth to a state of opening his/her mouth, and stop the measurement with the timer when swallowing is detected by the throat microphone 461. The time when the timer stops represents the swallowing time. This makes it possible to precisely determine whether a care receiver is in a situation where the aspiration risk is high in the meal and a care giver should execute some sort of action, and thus possible to use the tacit knowledge of a skilled worker appropriately.

For example, the swallowing and choking detection device 460 may output the swallowing time as a processing result to the server system 100. Moreover, the processor may determine a pace of the meal based on the swallowing time. Note that, the processor may determine whether the swallowing time is long based on a change in the swallowing time during one meal (such as the amount of increase and the ratio of the swallowing time with respect to those in the initial phase). Alternatively, the processor may obtain average swallowing time etc. of a single care receiver for each meal in a plurality times of meals, and determine whether the swallowing time becomes longer based on a change in the average swallowing time.

For example, by using the result of determination on whether the mouth of a care receiver is open or closed based on images taken by the terminal device 462, it is possible to determine whether the care receiver is in a situation of no longer opens his/her mouth even if a care giver brings a spoon and the like closer to the care receiver. If the swallowing time becomes longer under a situation where the care receiver is not willing to open his/her mouth, it is possible to presume that the care receiver is in a situation where accumulation in which food remains in his/her mouth has occurred. In addition, by using the result of mouth recognition using taken images, i.e., whether food is spilling out of the mouth of the care receiver and whether the care receiver is chewing food, it is possible to determine whether the care receiver is in a situation where the care receive is no longer able to chew off food. For example, if the swallowing time is long although the number of times of chewing is as usual, it is possible to presume that the care receiver is in a situation where the care receiver is no longer able to chew off food. Meanwhile, if it has been determined using taken images that the eyes of the care receiver are closed, it is possible to determine that the care receiver is in a situation of becoming sleepy.

In addition, through processing of recognition of chopsticks, a spoon, and the like using taken images, it may be determined whether a care receiver is in any of situations such as playing with food, not being able to hold a dish in his/her hands, and spilling food.

As in the foregoing, various kinds of situations in the meal can be determined using the swallowing and choking detection device 460. In the swallowing and choking detection device 460 of input data in the embodiment, each of these determinations may be implemented as an application corresponding to tacit knowledge. For example, the swallowing and choking detection device 460 may include an application that detects choking and swallowing and calculates swallowing time, an application that detects the frequent occurrence of choking, an application that detects dangerous choking, an application that determines a sleepy situation, an application that determines playing with food, and the like. Moreover, in the embodiment, an active/inactive state of each application is controlled based on ability information and the like. In other words, execution/non-execution of the abovementioned various kinds of processing may be able to be flexibly settable. Moreover, in a case where a predetermined situation has been determined, the swallowing and choking detection device 460 transmits information indicating the fact to the server system 100. The server system 100 may cause a terminal device that is used by a care giver to execute the notification processing based on the information, for example. Moreover, the server system 100 may output information indicating an appropriate action in accordance with a meal situation detected by the swallowing and choking detection device 460 to a device that is used by a care giver, and the device may present an action based on the information. For example, the action may be presented to a care giver by outputting voice to a headset, may be presented by displaying it on the display of the smartphone or the like, or may be presented using other methods.

### <Motion of Device Related to Falling-Down Risk>

Moreover, as illustrated in FIG. 5, even a care receiver who is difficult to take a meal has a high falling-down risk because there is a possibility that the care receiver performs a movement starting motion such as standing-up. Therefore, also in a case for the care receiver who is difficult to take a meal, the movement start detection by each of the devices 200 illustrated in FIGs. 8 and 9 is preferably continued. Each of the devices 200 may operate in the abovementioned operation mode 2, for example. Moreover, each of the devices 200 may operate in an operation mode 3 in which the movement start detection is possible at an earlier stage than the operation mode 2.

### <Operation of Device Related to Fall Risk>

Moreover, even a care receiver who is difficult to take a meal has a high fall risk because it can be considered that the care receiver uses the wheelchair 630 or the like. Therefore, even in a case for the care receiver who is difficult to take a meal, the processing related to a wheelchair position by each of the devices 200 illustrated in FIGs. 10 and 11 is preferably continued. For example, each of the devices 200 operates in the operation mode 1, similar to the case for a care receiver who is unable to walk. Moreover, each of the devices 200 may operate in a mode different from that in the case for a care receiver who is unable to walk. For example, each of the devices 200 may operate in an operation mode in which only the determination of forward displacement/lateral displacement is performed in a case for a care receiver who is unable to walk, and operate in an operation mode in which the determination of a fall possibility is executable in addition to the determination of forward displacement /lateral displacement in a case for the care receiver who is difficult to take a meal.

### <Update Ability Information>

Moreover, as illustrated at Step S205 in FIG. 7, the ability information acquisition unit 111 of the server system 100 may perform processing of updating ability information based on the sensing data. For example, the ability information acquisition unit 111 determines a change in a swallowing ability based on the sensing data.

For example, the ability information acquisition unit 111 may determine a higher swallowing ability as the swallowing time from when a care receiver open his/her mouth to when the care receiver swallows is shorter, and determine a lower swallowing ability as the swallowing time is longer. For example, the ability information acquisition unit 111 may determine that the swallowing ability has decreased when the most recent average swallowing time of a care receiver becomes longer by a threshold or more than the past average swallowing time of the care receiver, determine that the swallowing ability is not decreased (maintained) when the average swallowing time does not become longer by the threshold or more, and determine that the swallowing ability has recovered when the average swallowing time becomes shorter. Moreover, if a change in the average swallowing time is equal to or less than the threshold before and after the eating pattern is changed, the ability information acquisition unit 111 may determine that the swallowing ability has recovered. Moreover, the ability information acquisition unit 111 may obtain ability information based on a combination of the swallowing ability and the sitting posture holding ability. For example, the ability information acquisition unit 111 may obtain ability information based on the use time and the use frequency of the wheelchair 630. For example, a care receiver whose sitting posture holding ability has decreased is shifted to the meal on the bed in some cases, so that the care receiver who can use the wheelchair 630 indicates a high sitting posture holding ability. For example, a higher ability is determined as the use frequency of the wheelchair is higher, and a lower ability is determined as the use frequency is low. Moreover, as mentioned above, the swallowing and choking detection device 460 may be able to execute various kinds of applications that execute different processing. For example, the swallowing and choking detection device 460 firstly makes an application that detects choking and swallowing and calculates the swallowing time to be active, and the ability information acquisition unit 111 may obtain ability information based on an output from the application. Moreover, in a case where the ability information has changed, another application such as an application that detects the frequent occurrence of the choking is changed from inactive to active. In this way, the processing content to be executed in the swallowing and choking detection device 460 can be appropriately controlled based on the ability information. Moreover, in a case where the number of active applications has increased, the ability information acquisition unit 111 may obtain a swallowing ability based on a larger number of sensing data by combining outputs from the respective applications.

### 2.5 Bedridden

### <Examples of Device And Motion: Bedsore Risk>

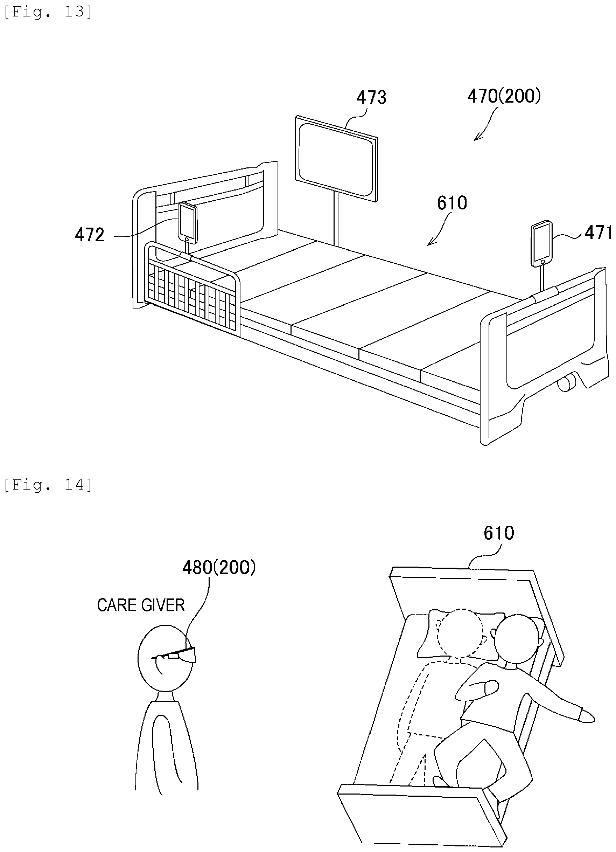

Next, the device 200 that operates for responding to a bedsore risk in a state where the ability is further lowered, and a care receiver has become a bedridden state will be described. FIGs. 13 and 14 illustrate an example of the device 200 that is used for bedsore risk determination.

FIG. 13 is a diagram illustrating an example of a bed position detection device 470 that is the device 200 to be disposed in the periphery of the bed 610. As illustrated in FIG. 13, the bed position detection device 470 includes a first terminal device 471 that is fixed to the bed 610 at a foot board side, a second terminal device 472 that is fixed to a side rail of the bed 610, and a display 473 that is fixed at an opposite side of the second terminal device 472. In addition, the display 473 is not limited to one that is fixed on the bed 610, and may be disposed at another position such that a care giver who adjusts the bed position can naturally view it. For example, the display 473 may be fixed on the wall surface or may be fixed, for example, to a support that stands on the floor surface on its own. Note that, the display 473 can be omitted. Moreover, either one of the first terminal device 471 and the second terminal device 472 may be omitted. For example, an example in which the first terminal device 471 is used for the adjustment of a bed position, and the second terminal device 472 is used for diaper changing is described hereinafter, but the embodiment is not limited thereto.

The first terminal device 471 and the second terminal device 472 are devices such as a smartphone having a camera. The first terminal device 471 transmits a taken image to the server system 100 directly. The second terminal device 472 transmits, directly or via the first terminal device 471, an image taken by the camera to the server system 100. The display 473 receives, directly or via another device such as the first terminal device 471, the image transmitted by the server system 100 and displays the image thus received. Note that, the first terminal device 471 and the second terminal device 472 may have a depth sensor instead of or in addition to the camera. In other words, these devices may each output a depth image.

For example, in the bed position adjustment, the processing of registering labeled training data and the position adjustment processing using the labeled training data may be executed. The labeled training data is information registered by a skilled care giver, for example. A care giver who is an unskilled worker selects labeled training data when adjusting the bed position, and adjusts the bed position such that an actual state of a care receiver becomes closer to that of the labeled training data. For example, the first terminal device 471 acquires an image in which a state where a care receiver whose bed position is to be adjusted is lying on the bed (including a state of a cushion and the like) is taken, and the display 473 displays an image representing a comparison result between the taken image and the labeled training data. This enables the care giver to perform the position adjustment in the same way as a skilled worker irrespective of the degree of proficiency of the care giver. For example, a skilled worker lays a care receiver on the bed 610, places him/her at a position preferable for measures against bedsore etc., and takes an image of the target care receiver using the first terminal device 471. The skilled worker selects a registration button after confirming that the care receiver is placed at an appropriate bed position. Accordingly, a still image displayed when the registration button has been operated is transmitted to the server system 100 as labeled training data. This makes it possible to register a position, which the skilled worker thinks is preferable, as labeled training data. At this time, additional information may be added, which is similar to the abovementioned example of the wheelchair position.

Meanwhile, when a care giver actually adjusts the bed position, the care giver first activates the first terminal device 471 and starts taking images. For example, a care giver activates the first terminal device 471 by voice, and the display 473 displays a moving image that the first terminal device 471 is taking. Moreover, the bed position detection device 470 may receive the selection processing of labeled training data by a care giver. The processing unit 110 determines labeled training data based on a user operation, and performs control of causing the display 473 to display the labeled training data.

Alternatively, the processing unit 110 may perform processing of automatically selecting labeled training data based on determination on similarity between the attributes of a care receiver whose bed position is to be adjusted and the attributes of a care receiver whose images are taken in labeled training data. The attributes mentioned here include information on the age, sex, height, weight, past medical history, medication history, and the like of the care receiver.

Alternatively, the bed position detection device 470 may perform processing of automatically selecting labeled training data based on processing of comparison between the attributes of a care receiver whose bed position is to be adjusted and additional information included in labeled training data. For example, it is assumed that a text indicating that "For a care receiver who has a tendency of XX, it is preferable to make adjustment such that the left shoulder may be YY" is included as additional information of labeled training data. In this case, if the care receiver whose bed position is to be adjusted corresponds to XX, the labeled training data is easily selected. For example, a care giver who adjusts a bed position may input information specifying a care receiver into the first terminal device 471, and the bed position detection device 470 may specify an attribute of the care receiver based on the information.

The bed position detection device 470 may output an image in which labeled training data subjected to the transmission processing is superimposed and displayed on a real-time taken image taken by the first terminal device 471, for example. At this time, additional information on the labeled training data may be displayed in a recognizable form. For example, in a case where the speech "Please tell a point" by a care giver using a microphone or the like of a headset has been detected, the bed position detection device 470 may output the text as voice from the headset via the server system 100, for example.

For example, the bed position detection device 470 may determine whether OK or NG based on the degree of similarity between an image taken during the position adjustment and labeled training data, and output a determination result. The determination result is displayed on the display 473 directly or via the server system 100. Moreover, the bed position detection device 470 may perform processing of displaying a specific point determined as NG. For example, the server system 100 or the bed position detection device 470 may perform processing of comparing an image taken by the first terminal device 471 with labeled training data, and weighted-displaying a location determined as having a large difference.

In this way, it is possible to present a bed position of a care receiver in comparison with an ideal bed position, and present information for implementing the ideal bed position.

Moreover, the bed position detection device 470 may be used for support of diaper changing. It has been found out that a skilled worker places great importance on the following points as tacit knowledge in diaper changing.
A. Whether a care receiver is in a lateral position.
B. Whether the position of a diaper is appropriate.
C. Whether a pad sticks out of a diaper.
D. Whether a diaper is mounted properly.

Therefore, the processing unit 210 of the bed position detection device 470 determines whether the abovementioned points A to D are satisfied, and transmits a determination result to the server system 100. The processing unit 210 herein corresponds to a processor of the second terminal device 472, for example. This enables a care giver to change a diaper properly irrespective of the degree of proficiency of the care giver.

For example, the second terminal device 472 performs skeleton tracking processing to each of images constituting a moving image of a care receiver taken using the camera, and outputs an image in which a skeleton tracking result is superimposed and displayed on the original image as a processing result. The processing result may be displayed on the display 473, for example. By doing so, a care giver can check the display 473 in a natural posture while changing a diaper of a care receiver.

Note that, in consideration of the case of changing a diaper in the nighttime, the second terminal device 472 may include a lighting unit. In addition, in consideration of a care receiver's privacy, a depth sensor or the like may be used instead of the camera. The depth sensor may be a sensor using the Time of Flight (ToF) method, may be a sensor using structured lighting, or may be a sensor using other methods.

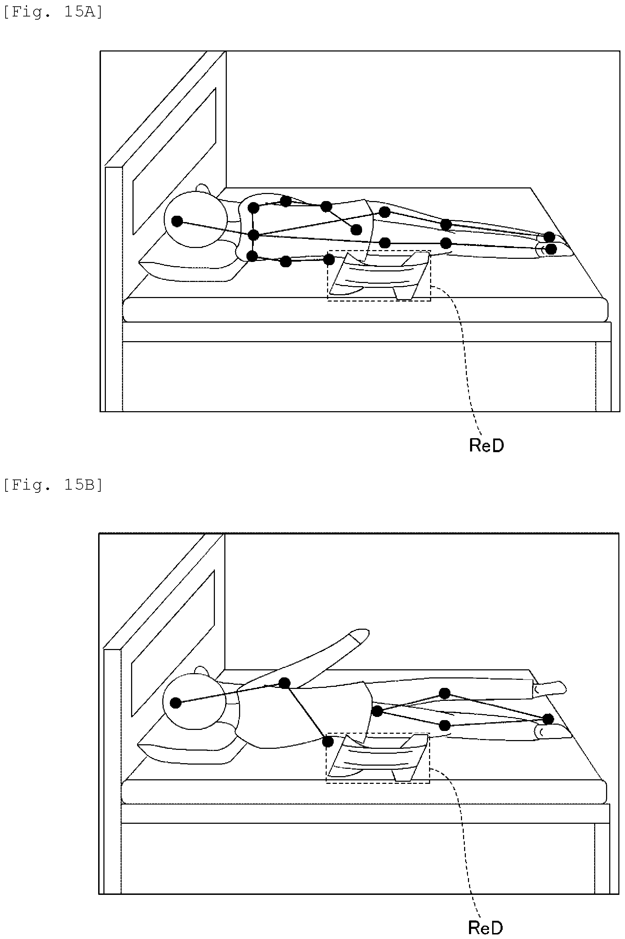

FIGs. 15A and 15B illustrate an example of images displayed on the display 473 in the case of changing a diaper. As described previously, each image includes a care receiver and the care receiver's skeleton tracking result.

In a state of FIG. 15A, a care receiver is laid stably in a lateral position, and the camera of the second terminal device 472 takes an image of the care receiver from his/her back straight. For example, in FIG. 15A, the difference between the front-rear direction of the body of the care receiver and the optical axis direction of the camera is small. As a result, as illustrated in FIG. 15A, many points are detected as points to be detected by the skeleton tracking.

On the other hand, in FIG. 15B, the posture is not stable as compared with that in FIG. 15A, and a care receiver is in a state of being likely to fall on his/her back. The camera of the second terminal device 472 is in a state of taking an image of the care receiver from obliquely behind, and therefore the number of points detected by the skeleton tracking decreases. For example, a point corresponding to the waist is hidden by a diaper and the like and not detected.

Accordingly, the second terminal device 472 may determine based on the skeleton tracking result whether the care receiver is in a lateral position as stated in A above. For example, the second terminal device 472 may determine that the care receiver is in a lateral position if a point corresponding to a specific portion such as the waist is detected by the skeleton tracking. However, a specific method for the lateral position determination is not limited to this, and whether a point other than the waist is detected, the relationship between multiple points, and the like may be used.

Moreover, the bed position detection device 470 performs the object tracking processing based on a moving image taken by the camera of the second terminal device 472, thereby continuously detecting a region of a diaper in the image. Since the object tracking is publicly known, its detailed description will be omitted. For example, in FIGs. 15A and 15B, a diaper region ReD is detected.

The bed position detection device 470 may determine whether the position of a diaper is appropriate as stated in B above based on the relationship between the skeleton tracking result and the diaper region ReD detected by the object tracking, for example. For example, while taking into consideration a position where a diaper is to be mounted, the bed position detection device 470 determines whether the waist position detected by the skeleton tracking and the diaper region ReD have a predetermined positional relationship. For example, the processing unit 210 may determine that the position of the diaper is appropriate if a straight line including two points corresponding to the pelvis passes through the diaper region ReD. Alternatively, machine learning may be performed in such a way that the skeleton tracking result from labeled training data of a skilled worker and the detection result of the diaper region ReD are extracted as feature data and the feature data is set as input data. The trained model is a model that outputs accuracy on whether the position of a diaper is appropriate upon receiving the skeleton tracking result and the detection result of the diaper region ReD, for example.

Moreover, the bed position detection device 470 may determine whether a pad sticks out of a diaper as stated in C above based on the length in the horizontal direction in the diaper region ReD. Since the pad is normally supposed to be fitted into the diaper, the length of the diaper region ReD in an image corresponds to the length of the diaper itself. Note that, the assumed size of the diaper region ReD can be presumed based on the type and size of the diaper, the optical characteristics of the camera of the second terminal device 472, and the like. On the other hand, when the pad sticks out, the length of the diaper region ReD in the image is longer by the amount that it sticks out. Accordingly, if the length of the diaper region ReD detected in the image is larger than the assumed length by a predetermined threshold or more, the bed position detection device 470 determines that the diaper sticks out of the pad and is thus inappropriate.

Meanwhile, the bed position detection device 470 may determine whether a diaper is mounted properly as stated in D above by detecting a tape for fixing the diaper in a state where the diaper is mounted. Normally, a member with a color different from the diaper main body is used for the tape. As an example, the diaper main body is white while the tape is blue. In addition, where and how the tape should be fixed in order to mount the diaper properly are known from the structure of the diaper. Accordingly, the processing unit 210 can detect a tape region in an image based on its color, and determine whether the diaper is mounted properly based on the relationship between the tape region and the diaper region ReD or based on the positional relationship between the tape region and the waist etc. detected by the skeleton tracking. Note that, in a case where multiple diapers of different manufacturers and types are used, the bed position detection device 470 may acquire information specifying a diaper and determine whether a diaper is mounted properly based on the type of the diaper etc. thus specified.

For example, the bed position detection device 470 determines OK or NG with respect to each of A to D above, and transmits a determination result to the server system 100. The server system 100 transmits the determination result to the display 473 and the like. Further, if determining that it is NG, the bed position detection device 470 may highlight a portion having a large difference from ground truth data.

With the above processes, it is possible to not only cause a care receiver to take a position that reduces a bedsore risk, but also use tacit knowledge in changing a diaper appropriately and cause a care giver to change a diaper properly. Note that, in work of the diaper changing, the body of the care receiver needs to be moved. For example, the care giver performs such work that the care receiver is caused to temporarily take a lateral position in order to easily dispose a diaper, and his/her legs are raised in order to cause the care receiver to put on the diaper. Accordingly, when the diaper changing has been completed, there is a possibility that the care receiver is not in a posture suitable to lie down on the bed. Therefore, the bed position detection device 470 may automatically execute processing for adjusting the abovementioned bed position, in a case where the completion of the diaper changing has been detected. For example, the first terminal device 471 starts taking an image of a care receiver, and the display 473 displays labeled training data subjected to the transmission processing by being superimposed on a real-time taken image taken by the first terminal device 471.

Moreover, in the foregoing, the example in which the first terminal device 471 or the second terminal device 472, such as a smartphone, is used for the bed position adjustment support or the diaper changing support has been described, but the specific device 200 is not limited thereto.

FIG. 14 is a diagram illustrating an example of an eyeglasses-type device 480, such as augmented reality (AR) glasses or mixed reality (MR) glasses, which is another example of the device 200 to be used for the bed position adjustment support or the diaper changing support. The eyeglasses-type device 480 has a camera that takes an image of a region corresponding to a user's field of view, for example. The eyeglasses-type device 480 has a lens portion a part or all of which serves as a display, and enables a user to visually check the situation of the surrounding by transmitting light from the surrounding through the display or by displaying an image corresponding to the user's field of view taken by the camera. Further, by using the display, the eyeglasses-type device 480 additionally displays some sort of information on the user's field of view.

Even in a case where the eyeglasses-type device 480 is used, an image in which a user on the bed 610 is taken can be similarly acquired. Therefore, the eyeglasses-type device 480 may perform processing of supporting the bed position adjustment by superimposing and displaying the taken image and the labeled training data as described above. Moreover, the eyeglasses-type device 480 may perform processing of supporting diaper changing by making a determination related to the diaper region ReD. Moreover, also in a case where the eyeglasses-type device 480 is used, the bed position adjustment may be similarly started in a case where the diaper changing has been completed. For example, in a case where the completion of the diaper adjustment has been detected, ground truth data related to the bed position adjustment may be transparently displayed on a display of the eyeglasses-type device 480.

Moreover, the eyeglasses-type device 480 may perform, based on an taken image obtained by taking a skin of a care receiver, processing of automatically detecting the presence or absence and a range of bedsore. In this way, it is possible to determine whether bedsore actually occur, and a state thereof if occurred. For example, a trained model is generated by machine learning based on training data in which an image obtained by taking an image of a care receiver is associated with ground truth data that specifies a bedsore region and is assigned by an expert such as a medical doctor. The eyeglasses-type device 480 makes a determination on bedsore by performing the processing based on the trained model.

Moreover, a mattress and a pillow that enable the pressure detection and the automatic body posture change are also known. The pressure detection is performed using a pressure sensor, similar to the bed side sensor 420 and the detection device 430. Moreover, the mattress and the pillow herein may prompt a care receiver to turn over by adjusting the height (thickness) for each portion using air and the like. Moreover, the device 200 that performs the pressure detection may be different from the device 200 that prompts the automatic body posture change. For example, in a case where the detection device 430 has determined that the same posture has continued based on the pressure value, the detection device 430 transmits information indicating the fact to the server system 100. The server system 100 may prompts a care receiver to change the body posture by controlling the mattress or the pillow based on the information. This can also reduce a bedsore risk of a care receiver who is bedridden. Moreover, the control method of the mattress and pillow is not limited to a general one. For example, ability information (ADL and the like) may be finely determined even in the same bedridden state, and a way to prompt the body posture change may be changed in accordance with the level of the ability information. For example, in a case of a care receiver whose ability is extremely lowered, the maintenance and the like of the posture are difficult, so that it can be considered that the posture is excessively changed when the body posture change is prompted. Therefore, a plurality of operation modes having the different frequencies of prompting the body posture change and the different amounts of air when the body posture change is prompted are prepared for the mattress and the pillow, and the operation mode may be controlled in accordance with the level difference of the ability information. Moreover, the mattress and the like may operate in a mode of performing improvement of sleep situation (for example, the suppression and the like of a snore by prompting a lateral position) while the ability is high, may operate in a mode of reducing a bedsore risk in a case where a care receiver becomes bedridden, and operation mode control in accordance with the ability information may be executed, including the states other than the bedridden state.

Moreover, in a case for a care receiver who is bedridden, tacit knowledge related to end-of-life care may be used. The device 200 in which an application corresponding to the tacit knowledge operates is, for example, a terminal device such as a smartphone. For example, the processing unit 210 of the device 200 acquires, as input data, five types of information including the amount or percentage of each type of food (e.g., may be for each of main and side dishes or may be for each of ingredients such as meat and fish) consumed at each meal, the amount of fluid intake, the timing when the meal is taken, information on diseases, and a weight (or BMI). A care giver may input the input data. Alternatively, by using an automatic recording device of the meal amount, part of the input data may be automatically acquired by the device 200.

Then, based on the input data, the processing unit 210 outputs output data indicating whether end-of-life care should be started after a predetermined period and whether it is the timing when the care contents should be changed after the end-of-life care is started. For example, machine learning may be performed based on training data in which ground truth data by a skilled worker is assigned to the input data. In this case, the processing unit 210 obtains output data by inputting the input data into a trained model. Besides, other machine learning methods such as SVM may be used, or methods other than machine learning may be used.

End-of-life care mentioned herein indicates assistance provided to a care receiver who is deemed to be highly likely to die in the near future. End-of-life care is different from normal assistance in that the emphasis is placed on alleviating physical and emotional pain, supporting a dignified life for a target care receiver, etc. In addition, since the state of a care receiver changes with time during end-of-life care, care assistance suitable for the target patient may change. In other words, by presenting the start timing of end-of-life care and the change timing of the care assistance contents during the end-of-life care, it is possible to provide appropriate care assistance to a care receiver to his/her last breath. For example, a skilled care giver has tacit knowledge of presuming the timing when end-of-life care is needed and the care contents from various perspectives such as the volume of meal, and other care givers can provide appropriate end-of-life care by digitizing such tacit knowledge.

In a case where the ability indicated by the ability information has decreased to a state that indicates the bedridden state, the processing unit 210 of the device 200 starts the determination based on the abovementioned input data, and outputs an analysis result screen indicating the determination result to the server system 100. Note that, the analysis result screen may be displayed on the display 240 of the device 200.

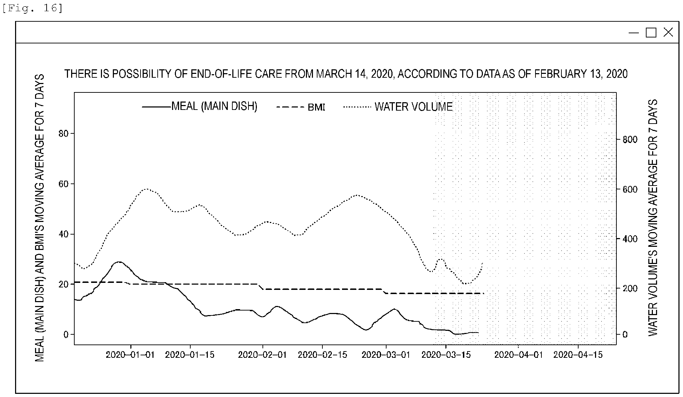

FIG. 16 is a diagram illustrating an example of an analysis result screen. The analysis result screen may include a time series change in feature data obtained based on the input data and the result of determination on whether end-of-life care should be started after a predetermined period. The feature data mentioned herein may be information, such as a moving average of the volume of meal, determined as important among the input information, or may be information obtained by calculation based on the five types of input information described above. For example, in the case of using the NN, the feature data may be an output from a given intermediate layer or output layer. For example, the input data includes the amount of main dish consumed, the amount of fluid, and a BMI actual measurement value acquired until February 13, 2020. The processing unit 210 may presume the transition of the amount of main dish consumed, the amount of fluid, and BMI since February 14, 2020 based on the trained model. The analysis screen may include a graph representing a time series change of the actual measurement value and the presumed value for each of the three items. Note that, FIG. 16 illustrates a graph indicating a 7 days moving average of each of these values. This enables a care giver to easily understand the transition of items important in end-of-life care. Note that, as described previously, the input data may include other items, and information to be displayed on the analysis result screen is not limited to that in the example of FIG. 16.

Meanwhile, a period in which end-of-life care may be carried out may be displayed on the analysis result screen. In the example of FIG. 16, a text "there is a possibility of end-of-life care from March 14, 2020" is displayed, and the corresponding period in the graph is displayed so as to be identifiable using a background color different from that of the other period. By doing so, the timing when and the period in which end-of-life care is presumed to be needed are specified clearly, so that information on end-of-life care can be presented to a user appropriately.

### <Motion of Device Related to Falling-Down Risk>

Moreover, as illustrated in FIG. 5, because the probability that a care giver who is bedridden performs a movement starting motion such as spontaneously standing-up is low, a low falling-down risk is assumed. Therefore, in a case for the care receiver who is bedridden, each of the devices 200 illustrated in FIGs. 8 and 9 may be set to the operation mode 0 that is an inactive state.

### <Operation of Device Related to Fall Risk>

Meanwhile, even a care receiver who is bedridden has a high fall risk because it can be considered that the care receiver uses the wheelchair 630 or the like. Therefore, even in a case for the care receiver who is bedridden, the processing related to a fall risk by each of the devices 200 illustrated in FIGs. 10 and 11 is preferably continued. At this time, the care receiver is difficult to spontaneously change the posture, so that a bedsore risk becomes high. Therefore, the seat surface sensor 440 illustrated in FIG. 10 may operate in the operation mode 3 in which in addition to the determination on forward displacement/lateral displacement and the fall determination, a level difference of the bedsore risk in the wheelchair 630 is determined. For example, the seat surface sensor 440 may determine that a bedsore risk is present, in a case where a state of a small change in the pressure value has continued for a predetermined time or longer. Similarly, the terminal device 450 illustrated in FIG. 11 may operate in an operation mode in which a position for suppressing bedsore is presented to a care giver.

### <Operation of Device Related to Aspiration Risk>

Moreover, even a care receiver who is bedridden continues to take a meal by mouth in some cases. Therefore, the swallowing and choking detection device 460 illustrated in FIG. 12 also operates in the case for a care receiver who is bedridden. Note that, the swallowing and choking detection device 460 may operate in the same mode in the case for a care receiver who is not bedridden but is difficult to take a meal, and in the case for a care receiver who is bedridden. Note that, the swallowing and choking detection device 460 may operate in the case for a care receiver who is bedridden in an mode in which an aspiration risk can be reduced more than in the case for a care receiver who is not bedridden but is difficult to take a meal. For example, the swallowing and choking detection device 460 may detect an aspiration risk easier by decreasing a threshold to be used for comparison with the swallowing time. Alternatively, the swallowing and choking detection device 460 may perform an operation of reducing an aspiration risk in a case for the care receiver who is bedridden by adding a determination related to an eating pattern. Alternatively, the swallowing and choking detection device 460 may perform an operation of reducing an aspiration risk in a case for the care receiver who is bedridden by adding, in addition to the processing such as the normal choking detection and the calculation of the swallowing time, a determination related to the presence or absence of dangerous choking. Moreover, the swallowing and choking detection device 460 may add processing of determining whether choking is frequently occurs, or may add processing of determining whether a care receiver is sleepy.

Moreover, as mentioned above in the abovementioned end-of-life care, in a case for the care receiver who is bedridden, automatic recording of the meal amount may be performed. For example the terminal device 462 of the swallowing and choking detection device 460 may take an image of a meal before and after the meal, and automatically record the meal amount based on a difference therebetween. In other words, the swallowing and choking detection device 460 may operate in an operation mode including the automatic recording of the meal amount in the case for a care receiver who is bedridden. Moreover, as the device 200 that automatically records the meal amount, a device different from the swallowing and choking detection device 460 may be used.

### <Update Ability Information>

Moreover, as illustrated at Step S205 in FIG. 7, the ability information acquisition unit 111 of the server system 100 may perform processing of updating ability information based on the sensing data. For example, the ability information acquisition unit 111 makes an evaluation related to bedsore based on the sensing data, and determines a change in an ability based on the evaluation result.

For example, the ability information acquisition unit 111 determines the degree of distribution of pressure values using the detection device 430 and the like, and determines that the ability is low because a posture change to suppress bedsore is not implemented in a case where a pressure is applied to a determined place for predetermined time or longer.

Moreover, the ability information acquisition unit 111 may determine the number of determined times as NG during the positioning adjustment and the number of times of an inappropriate position of a diaper based on the information from the bed position detection device 470, and obtain ability information based on these numbers of times. For example, a higher ability is determined as the number of determined times as NG is less or the number of times of an inappropriate position of a diaper is less, and a lower ability is determined as the number of times is more.

Moreover, the ability information acquisition unit 111 may update ability information based on the presence or absence of bedsore output from the eyeglasses-type device 480 such as MR glasses. For example, in a case where no bedsore is present, a higher ability is determined compared with a case where bedsore is present.

### 2.6 Summary

In the foregoing, the specific example and the motion example of the device 200 have been described using FIGs. 8 to 14 and other drawings. As mentioned above, each of the devices 200 switches the operation mode at least between the operation mode 0 indicating an inactive state and the operation mode 1 indicating an active state in accordance with the ability information. Moreover, as mentioned above, the operation mode indicating an active state is not limited to one, but a plurality of operation modes having different processing contents may be set.

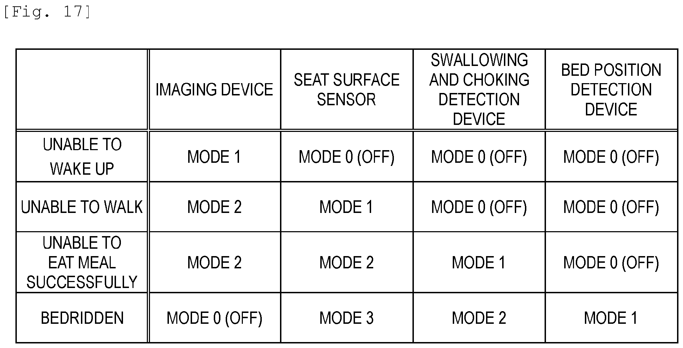

FIG. 17 illustrates an example of operation modes of the respective devices 200. As illustrated in FIG. 17, the imaging device 410 that detects a falling-down risk operates in the operation mode 1 for a care receiver who is unable to wake up, operates in the operation mode 2 for a care receiver who is unable to walk and for a care receiver who is unable to eat a meal successfully, and operates in the operation mode 0 indicating an inactive state for a care receiver who is bedridden.

For example, the operation mode 1 may be a mode in which a movement start is determined in a case where an edge sitting position has been detected, and the operation mode 2 may be a mode in which a movement start is determined in a case where awakening has been detected. The operation mode 2 in which a movement start is detected at an earlier stage than the operation mode 1 can further reduce a falling-down risk.

Moreover, the seat surface sensor 440 that detects a fall risk operates in the operation mode 0 indicating an inactive state for a care receiver who is unable to wake up, operates in the operation mode 1 for a care receiver who is unable to walk, operates in the operation mode 2 for a care receiver who is unable to eat a meal successfully, and operates in the operation mode 3 for a care receiver who is bedridden.

For example, the operation mode 1 may be a mode in which only the determination on forward displacement/lateral displacement is made, the operation mode 2 may be a mode in which the determination on a fall possibility is added, and the operation mode 3 may be a mode in which the bedsore determination in the wheelchair is added. In this example, objects to be determined are added with the decrease in the ability, so that it is possible to appropriately respond to an increase in risk with the ability change.

Moreover, the swallowing and choking detection device 460 that detects an aspiration risk operates in the operation mode 0 indicating an inactive state for a care receiver who is unable to wake up and for a care receiver who is unable to walk, operates in the operation mode 1 for a care receiver who is unable to eat a meal successfully, and operates in the operation mode 2 for a care receiver who is bedridden.

For example, the operation mode 1 may be a mode in which the determination based on the swallowing time is made, and the operation mode 2 may be a mode in which, in addition to the operation mode 1, an option such as the eating pattern is added. In this example, objects to be determined are added with the decrease in the ability, so that it is possible to appropriately respond to an increase in risk with the ability change.

Moreover, the bed position detection device 470 that detects a bedsore risk operates in the operation mode 0 indicating an inactive state for a care receiver who is unable to wake up, a care receiver who is unable to walk, and a care receiver who is unable to eat a meal successfully, and operates in the operation mode 1 for a care receiver who is bedridden.

As is in the foregoing, with the method in the embodiment, after considering the relationship between the ability and various kinds of risks, it is possible to appropriately determine the presence or absence of a risk in a highly necessary scene, and perform processing for reducing the risk. Note that, FIG. 17 illustrates one example of relationship among the devices 200, ability information, and the operation modes, and the method in the embodiment is not limited thereto.

In a case where an operation mode of the device 200 is set based on the ability information, as an ability of the care receiver is decreased more, it is assumed that the function of the device 200 is added more in order to compensate the decrease. Further, a first device in the devices 200 may be set to, within a range in which an ability value indicated by the ability information is equal to or more than a predetermined threshold, an operation mode in which a function to be used is increased with a decrease in the ability value, and set to, within a range in which the ability value is less than the predetermined threshold, an operation mode in which the number of functions to be used is less than that within the range equal to or more than the predetermined threshold. In other words, as for some decrease in the ability, a part of the devices in the embodiment operates in a direction so as to increase the function for compensating the decrease, but may shift to a direction so as to decrease the function in a case where a decrease in the ability beyond a given level is seen. In this way, it is possible to reduce a processing load by appropriately considering the necessity or unnecessity of each tacit knowledge in accordance with the ability, and setting an application corresponding to the tacit knowledge with low necessity to be inactive.

The first device herein may be the device 200 that is used for the determination on a falling-down risk in the movement start by a care receiver. For example, the first device is at least one of the imaging device 410 illustrated in FIG. 8, the bed side sensor 420 illustrated in FIG. 9, and the detection device 430 illustrated in FIG. 9. In the example of FIG. 17, within the ranges of "UNABLE TO WAKE UP", "UNABLE TO WALK", and "UNABLE TO EAT MEAL SUCCESSFULLY", these operation modes of the device 200 change in a direction so as to add a processing content, whereas in "BEDRIDDEN" that is the lower ability, the operation mode corresponding to the inactive state is set. This can prevent the operation by the device in which the necessity has decreased from continuing. Note that, the detection device 430 may be used for an end-of-life care determination in "BEDRIDDEN", and all the devices 200 to be used for the determination on a falling-down risk in the movement start do not need to decrease the function in a state at a low ability value.

### 3. Device Control Based on Scene Information And Device Type Information

Moreover, in the method in the embodiment, information other than ability information may be used for setting an operation mode of the device 200. Hereinafter, scene information and device type information will be described.

### 3.1 Overview

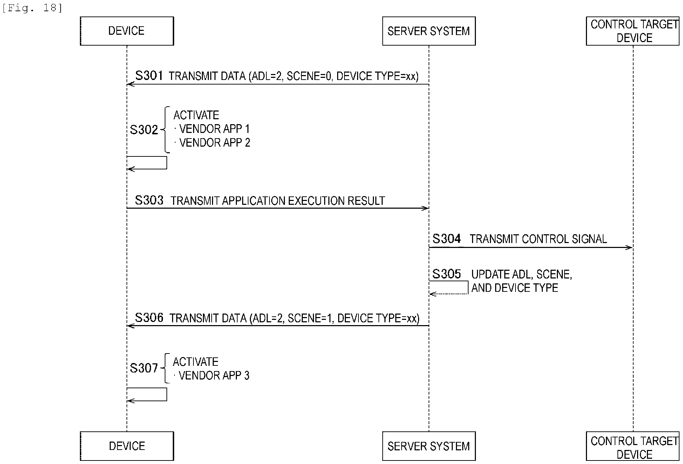

FIG. 18 is a sequence diagram illustrating operations of the server system 100 and the device 200, and is a diagram illustrating an example in which the operation mode of the device 200 is changed based on ability information on a care receiver, scene information, and device type information.

Firstly, at Step S301, the server system 100 performs processing of transmitting data including ability information, scene information, and device type information to the device 200. Note that, either one of scene information and device type information may be omitted. FIG. 18 illustrates the example in which data including an indicator value of ADL being 2, a scene ID for specifying a scene being 0, and a device type ID for specifying a device type being xx is transmitted.

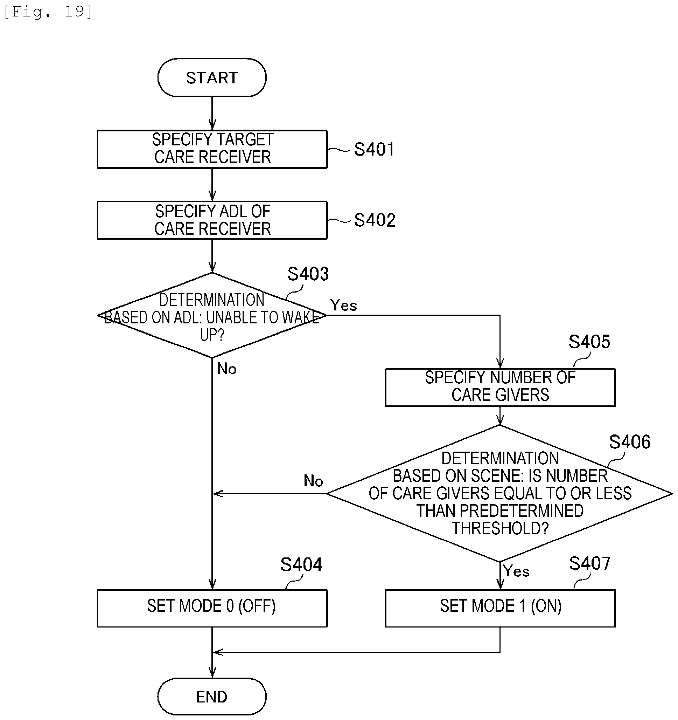

At Step S302, the device 200 controls an active/inactive state of the installed vendor app based on the acquired ability information, scene information, and device type information. For example, the storing unit 220 of the device 200 may store table data in which the indicator value of ADL, the scene ID, and the device type ID, are associated with the active/inactive state of each application. The processing unit 210 of the device 200 extracts a record coincident with the received data in the table data, thereby determining an active/inactive state of each application. Moreover, as is described later using FIGs. 19, 20, and 22, each of the devices 200 may store an algorithm that determines an operation mode based on the ability information, the scene information, and the device type information. FIG. 18 illustrates the example of setting an operation mode in which in the vendor app 1 to the vendor app 3, the vendor apps 1 and 2 become active, and the vendor app 3 becomes inactive.

After the processing at Step S302, the device 200 executes processing in accordance with the vendor app 1, and processing in accordance with the vendor app 2. At Step S303, the device 200 transmits a processing result to the server system 100. The processing result herein corresponds to a result of determination executed using the tacit knowledge of the skilled worker. Moreover, the processing result herein may include a log and the like of sensing data detected by the device 200.

At Step S304, the server system 100 executes control based on the processing result received from the device 200. For example, the processing unit 110 may perform processing of specifying a control target device, and transmitting a control signal for operating the control target device.

Moreover, at Step S305, the server system 100 executes processing of updating ability information on a care receiver, scene information, and device type information. The update processing of ability information is as mentioned above. Moreover, the scene information acquisition unit 112 obtains scene information based on at least one of information related to the care receiver such as the log of the sensing data and the attribute acquired from the device 200, and information related to a care giver such as a schedule. Moreover, the device type information acquisition unit 113 acquires information indicating the type of another device 200 that is used with the target device 200, as device type information. An example of the specific processing will be described later.

At Step S306, the server system 100 performs processing of transmitting data including the updated ability information, scene information, and device type information to the device 200. FIG. 18 illustrates the example in which the scene ID is changed from 0 to 1.

At Step S307, the device 200 controls an active/inactive state of the installed vendor app based on the acquired data. For example, as mentioned above, the device 200 determines an active/inactive state of each vendor app based on table data. In the example of FIG. 18, the vendor apps 1 and 2 are maintained in the active state, and the vendor app 3 is changed from the inactive state to the active state. Accordingly, at and after Step S307, the device 200 is shifted in a state of operating in an operation mode in which all the vendor app 1 to the vendor app 3 become active. Operations at and after Step S307 are similar to those at Steps S303-S306, for example.

Hereinafter, a setting example of an operation mode based on the scene information, and a setting example of an operation mode using the device type information will be respectively described.

### 3.2 Specific Example of Processing Based on Scene Information

The server system 100 may obtain scene information specifying a scene of care assistance for a care receiver, and transmit the obtained scene information to the device. The device 200 determines in which operation mode among a plurality of operation modes the device 200 operates based on the ability information and the scene information. For example, the storing unit 220 of the device 200 stores information in which the ability information and the scene information are associated with an operation mode. The processing unit 210 obtains an operation mode based on the information, and the ability information and the scene information acquired from the server system 100. The information in which the ability information and the scene information are associated with the operation mode may be, for example, table data in which a value of the ability information, a value of the scene information, and an active/inactive state of each application are associated with one another. Alternatively, information in which the ability information and the scene information are associated with the operation mode may be an algorithm that obtains an active/inactive state of each application based on the ability information and the scene information. In this way, it is possible to cause the appropriate device 200 to operate in accordance with the care assistance scene, in other words, to cause care assistance using the appropriate tacit knowledge to be executed.

For example, the scene information acquisition unit 112 may obtain information related to a care giver who executes care assistance for the care receiver, as scene information. Specifically, scene information may be information related to the number of care givers who can execute care assistance for the care receiver, or may be information related to service years, the degree of proficiency, holding qualification, and the like. Hereinafter, an example in which the number of care givers is used will be described. In this case, the device 200 determines an operation mode in accordance with the number of care givers.

The device 200 herein may be, for example, the imaging device 410 mentioned above using FIG. 8. For example, in a case where care givers of equal to or more than the fixed number are present in a living room or the like where care receivers perform activities together, the care givers can help to each other, so that the care giver can visually determine a movement start of the care receiver, and appropriately perform intervention in a case where the care giver has determined that a falling-down risk is present. The intervention herein indicates supporting a movement start, for example, the care giver moves closer to a care receiver who is going to start movement and support his/her body if necessary. In this case, the necessity of the imaging device 410 becoming active is relatively low.

On the other hand, in a case where the number of care givers who are positioned in the target space is small, one care giver should execute many tasks, so that observing the movement by the care receiver in detail is difficult, and the intervention at the appropriate timing may be difficult. In this case, the necessity of the imaging device 410 becoming active is relatively high.

In consideration of the above, by determining an operation mode of the imaging device 410 using the scene information in addition to the ability information, it is possible to cause the imaging device 410 to appropriately operate.

FIG. 19 is a flowchart illustrating determination processing of an operation mode in the imaging device 410. Firstly, at Step S401, the processing unit 210 of the imaging device 410 performs processing of specifying a care receiver serving as a target. For example, the processing unit 210 performs face recognition processing based on a taken image, thereby specifying a care receiver.

At Step S402, the processing unit 210 acquires ability information on the care receiver. For example, the processing unit 210 receives data illustrated at Steps S301 and S306 in FIG. 18 from the server system 100, thereby specifying ability information.

At Step S403, the processing unit 210 determines whether the ability of the care receiver is lowered to a state where he/she is unable to wake up based on the ability information. If the ability is not lowered (Step S403: No), a falling-down risk is low, so that at Step S404, the processing unit 210 sets an operation mode of the imaging device 410 to the mode 0 corresponding to the inactive state. Note that, the ability information in the embodiment is not limited to an indicator value of ADL, but may be more detailed information, for example, information indicating the abovementioned way of standing-up, a sitting posture holding ability, a swallowing ability, a walking ability, and the like. In addition, it is possible to use various kinds of sensing data when a sitting posture holding ability and the like are obtained, and various kinds of modifications are executable for an algorithm when a sitting posture holding ability and the like are obtained from the sensing data. In the determination on whether the ability of the care receiver is lowered to a state where he/she is unable to wake up illustrated at Step S403, as mentioned above, processing that uses detailed ability information having a data amount more than the indicator value of ADL may be executed. In this case, a processing load at Step S403 becomes large, so that the processing may be executed in the server system 100.

If the ability of the care receiver is lowered to a state where he/she is unable to wake up (Step S403: Yes), the processing unit 210 specifies the number of care givers based on the scene information at Step S405. For example, the scene information acquisition unit 112 of the server system 100 may determine the number of care givers based on the taken image, or may determine the number of care givers based on a care assistance schedule (for example, a work schedule) in the care facility or the like. Alternatively, a radio frequency identifier (RFID) tag or the like is mounted to a care giver, and a reading apparatus is provided in an entrance or the like in a target space, so that information indicating the number of care givers in the target space may be obtained. The processing unit 210 of the device 200 acquires the information from the server system 100, thereby executing the processing illustrated at Step S405.

At Step S406, the processing unit 210 makes a determination based on scene information. Specifically, the processing unit 210 determines whether the number of care givers is equal to or less than a predetermined threshold. If the number of persons is more than the predetermined threshold (Step S406: No), persons who can respond to a falling-down risk are secured, so that the processing is shifted to Step S404, and the processing unit 210 sets the operation mode of the imaging device 410 to the mode 0 corresponding to the inactive state.

If the number of persons is equal to or less than the predetermined threshold (Step S406: Yes), the care givers are insufficient and support by the device 200 becomes important for suppressing a falling-down risk. Therefore, at Step S407, the processing unit 210 sets an operation mode of the imaging device 410 to the mode 1 corresponding to the active state. Note that, although a form in which the processing unit 210 performs Steps S405 and S406 has been described in FIG. 19, the embodiment is not limited thereto, and for example, the server system 100 may conduct Steps S405 and S406, and the processing unit 210 may receive only a result thereof and determine whether the processing is transitioned to Step S404 or transitioned to Step S407.

Moreover, the scene information is not limited to information related to a care giver. For example, scene information acquisition unit 112 of the server system 100 may obtain information related to a care receiver as scene information. Specifically, scene information may be attribute information indicating an attribute of a care receiver. Note that, the attribute information herein includes an age, a body height, a body weight, a gender, a past medical history, a medication history, and the like of the care receiver. For example, the scene information acquisition unit 112 obtains information indicating whether a care receiver is dementia as scene information.

In order to reduce a falling-down risk at the start of movement, it is important to temporarily stop the motion by calling performed by a care giver, but a dementia patient do not react to the calling by the care giver in some cases. However, there are findings that even a dementia patient remembers voices of persons having a close relation such as a family in many cases, and the probability that the dementia patient reacts to the calling by the family is high.

Therefore, the imaging device 410 may have an operation mode in which voice data with a voice of the family recorded or moving image data with caution given by the family is stored, and the voice data or moving image data is output using a speaker, which is not illustrated. For example, the processing unit 210 of the imaging device 410 determines an active/inactive state based on ability information, and scene information, which is the number of care givers, as illustrated in FIG. 19. Moreover, in a case where the imaging device 410 is set to be active, the processing unit 210 determines whether the care receiver specified at Step S401 is a dementia patient. The imaging device 410 operates in an operation mode in which voice data or the like of the family is output if the care receiver is a dementia patient, and operates in an operation mode in which the voice data or the like is not output if the care receiver is not a dementia patient. In this way, it is possible to set an operation mode in accordance with the attribute of the care receiver. Note that, a plurality of pieces of voice data of the family and the like may be prepared for one care receiver. For example, if calling by the family and the like is only one type, the care receiver remembers the calling and may react dully. Therefore, the imaging device 410 may perform processing of outputting one selected on a random basis among the plurality of pieces of voice data stored in association with the target care receiver. In this way, variations of calling by the family and the like can be increased, so that it is possible to effectively stop the movement start by the care receiver.

Moreover, the scene information in the embodiment may be information indicating a type of care assistance such as meal care assistance, excretion care assistance, and movement and transfer care assistance. For example, the scene information acquisition unit 112 may acquire scene information indicating a type of care assistance based on a user input by a care giver. Moreover, the scene information acquisition unit 112 may obtain a type of care assistance being executed based on a relationship between a care assistance schedule in a care facility or the like and the current time. Alternatively, the scene information acquisition unit 112 may obtain a type of care assistance by estimating a position of a care receiver in the care facility or the like. For example, the scene information acquisition unit 112 determines that meal care assistance is provided if a care receiver is in a dining room, and excretion care assistance or care assistance for falling-down prevention is performed if the care receiver is in a toilet. The position determination may be performed using human-presence sensors and the like disposed in various places of the facility. Alternatively, access points (APs) are disposed in various places of the facility, and the position determination may be performed in accordance with to which AP a station apparatus (STA) that is carried by a care giver is connected.

For example, the seat surface sensor 440 illustrated in FIG. 10 can execute the forward displacement/lateral displacement determination and the determination on a fall possibility. However, in a case where a care receiver sits on the wheelchair 630 and eats a meal, as illustrated in FIG. 12, a table or the like on which dishes are arranged is disposed in front to the care receiver, and the table serves as a support, so that the possibility that a fall occurs is low. Therefore, the seat surface sensor 440 can omit the processing with low necessity by setting the determination on a fall possibility during meal to be inactive.

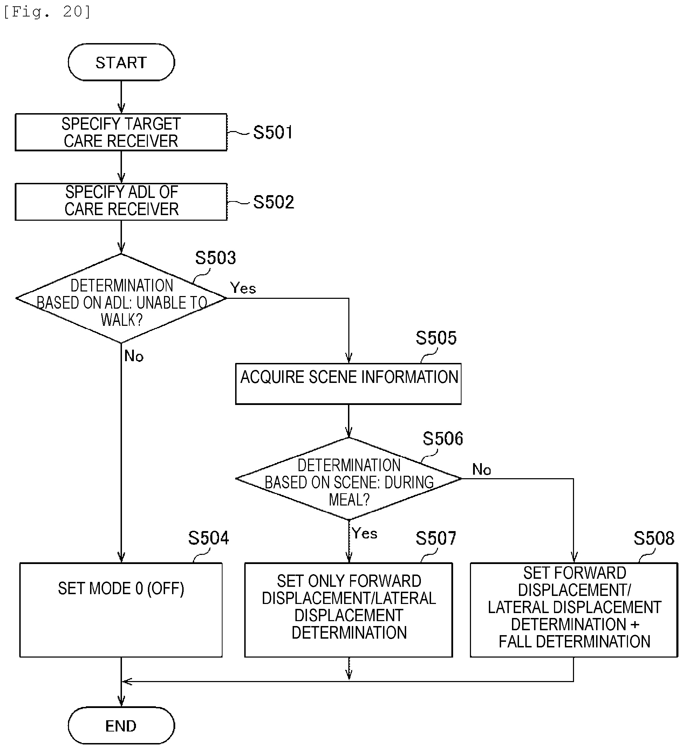

FIG. 20 is a flowchart illustrating determination processing of an operation mode in the seat surface sensor 440. Firstly, at Step S501, the processing unit 210 of the seat surface sensor 440 performs processing of specifying a care receiver serving as a target. For example, the processing unit 210 may specify a care receiver based on a care assistance schedule and the like, or may specify a care receiver based on a user input.

At Step S502, the processing unit 210 acquires ability information on a care receiver. For example, the processing unit 210 receives data illustrated at Steps S301 and S306 in FIG. 18 from the server system 100, thereby specifying ability information.

At Step S503, the processing unit 210 determines whether the ability of the care receiver is lowered to a state where he/she is unable to walk based on the ability information. If the ability is not lowered (Step S503: No), the necessity of the determination using the seat surface sensor 440 is low, so that at Step S504, the processing unit 210 sets an operation mode of the seat surface sensor 440 to the mode 0 corresponding to the inactive state. Moreover, similar to the example mentioned above at Step S403, processing in which more detailed information on ability information is considered may be executed at Step S503. Therefore, the processing at Step S503 may be executed in the server system 100.

If the ability of the care receiver is lowered to a state where he/she is unable to walk (Step S503: Yes), the processing unit 210 acquires information indicating a type of care assistance as scene information at Step S505. As mentioned above, the processing at Step S505 may be performed based on the user input, or may be performed based on a some sort of the sensing data.

At Step S506, the processing unit 210 makes a determination based on scene information. Specifically, the processing unit 210 determines whether the care assistance type is meal care assistance . If the care assistance type is meal care assistance (Step S506: Yes), the forward displacement/lateral displacement determination is useful but the determination on a fall possibility has low necessity. Therefore, at Step S507, the processing unit 210 sets an operation mode of the seat surface sensor 440 to a mode in which the forward displacement/lateral displacement determination is performed, and the determination on a fall possibility is not performed.

If the care assistance type is not meal care assistance (Step S506: No), a table is not always present in front of a care receiver, and a fall risk from the wheelchair 630 is high. Therefore, at Step S508, the processing unit 210 sets an operation mode of the seat surface sensor 440 to a mode in which both of the forward displacement/lateral displacement determination and the determination on a fall possibility are executed. Therefore, at Step S508, the processing unit 210 sets an operation mode of the seat surface sensor 440 to a mode in which the forward displacement/lateral displacement determination is not executed, and the determination on a fall possibility is executed.

Note that, in the foregoing, as for the imaging device 410 and the seat surface sensor 440, the example in which processing based on scene information is performed has been described. Further, it is needless to say that an operation mode of another device 200 may be determined based on scene information.

3.3 Specific Example of Processing Based on Device Type Information

Moreover, the server system 100 (the device type information acquisition unit 113) may obtain device type information specifying a type of a combined device to be used for care assistance of the same care receiver as in the device 200. The server system 100 transmits device type information to the device 200. The device 200 determines in which operation mode among a plurality of operation modes the device 200 operates based on ability information and device type information. For example, the storing unit 220 of the device 200 stores information in which ability information and device type information are associated with an operation mode. The processing unit 210 obtains an operation mode based on the information, and ability information and device type information acquired from the server system 100. The information in which ability information and device type information are associated with an operation mode may be, for example, table data in which a value of ability information, a value of device type information, and an active/inactive state of each application are associated with one another. Alternatively, the information in which ability information and device type information are associated with an operation mode may be an algorithm that obtains an active/inactive state of each application based on the ability information and the device type information.

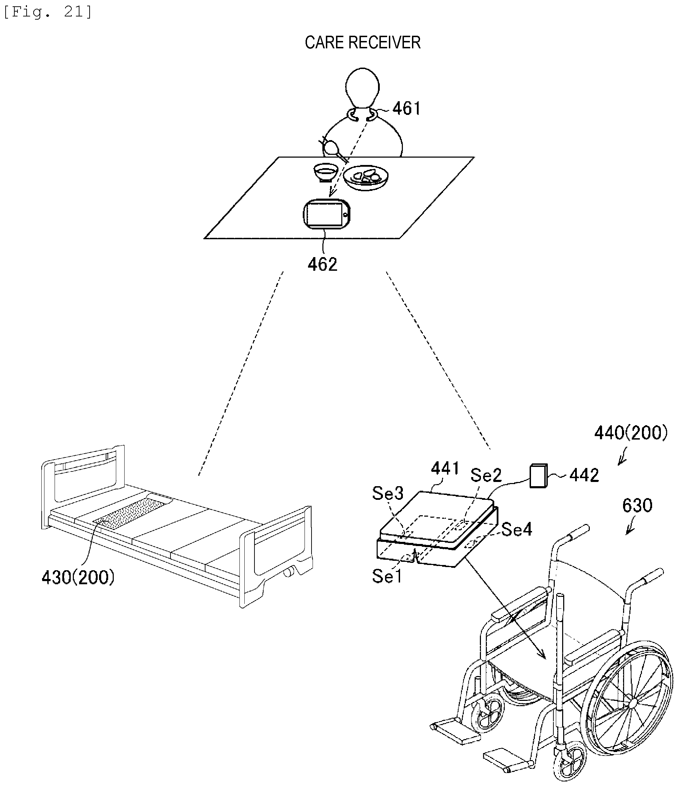

FIG. 21 is a diagram illustrating an example of the swallowing and choking detection device 460 mentioned above using FIG. 12 and a combined device. The combined device herein may be specifically the device 200 according to the embodiment. The swallowing and choking detection device 460 is used during meal, meanwhile, having the meal may be performed using the wheelchair 630 or may be performed using the bed 610.

For example, the combined device of the swallowing and choking detection device 460 may be the seat surface sensor 440 illustrated in FIG. 10. The device type information acquisition unit 113 may determine that the swallowing and choking detection device 460 and the seat surface sensor 440 are used in combination, in a case where the swallowing and choking detection device 460 is active, and a detection result from the seat surface sensor 440 or a log of the sensing data has been acquired. Alternatively, the device type information acquisition unit 113 may determine that the swallowing and choking detection device 460 and the seat surface sensor 440 are used in combination, in a case where it has been determined that a care receiver is seated on the wheelchair 630 based on the detection result from the seat surface sensor 440 or the log of the sensing data. In this case, it can be considered that the care receiver eats a meal using the wheelchair 630.

Similarly, the combined device of the swallowing and choking detection device 460 may be the detection device 430 illustrated in FIG. 9. The device type information acquisition unit 113 may determine that the swallowing and choking detection device 460 and the detection device 430 are used in combination, in a case where the swallowing and choking detection device 460 is active, and a detection result from the detection device 430 or a log of the sensing data has been acquired. Alternatively, the device type information acquisition unit 113 may determine that the swallowing and choking detection device 460 and the detection device 430 are used in combination, in a case where it has been determined that a care receiver is in a bed presence state based on the detection result from the detection device 430 or the log of the sensing data. In this case, it can be considered that the care receiver eats a meal using the bed 610.

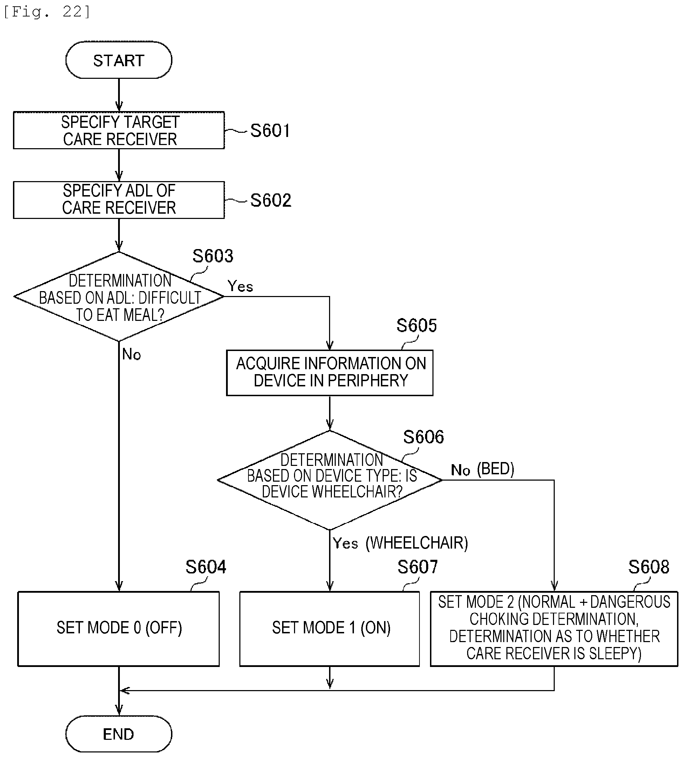

FIG. 22 is a flowchart illustrating determination processing of an operation mode in the swallowing and choking detection device 460. Firstly, at Step S601, the processing unit 210 of the swallowing and choking detection device 460 performs processing of specifying a care receiver serving as a target. For example, the processing unit 210 may specify a care receiver based on face recognition processing with respect to a taken image taken by the terminal device 462.

At Step S602, the swallowing and choking detection device 460 acquires ability information on the care receiver. For example, the processing unit 210 receives data illustrated at Steps S301 and S306 in FIG. 18 from the server system 100, thereby specifying ability information.

At Step S603, the swallowing and choking detection device 460 determines whether the ability of the care receiver is lowered to a state where he/she is unable to eat a meal successfully based on the ability information. If the ability is not lowered (Step S603: No), the necessity of the determination using the swallowing and choking detection device 460 is low, so that at Step S604, the processing unit 210 sets an operation mode of the swallowing and choking detection device 460 to the mode 0 corresponding to the inactive state.

If the ability of the care receiver is lowered to a state where he/she is unable to eat a meal successfully (Yes at Step S603), the swallowing and choking detection device 460 acquires information specifying a combined device from the device type information acquisition unit 113 at Step S605. Note that, as is understood from the abovementioned example, it only needs to allow the determination on whether the meal in the bed 610 or the meal in the wheelchair 630, and the type of the combined device is thus important, while the necessity of specifying a vendor or a model is low. Therefore, at Step S605, the swallowing and choking detection device 460 acquires a device type ID of the combined device.

At Step S606, the swallowing and choking detection device 460 makes a determination based on device type information. Specifically, the swallowing and choking detection device 460 determines whether having the meal is performed using the wheelchair 630. Specifically, as mentioned above, the swallowing and choking detection device 460 may determine whether the combined device indicated by the device type information is the seat surface sensor 440 or the detection device 430. If having the meal is performed using the wheelchair 630 (Step S606: Yes), the movement to the dining room or the like is possible, and a state of the care receiver is relatively good. Therefore, at Step S607, the processing unit 210 sets an operation mode of the swallowing and choking detection device 460 to a mode in which a normal determination using the swallowing time and the like is made.

On the other hand, if having the meal is performed using the bed 610 (Step S606: No), it is estimated that the care receiver does not move easily, or is in a state in which eating a meal is difficult without a device capable of flexibly setting a back angle, such as the bed 610. Therefore, at Step S608, the processing unit 210 sets an operation mode of the swallowing and choking detection device 460 to a mode in which further reduction in an aspiration risk is possible. For example, the swallowing and choking detection device 460 not only simply detects the presence or absence of choking but may perform processing of determining a content of the choking. For example, the swallowing and choking detection device 460 may determine whether the choking is dangerous choking easily leading to aspiration. Moreover, the swallowing and choking detection device 460 may determine whether a care receiver is sleepy. The determination as to whether the care receiver is sleepy may be made based on opening and closing of his/her eyes, the frequency of movement of his/her mouth or hand, and the like, based on the taken image taken by the terminal device 462, for example. Moreover, the determination as to whether the care receiver is sleepy may be made using the detection device 430.

Note that, in the foregoing, the two types of combined devices of the wheelchair 630 and the bed 610 have been exemplified, but the embodiment is not limited thereto. For example, as the wheelchair 630, a normal wheelchair and a reclining wheelchair may be used. In this case, the flexibility of the back angle adjustment becomes higher in the order from the normal wheelchair, the reclining wheelchair, toward the bed 610. Therefore, the operation mode of the swallowing and choking detection device 460 may be controlled such that more careful care may be executed in the order from the normal wheelchair, the reclining wheelchair, toward the bed 610. For example, in the swallowing and choking detection device 460, the number of applications to be set active may be increased in the abovementioned order.

Moreover, in the foregoing, the example in which the swallowing and choking detection device 460 is the device 200 serving as a setting target of the operation mode, and the seat surface sensor 440 or the detection device 430 is the combined device has been described. However, the seat surface sensor 440 or the detection device 430 may be the device 200 serving as a setting target of the operation mode, and the swallowing and choking detection device 460 may be the combined device. In other words, in the method in the embodiment, in a case where a plurality of devices 200 are used in combination, an operation mode may be changed by mutual collaboration of the plurality of devices 200. For example, by using even either one of the seat surface sensor 440 illustrated in FIG. 10 and the terminal device 450 illustrated in FIG. 11 that is used for the adjustment of a wheelchair position, it is useful to reduce a fall risk. Further, both of the seat surface sensor 440 and the terminal device 450 may be used in combination. Moreover, in this case, in conjunction with the control of one of the devices 200, the other device 200 may be controlled.

For example, it has been determined that a care receiver has been shifted to a state of being unable to walk based on the ability information, the seat surface sensor 440 may firstly be shifted to an operation mode corresponding to the active state, and the terminal device 450 may maintain an operation mode corresponding to the inactive state. In this way, the front displacement/lateral displacement is firstly determined, and a sitting posture holding ability is obtained using log data.

Further, if it has been determined that the sitting posture holding ability has decreased to equal to or less than the predetermined level, the terminal device 450 is shifted to the operation mode corresponding to the active state. Accordingly, the device type information acquisition unit 113 transmits data indicating that the terminal device 450 is a combined device, to the seat surface sensor 440. Moreover, in a case where the terminal device 450 is used in combination, the seat surface sensor 440 may be set to an operation mode different from a case where terminal device 450 is not used in combination. For example, the processing unit 210 of the seat surface sensor 440 may change a threshold in the determination. Alternatively, the seat surface sensor 440 may be shifted from an operation mode in which a reference (initial value) in the front displacement/lateral displacement determination and the like is internally determined to an operation mode in which the reference is determined based on the timing when the position adjustment using the terminal device 450 has been performed, and the like. Moreover, in the other cases, similarly, based on information on either one of the seat surface sensor 440 and the terminal device 450, the control of the other may be changed.

Alternatively, the operation mode of the seat surface sensor 440 may be controlled based on the information acquired in the terminal device 450. For example, a point to which a care giver needs to pay attention when adjusting a posture of a care receiver can be input into the terminal device 450. For example, if points of paying attention to a position of the right shoulder, putting a cushion under the right arm, and the like are input, the terminal device 450 can estimate an attribute of the care receiver based on the points. In the abovementioned example, an attribute that the care receiver has a suspicion of a right shoulder paralysis is obtained. The seat surface sensor 440 may determine an operation mode based on the information. For example, the seat surface sensor 440 may store, as an application that makes a forward displacement/lateral displacement determination, a plurality of different applications in accordance with the presence or absence of the paralysis and a site where the paralysis occurs. Moreover, based on the attribute acquired from the terminal device 450, the seat surface sensor 440 controls an active/inactive state of each application. In the abovementioned example, the seat surface sensor 440 sets an application that makes a forward displacement/lateral displacement determination suitable for a care receiver having the right shoulder paralysis to be active, and sets an application that makes another forward displacement/lateral displacement determination to be inactive. In this way, in the seat surface sensor 440 capable of using a plurality pieces of tacit knowledge for the forward displacement/lateral displacement determination, it is possible to switch the tacit knowledge in accordance with the attribute of the care receiver.

Moreover, the server system 100 in the embodiment may acquire mode information specifying an operation mode of a combined device, and may transmit the device type information and the mode information to the device 200. The device 200 determines in which operation mode among a plurality of operation modes the device 200 operates based on the ability information, the device type information, and the mode information.

For example, in a case where the seat surface sensor 440 has been shifted to an operation mode in which in addition to the forward displacement/lateral displacement determination, a determination on a fall possibility is made, the terminal device 450 may be shifted to an operation mode in which in addition to the normal processing of determining the suitability of a wheelchair position, additional processing of recommending a cushion and the like is executed. Alternatively, in a case where the operation mode has changed in the seat surface sensor 440, processing of switching labeled training data for determining ground truth may be executed in the terminal device 450. In addition, various kinds of modifications are executable for a cooperation method of a specific operation mode.

In this way, the operation mode is not limited to the simple active/inactive state, but it is possible to operate a plurality of devices 200 together using a more detailed operation mode. In the abovementioned example, the device 200 related to the detection of a wheelchair position and the device 200 related to the adjustment support of a wheelchair position are appropriately cooperated to each other, so that it is possible to further reduce a fall risk and the like of the care receiver. Note that, the cooperation between the seat surface sensor 440 and the terminal device 450 has been described herein, but another device 200 is not prevented from operating in cooperation.

Moreover, in the foregoing, the combination of ability information and scene information, and the combination of ability information and device type information have been described, but three pieces of information, which are ability information, scene information, and device type information, may be combined.

Moreover, in the method in the embodiment, the type of care assistance may be associated with the type of the device 200. For example, the swallowing and choking detection device 460 illustrated in FIG. 12 is the device 200 specific to meal care assistance, so that in a case where the swallowing and choking detection device 460 is operating, the probability that meal care assistance to a care receiver is performed is high. In this manner, among the devices 200, device type information on some devices can be associated with the type of care assistance. For example, the swallowing and choking detection device 460, if in an active state, communicates with the server system 100 via the gateway 300, so that the scene information acquisition unit 112 can determine an operation state of the swallowing and choking detection device 460 based on the presence or absence of the communication with the swallowing and choking detection device 460.

For example, the scene information acquisition unit 112 of the server system 100 may obtain scene information indicating a type of care assistance based on device type information acquired by the device type information acquisition unit 113, and transmit the scene information to the device 200. For example, the scene information acquisition unit 112 determines that the care receiver is during meal if the swallowing and choking detection device 460 is active, and determines that the care receiver is not during meal if the swallowing and choking detection device 460 is inactive. Alternatively, the server system 100 may transmit device type information acquired by the device type information acquisition unit 113 to the device 200, and perform processing of determining an operation mode in accordance with a care assistance type associated with the device 200 in the device 200. For example, in a case where the swallowing and choking detection device 460 is a combined device, the device 200 may perform processing of selecting an operation mode suitable for meal care assistance. As is in the foregoing, the operation mode setting in accordance with the type of care assistance may be executed as processing related to scene information, may be executed as processing related to device type information, and various kinds of modifications are executable for the specific embodiment.

### 4. Another Example of Device

Moreover, the device 200 in the embodiment is not limited to that mentioned above. For example, the device 200 in the embodiment may be an MCI determination device that makes a determination of mild cognitive impairment (MCI). For example, the MCI determination device performs processing of asking a question to a care receiver using a voice or an image, and receiving a response with respect to the question. The question herein may be a question in which mini-mental state examination (MMSE) and the like are used, or may be a question in which another method is used. The MCI determination device makes an MCI determination based on a response by a care receiver. Moreover, the MCI determination device may make an MCI determination based on information and the like related to sleep of a care receiver.

Moreover, the device 200 in the embodiment may be a care assistance recording device that automatically records a history of care assistance. For example, the care assistance recording device may be the device 200 that detects position information on at least either one of a care giver and a care receiver. The care assistance recording device performs, for example, processing of determining the time when a target person is present in the bed 610, the time when being in the toilet, the time when being in the bath, the time when being in the dining room, and the like, and storing what type of care assistance has been provided, and what frequency and how long the care assistance has been provided as a care assistance record based on the determination result. The care assistance recording device is useful, for example, for at-home care and the like in which a care assistance schedule and the like are not strictly set.

Moreover, the device 200 in the embodiment may include the reclining wheelchair 510 in which an angle of a backrest can be adjusted illustrated in FIG. 23, or the care bed 520 in which an angle of a section surface can be adjusted illustrated in FIG. 24. The reclining wheelchair 510 and the care bed 520 are control target devices illustrated in FIGs. 7 and 18, for example. For example, in a case where choking has been detected by the swallowing and choking detection device 460, an angle each of the backrest and the sections is controlled to an angle suitable for swallowing of the care receiver.

Moreover, the reclining wheelchair 510 may be controlled based on the processing results of the seat surface sensor 440 and the terminal device 450. For example, the wheelchair 630 illustrated in FIGs. 10 and 11 may be the reclining wheelchair 510 serving as a control target device. Similarly, the care bed 520 may be controlled based on the processing results of the bed side sensor 420, the detection device 430, the bed position detection device 470, the eyeglasses-type device 480, and the like. For example, the bed 610 illustrated in FIGs. 9, 13, and 14 may be the care bed 520 serving as a control target device.

In addition, various kinds of modifications are executable for the shape, the number and types of sensors, processing contents, and the like of the device 200 that is used in the embodiment.

### 5. Inter-Device Communication

In the above description, for example, as illustrated at Step S303 in FIG. 18, the server system 100 receives a processing result in the device 200. Moreover, as illustrated at Step S304 in FIG. 18, the server system 100 transmits a control signal for causing a control target device to perform control based on the processing result, to the control target device. Moreover, as illustrated at Step S306 in FIG. 18, if information for determining an operation mode has been updated, the server system 100 also executes a notification of the information to the device 200.

However, from the viewpoint of preventing a processing load in the server system 100 from excessively increasing, in the embodiment, at least either one of the control of a control target device based on a processing result in the device 200 and the notification of scene information and the like may be executed using a serverless function. Hereinafter, the description will be made while exemplifying the specific information processing system 10.

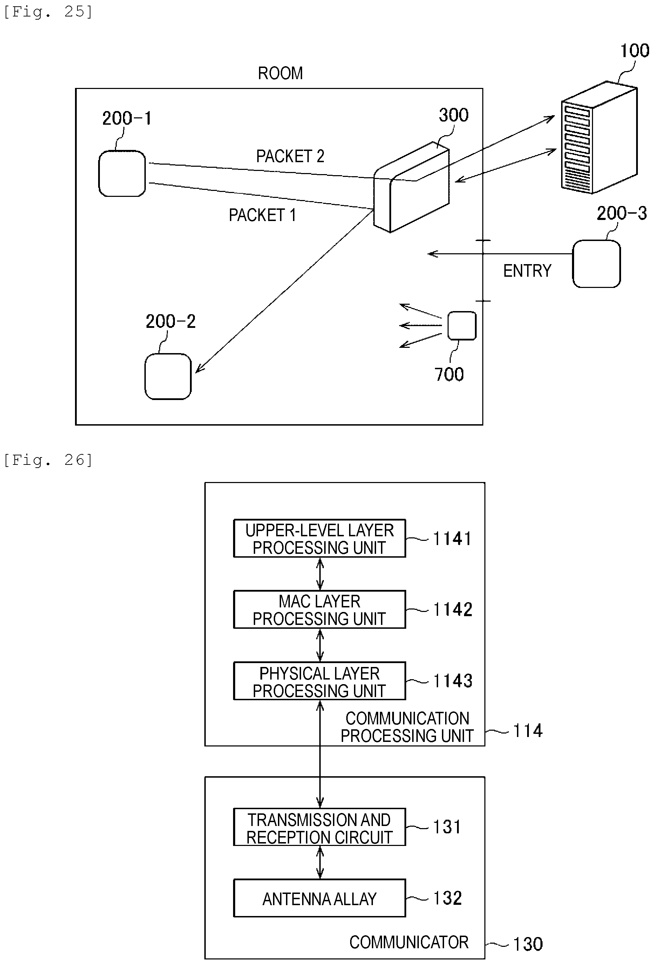

FIG. 25 is a diagram illustrating a configuration example of the information processing system 10 in a case where control using a serverless function is executed. The information processing system 10 in the embodiment may include an entry/exit management apparatus 700 that detects coming and going of the device 200 into and out of a given space, and the gateway 300 that relays communication between the device 200 positioned within the space and the server system 100. Moreover, similar to FIG. 1, the information processing system 10 includes the server system 100 and the device 200. In the example of FIG. 25, as the device 200, a device 200-1, a device 200-2, and a device 200-3 are exemplified.

As illustrated in FIG. 25, the gateway 300 is disposed, for example, in a room in the care facility or the like, and is an apparatus that allows transmission and reception of radio waves with the device 200 positioned at least in the room with sufficient intensity.

Moreover, the entry/exit management apparatus 700 illustrated in FIG. 25 is an apparatus that is disposed in the vicinity of an entrance of the room, for example, and detects entry or exit of the device 200. The entry/exit management apparatus 700 is an apparatus that performs near field communication, such as Bluetooth Low Energy (BLE), for example, and performs communication with a Bluetooth compatible apparatus positioned within a predetermined distance or shorter. For example, the entry/exit management apparatus 700 may be disposed in a room, and determine that the device 200 capable of performing the BLE communication with the entry/exit management apparatus 700 is positioned in the room.

Alternatively, the entry/exit management apparatus 700 may perform a more detailed determination. For example, a plurality of entry/exit management apparatuses 700 may be disposed in different positions in the room. Moreover, each of the plurality of entry/exit management apparatuses 700 performs communication with the device 200, and estimates a distance to the device 200 based on the radio field intensity (for example, RSSI: Received Signal Strength Indicator) in the communication. In this way, distances from a plurality of points the positions of which are known can be obtained, so that it is possible to estimate a position of the device 200. The entry/exit management apparatus 700 may determine whether the device 200 is positioned in the room based on the estimated position, and the size, shape, and the like of the room, which are known information. In addition, various kinds of modifications are executable for the specific determination on entry/exit.

The storing unit 220 of the device 200 stores information related to the device 200. The information related to the device 200 includes at least a device ID specifying the device 200. Moreover, the information related to the device 200 may include a part or all of the device information 122 mentioned above using FIG. 3. The entry/exit management apparatus 700 acquires information related to the device 200 serving as a communication target in the BLE communication with the device 200. In this way, the entry/exit management apparatus 700 can appropriately specify the device 200 that enters the target space.

Moreover, the storing unit 220 of the device 200 may store address information that is used by the target device 200 in the communication via the gateway 300. The address information herein may be, for example, an IP address, or another information (for example, MAC address) that can specify an IP address. The entry/exit management apparatus 700 acquires address information on the device 200 serving as a communication target in the BLE communication with the device 200.

Moreover, the entry/exit management apparatus 700 is not limited to an apparatus that uses BLE, but another communication scheme and the like, such as Near Field Communication (NFC), are widely applicable thereto.

The entry/exit management apparatus 700 includes a memory, which is not illustrated, and the memory may store existing device information specifying an existing device that is positioned in the room. In this way, it is possible to appropriately manage what kind of the device 200 is positioned in the target room. In the example of FIG. 25, the device 200-1 and the device 200-2 already entered the room. For example, when the device 200-1 has entered, the entry/exit management apparatus 700 executes processing of adding a device ID of the device 200-1 to existing device information based on the BLE communication with the device 200-1. Similarly, when the device 200-2 has entered, the entry/exit management apparatus 700 executes processing of adding a device ID of the device 200-2 to existing device information. Accordingly, a state where the device 200-1 and the device 200-2 are present in the room is held as the existing device information.

The entry/exit management apparatus 700 may perform processing of transmitting the holding existing device information to the already entered device 200. For example, the entry/exit management apparatus 700 transmits existing device information including the device IDs of the device 200-1 and the device 200-2, to the device 200-1. In this way, the device 200-1 can recognize that the device 200-2 is present in the room, in addition to the device 200-1 itself. Similarly, the entry/exit management apparatus 700 transmits existing device information to the device 200-2, whereby the device 200-2 can recognize that the device 200-1 is present in the room.

Moreover, the existing device information may be information in which a device ID is associated with address information. For example, the entry/exit management apparatus 700 transmits a device ID and address information of the device 200-1 and a device ID and address information of the device 200-2, to each of the devices 200. In this way, each of the devices 200 can acquire, in addition to the information indicating that another device 200 is present, address information when communication with another device 200 via the gateway 300 is performed.

As in the example in FIG. 25, the same applies to a case where the device 200-3 newly enters the room. In a case where the entry/exit management apparatus 700 has detected that a new device has entered into the space, the entry/exit management apparatus 700 may perform communication with the new device to acquire address information on the new device that is used in the communication via the gateway 300, and notify the device 200 positioned in the space of the acquired address information. For example, the entry/exit management apparatus 700 performs BLE communication with the device 200-3, thereby performing processing of adding a device ID and address information of the device 200-3 to the existing device information. Note that, the device ID and address information are not limited to those that are simultaneously acquired. For example, in a case where immediately after the entry, the connection between the device 200-3 and the gateway 300 has not been completed and a dynamic IP address has not been assigned, the device 200-3 may transmit address information to the entry/exit management apparatus 700 after waiting the connection establishment with the gateway 300.

After updating existing device information with the entry of the device 200-3, the entry/exit management apparatus 700 transmits the existing device information to the device 200-1 to the device 200-3. Accordingly, each of the devices 200 that are positioned in the room can specify another device 200 in the room, and address information.

Note that, the entry/exit management apparatus 700 may switch information to be transmitted between the existing devices (the device 200-1 and the device 200-2), and the newly entered new device (the device 200-3). For example, the entry/exit management apparatus 700 may transmit only the device ID and address information of the new device to the existing devices, and omit the transmission of the other records of the existing device information. Moreover, the entry/exit management apparatus 700 may transmit the existing device information before an update to the new device. This can omit the transmission of information with low necessity, so that it is possible to reduce a communication load.

Moreover, the same applies to a case where any of the devices 200 exits. For example, the entry/exit management apparatus 700 determines that the device 200 with which the BLE communication becomes impossible, or the device 200 the estimated position of which is determined to be out of the room, has exited from the target room. In this case, the entry/exit management apparatus 700 performs processing of deleting the device ID and the address information of the exited device 200 from the existing device information. Moreover, the entry/exit management apparatus 700 notifies the existing device of information indicating that the exit of the device 200 has occurred. For example, the entry/exit management apparatus 700 may transmit updated existing device information to each of the devices 200. Moreover, the entry/exit management apparatus 700 may transmit the device ID and the like of the exited device 200 to each of the devices 200, and instruct each of the devices 200 to delete the corresponding record from the existing device information that is held by each of the devices 200.

Moreover, the entry/exit management apparatus 700 is not limited to storing existing device information on a device that is positioned in the target space, but may store log data on entry/exit. The log data is, for example, information associated with the entry time, the exit time, the device ID, and the like.

Next, serverless communication will be described. For example, it is assumed that the device 200-1 operates in an operation mode indicating an active state, and as a processing result thereof, control of the device 200-2 is determined to be necessary. For example, the device 200-1 is the swallowing and choking detection device 460, and dangerous choking has been detected, so that it is determined that a change in the section angle of the device 200-2 serving as the care bed 520 is necessary.

In the example mentioned above using FIGs. 7 and 18, the processing via the server system 100 has been performed. Further, in the example in FIG. 25, as mentioned above, the device 200-1 knows that the device 200-2 is present in the same room, and also knows an IP address thereof. Therefore, the device 200-1 may transmit a control signal to the device 200-2 without passing through the server system 100. Specifically, the device 200-1 transmits a control signal to the gateway 300 using a packet with the IP address of the device 200-2 designated. The gateway 300 determines that the transmission destination of the packet is the device 200-2 that is an apparatus in the same network by the determination based on the IP address. Therefore, the packet including the control signal is transmitted to the device 200-2 without passing through the server system 100, as "PACKET 1" illustrated in FIG. 25. Accordingly, control of the necessary device 200 can be executed without passing through the server system 100, so that it is possible to reduce a load on the server system 100, and shorten the time to be required for the control.

Moreover, the scene information and the device type information are not limited to those that are obtained by the processing unit 110 of the server system 100. For example, in a case where the number of care givers is used as scene information, if the device 200-1 is the imaging device 410 or the like, it is also possible to obtain the number of care givers in the room based on the image processing to a taken image. Moreover, the device type information is information indicating a type of another device 200 that is used together with a given device 200. Therefore, there is a possibility that the device type ID of the device 200-1 becomes device type information on the device 200-2 and the device 200-3 that operate in the same room.

Moreover, in the device 200 in the embodiment, obtaining ability information such as an ADL indicator is not prevented. For example, as mentioned above, a log of sensing data can be used for the calculation of ability information, so that the device 200 that acquires the sensing data may calculate ability information.

In the examples mentioned above using FIGs. 7 and FIG. 18, ability information, scene information, and device type information are obtained in the server system 100, and data including these is transmitted to the device 200. Further, in the example in FIG. 25, the device 200-1 knows that the device 200-2 is present in the same room, and also knows an IP address thereof. Therefore, the device 200-1 transmits the ability information, the scene information, the device type information, and the like to the device 200-2 without passing through the server system 100. Specifically, similar to the abovementioned control signal, the device 200-1 can transmit the ability information and the like to the device 200-2 using a packet with the IP address of the device 200-2 designated, without passing through the server system 100. Accordingly, an operation mode in accordance with the ability information and the like can be set without passing through the server system 100, so that it is possible to reduce a load on the server system 100, and shorten the time to be required for the control. For example, each of a plurality of devices 200 that are positioned in the room may execute processing of specifying a combined device by mutually notifying the other devices 200 of device type information on the device 200 itself.

Moreover, the device 200-1 may transmit log data related to control with respect to a control target device (for example, the device 200-2) to the server system 100. In the abovementioned example, data and the like indicating that when and what extent the device 200-1 that is the swallowing and choking detection device 460 has changed a back angle of the device 200-2 that is the care bed 520, serve as log data. Similarly, in a case where ability information and the like have been notified to the device 200-2, the timing of the notification and details of the ability information and the like, which are notification contents, serve as log data. These pieces of the log data are transmitted to the server system 100, whereby even in a case where the serverless communication has been performed, it is possible to share contents thereof with the server system 100.

For example, as illustrated in FIG. 25, the device 200-1 may transmit a control signal, ability information, scene information, device type information, and the like using a packet 1, and transmit log data using a packet 2. The log data herein is data indicating a history of the communication using the packet 1, as mentioned above. Moreover, the packet 1 is a packet that is transmitted and received in the network to which the device 200-1 belongs. The packet 2 is a packet that is transmitted over the gateway 300 forming the network to which the device 200-1 belongs. The packet 2 may be a packet that designates an IP address of the server system 100 in a narrow sense. The packets are switched in accordance with the content of data to be transmitted in this manner, whereby it is possible to reduce a communication load on the server system 100.

Note that, in the foregoing, the example in which the serverless data transmission from the device 200-1 to another device 200 is performed has been indicated, but another device 200 such as the device 200-2 may become a transmission source of data.

As is in the foregoing, the device 200 in the embodiment may select whether transmitting an operation result to the server system 100 (at Step S203 and the like in FIG. 7), or transmitting, based on the operation result, without passing through the server system 100, to a control target device different from the device 200, information for controlling the control target device (the packet 1 in FIG. 25). In this way, whether performing processing to be led by the server system 100 or performing serverless processing can be flexibly changed.

Specifically, in a case where address information on a control target device has been acquired based on the information from the entry/exit management apparatus 700, the device 200 may control the control target device without passing through the server system 100. In this way, after a specific communication situation is considered, it is possible to appropriately determine whether serverless processing is performed.

Note that, in the foregoing, the example in which serverless communication is performed using the entry/exit management apparatus 700 has been described, but the method in the embodiment is not limited thereto. For example, the server system 100 may determine, by referring to a packet to be transmitted from the device 200, via which gateway 300 the packet as data is transmitted. Moreover, in a case where a plurality of devices 200 connected to the same gateway 300 are present, the server system 100 may determine that the plurality of devices 200 are present within a close distance.

Moreover, the server system 100 transmits address information on another device 200 in which a close distance from the device 200 has been determined, to each of the devices 200. For example, the server system 100 obtains information in which identification information and address information on a plurality of devices 200 to be connected to the same gateway 300 are associated with each other, and periodically transmits the information to the abovementioned plurality of devices 200. With such a method, each of the devices 200 also can specify address information on another device 200 present near, and thus can implement serverless communication.

### 6. Detailed Example of Communication

### 6.1 Overview

In the information processing system 10 illustrated in FIG. 1, communication using a wireless communication scheme is performed between at least one device 200 and the gateway 300. The wireless communication scheme herein may be, for example, a communication scheme in conformity with IEEE802. 11. Moreover, the server system 100 may perform communication using a wired communication scheme, or may perform communication using a wireless communication scheme. The wired communication scheme herein may be, for example, a communication scheme in conformity with IEEE802. 3. Hereinafter, for simple description, an example in which each of the server system 100, the device 200, and the gateway 300 uses the communication scheme in conformity with IEEE802. 11 will be described.

FIG. 26 is a diagram illustrating configuration examples of the communication processing unit 114 and the communicator 130 of the server system 100. As illustrated in FIG. 26, the communication processing unit 114 includes an upper-level layer processing unit 1141, an MAC layer processing unit 1142, and a physical layer processing unit 1143. The communicator 130 includes a transmission and reception circuit 131 and an antenna array 132. The configurations of the communication processing unit 114 and the communicator 130 are not limited to those in FIG. 26, but various modifications such as omitting a part of the configuration, adding another configuration, and the like can be made.

The upper-level layer processing unit 1141 performs processing in an upper-level layer from the MAC layer. The upper-level layer herein may be Transmission Control Protocol/Internet Protocol (TCP/IP), may be User Datagram Protocol/Internet Protocol (UDP/IP), or may be an application layer. For example, processing of obtaining ability information, scene information, and device type information mentioned above are executed by software that operates on the server system 100, and the application layer herein may correspond to the software. The upper-level layer processing unit 1141 is connected to the MAC layer processing unit 1142.

The MAC layer processing unit 1142 performs transmission processing and reception processing in the MAC layer. The MAC layer processing unit 1142 is connected to the physical layer processing unit 1143.

The physical layer processing unit 1143 performs processing in the physical layer. The physical layer processing unit 1143 is connected to the antenna array 132 via the transmission and reception circuit 131.

The transmission and reception circuit 131 includes circuits such as a digital/analog conversion circuit (hereinafter, expressed as D/A conversion circuit) and an RF circuit, and converts a digital signal from the physical layer processing unit 1143 into an analog signal and outputs the analog signal to the antenna array 132. Moreover, the transmission and reception circuit 131 includes an analog/digital conversion circuit (hereinafter, expressed as A/D conversion circuit), and converts a signal received by the antenna array 132 into a digital signal and outputs the digital signal after the conversion to the physical layer processing unit 1143. Note that, the physical layer processing unit 1143 is not prevented from having the A/D conversion circuit or the D/A conversion circuit.

The antenna array 132 includes a plurality of antennas that perform the transmission of radio waves based on analog signals output from the transmission and reception circuit, and output of analog signals based on the received radio waves.

For example, in the data reception, the respective units illustrated in FIG. 26 operate as follows. Firstly, the antenna array 132 receives radio waves in a predetermined frequency band, and outputs an analog signal corresponding to the radio waves to the transmission and reception circuit 131. The transmission and reception circuit 131 converts the analog signal received by the antenna array 132 into a base band signal. Moreover, the transmission and reception circuit 131 performs A/D conversion of the base band signal, and outputs a digital signal after the conversion to the physical layer processing unit 1143.

The physical layer processing unit 1143 performs processing such as demodulation, error correction, and the like of the received signal. Moreover, the physical layer processing unit 1143 removes a physical header that is a header corresponding to a physical layer, and outputs a payload portion to the MAC layer processing unit 1142.

The MAC layer processing unit 1142 processes a received payload portion as an MAC frame. The MAC frame may be a MAC Protocol data unit (MPDU) in IEEE802. 11. The MAC layer processing unit 1142 delivers a frame body, which is a portion in which an MAC header serving as a header in the MAC layer and a trailer are excluded from the MAC frame, to the upper-level layer processing unit 1141.

The upper-level layer processing unit 1141 outputs data corresponding to the frame body received from the MAC layer processing unit 1142, to software and the like.

Moreover, processing reverse to this processing is executed in the data transmission. Firstly, data to be transmitted is generated by software that operates on the server system 100, the data is output to the upper-level layer processing unit 1141.

The upper-level layer processing unit 1141 assigns a header and the like in the upper-level layer to the data to create data corresponding to a frame body, and outputs the created data to the MAC layer processing unit 1142.

The MAC layer processing unit 1142 assigns a MAC header and a trailer to the received data to create a MAC frame. The MAC layer processing unit 1142 outputs the created MAC frame to the physical layer processing unit 1143.

The physical layer processing unit 1143 assigns a physical header and the like to the received data to create a physical packet. The physical layer processing unit 1143 outputs the created physical packet to the transmission and reception circuit 131.

The transmission and reception circuit 131 performs the A/D conversion, the modulation, and the like of the data received from the physical layer processing unit 1143 to generate an analog signal. The transmission and reception circuit outputs the analog signal to the antenna array 132. The antenna array 132 sends out radio waves corresponding to the received analog signal.

The MAC layer processing unit 1142 may use a data frame, a control frame, and management frame as the abovementioned MAC frame. The data frame is a frame that is used when, in a state where a communication link between terminals is established, data is transmitted and received between the terminals. For example, as mentioned above, in a case where software that operates on the server system 100 creates data including ability information and the like, and transmits the data to the device 200, the data frame is used.

Moreover, the management frame is a frame for managing a communication link between terminals. Note that, various kinds of information that is transmitted and received using the management frame are defined in IEEE802. 11, and are widely applicable in the embodiment as well. The control frame is a frame that is used for transmission and reception control of the management frame and the data frame. The control frame includes various kinds of frame, such as a Request To Send (RTS) frame, a Clear to Send (CTS) frame, an Acknowledgement (ACK) frame, and the like. These frames are defined within the standard of IEEE802. 11, and detailed descriptions are thus omitted.

Moreover, in the foregoing, the configuration related to the communication of the server system 100 has been exemplified, and the similar configuration may be used for the device 200 and the gateway 300. Moreover, as mentioned above, the server system 100 may perform communication using the wired communication scheme, and in that case, it is possible to replace a part of the abovementioned content with a content in conformity with IEEE802. 3, for example.

### 6.2 Frame Configuration

In the method in the embodiment, the configuration of the MAC frame (data frame) may be made common independent of the device 200 serving as a communication target. As mentioned above using FIG. 7 and the like, the device 200 in the embodiment and an application that operates in the device 200 may be created by a device vendor. Therefore, the devices 200 created by various kinds of vendors are registered in the information processing system 10 in some cases. In that regard, the configuration of a data frame is made common, whereby it is possible to standardize the communication control independent of the vendors. Note that, the configuration of a data frame is bit allocation in the frame body in a narrow sense.

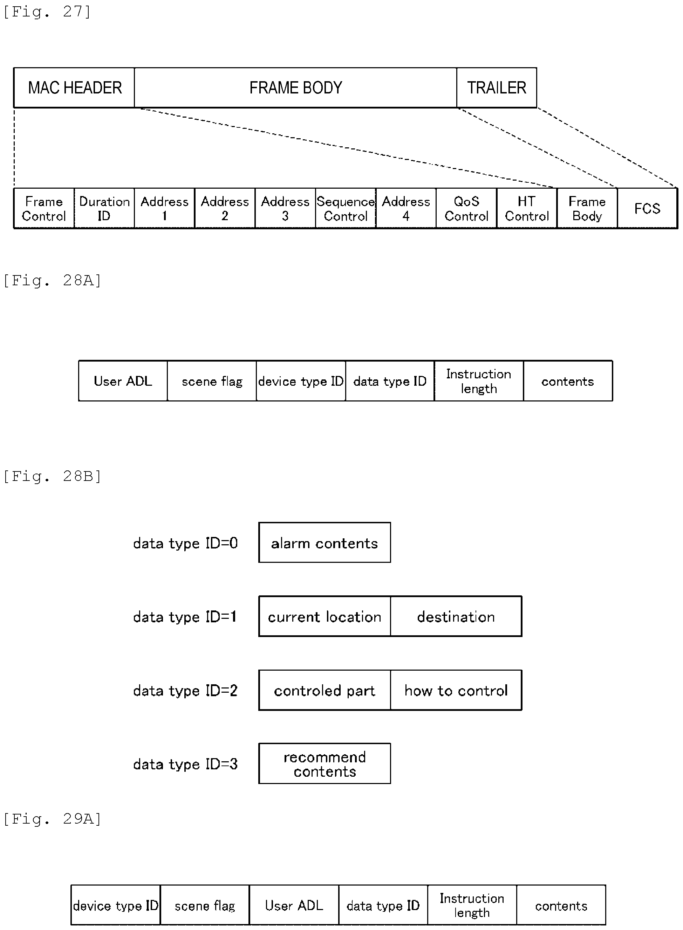

FIG. 27 illustrates a format example of a MAC frame. Note that, the format example illustrated in FIG. 27 is applicable to a management frame and a control frame, and the description will be made using a data frame as an example hereinafter. As illustrated in FIG. 27, the MAC frame includes a MAC header, a frame body, and a trailer.

The MAC header includes respective fields of Frame Control, Duration ID, Address 1, Address 2, Address 3, Sequence Control, Address 4, QoS Control, and HT Control. Moreover, a part of these may be omitted.

The field of Frame Control includes a type field for determining whether a target MAC frame is a data frame, a management frame, or a control frame. Moreover, the field of Frame Control may include a subtype field for specifying a more detailed type. Information indicating a schedule period during which radio waves are used is stored in the field of Duration/ID. The schedule period during which radio waves are used may be expressed as time necessary for frame transmission. The field of Duration/ID is used in RTS/CTS and the like.

The fields of Address 1 to Address 4 each store information indicating an address of an apparatus in a reception destination or a transmission source. For example, Address 1 corresponds to a reception destination address, and Address 2 corresponds to a transmission source address. Data in accordance with a frame usage is stored in each of the fields of Address 3 and Address 4.

The field of Sequence Control corresponds to a sequence number of data to be transmitted. The field of QoS Control stores information that is used in QoS control. The QoS control indicates control in which the transmission is performed by considering the degree of priority of the frame. The field of HT Control is a field that is used in a management frame, for example.

The configuration of the frame body will be described later using FIGs. 28A and 28B. Moreover, the trailer is Frame Check Sequence (FCS), for example. FCS is information that is used in error detection of the frame, and a check sum code, for example. FSC is Cyclic Redundancy Code (CRC), for example.

The information processing system 10 according to the embodiment includes the server system 100 and the device 200 as mentioned above using FIG. 1. The device 200 includes an application that executes processing corresponding to tacit knowledge of a skilled worker. As illustrated in FIG. 27, the server system 100 transmits a data frame including a Media Access Control (MAC) header, a frame body, and a trailer in a data link layer in the communication with the device 200. The server system 100 transmits herein a data frame in which a frame body includes a fixed-length region including a first region that stores ability information indicating an activity ability of a care receiver, a second region that stores scene information specifying a scene of care assistance for the care receiver, and a third region that stores device type information specifying a type of a combined device to be used with the device 200. Moreover, as mentioned above, the device 200 determines an active or inactive state of an application based on at least one of the ability information, the scene information, and the device type information.

In this way, it is possible to standardize a data format when the server system 100 transmits the ability information and the like to the device 200. For example, even in a case where the devices 200 by various kinds of vendors are registered in the information processing system 10 in the embodiment, the configuration of the data frame can be standardized independent of the vendors. In particular, the first to third regions each have a fixed length to allow the device 200 having received a data frame to easily interpret the data frame, so that it is possible to reduce a processing load related to the communication. For example, the first to third regions may be disposed in a head part excluding a header and the like in the upper-level layer, in the frame body.

Note that, as illustrated at Step S203 in FIG. 7, if an application is set to be active, the device 200 may transmit an operation result of the application to the server system 100. The operation result herein is output from the application, and indicates an execution result by the processing corresponding to the tacit knowledge. For example, the operation result includes a determination result related to any one of an event that can occur in the care assistance, a care receiver, and a care giver. Specifically, the operation result may be a result of a determination as to whether a some sort of event has occurred in the care assistance, a result of a determination as to whether the state of the care receiver is normal, or a result of a determination as to whether the care assistance by the care giver is ground truth. In this way, it is possible to compile the information indicating the operation results to the server system 100. Further, the processing via the server system 100 is not indispensable, but the serverless processing may be executed as mentioned above using FIG. 25.

FIGs. 28A and 28B are diagrams illustrating bit allocation of a frame body in a data frame that is transmitted by the server system 100. As illustrated in FIG. 28A, the frame body may include respective fields of User ADL, scene flag, device type ID, data type ID, Instruction length, and contents.

The field of User ADL is a field that stores ability information on a care receiver, and corresponds to the abovementioned first region. For example, the ability information is numerical value data indicating, in a case where the degree of the ability is sectioned in a predetermined number of stages, to which stage the ability of a care receiver belongs. For example, if the number of stages is equal to or less than eight, the User ADL is a 3-bit field. Moreover, in a case where the number of stages is equal to or more than nine, the User ADL may be a 4-bit or more field. The number of bits necessary in accordance with the definition of the ability information is known, so that the field of User ADL may be a fixed-length field. The User ADL may include an ID for specifying a care receiver. Moreover, as mentioned above at Step S403 in FIG. 19 and the like, the ability information in the embodiment is not limited to an indicator value of ADL, but may be more detailed information such as information indicating the abovementioned way of standing-up, a sitting posture holding ability, a swallowing ability, and a walking ability. Therefore, the field of User ADL may be a field having the number of bits that can represent these respective abilities.

The field of Scene flag is a field that stores scene information, and corresponds to the abovementioned second region. For example, the scene information may include a bit indicating whether the number of care givers is equal to or more than a predetermined number. A case where the bit is a first value (for example, 0) indicates that the number of care givers is equal to or more then the predetermined number, and a case of a second value (for example, 1) indicates that the number of care givers is less than the predetermined number. Moreover, the scene information may include a bit for specifying a type of care assistance. For example, in a case where as types of care assistance, four types of meal care assistance, excretion care assistance, movement and transfer care assistance, and another are identified, scene information includes 2 bits as a bit for specifying a type of care assistance. For example, a case where the 2 bits are 00 indicates meal care assistance, a case of 01 indicates excretion care assistance, a case of 10 indicates movement and transfer care assistance, and a case of 11 indicates another. Moreover, the scene information is not limited to these, but another information may be used. Therefore, various kinds of modifications are also executable for the specific number of bits and the meaning of the Scene flag. Further, what kind of scene information is used is known, and the number of bits necessary for the expression of the scene information is also known, so that the field of Scene flag may be a fixed-length field.

The field of device type ID is a field that stores device type information, and corresponds to the abovementioned third region. The device type information may be different information for each of the devices 200 mentioned above using FIGs. 8 to 14. Alternatively, as for the device 200 that responds to a falling-down risk, the same device type ID may be assigned to the imaging device 410 in FIG. 8 and the bed side sensor 420 in FIG. 9. Alternatively, a device type ID may be assigned based on the type and the like of a sensor included in the device 200. The number of target device type IDs is known, so that the field of device type ID may be a fixed-length field.

Moreover, the server system 100 may obtain, based on an operation result in the device 200, a control type and a control content of control with respect to a control target device. Moreover, the server system 100 may transmit a data frame in which the frame body includes a fixed-length fourth region that stores a control type, and a fifth region that stores a control content and has a different length in accordance with the control type in a rear portion from the region including the first region, the second region, and the third region, to the control target device. The control type indicates a type of control to be executed, and the control content indicates information specifically specifying a content of the control to be executed. In this way, it is possible to use a data frame common to both of the notification of ability information and the like, and the control of the control target device. At this time, the first to third regions that are fixed-length fields are disposed relatively in front, and the fifth region that is a variable-length field is disposed relatively in the rear, whereby the data structure in front can be fixed so as to include the number of bits. As a result, the interpretation processing of the data frame in the device 200 becomes easy.

For example, data type ID, Instruction length, and contents illustrated in FIG. 28A are fields that are used for the transmission of a control signal to the control target device. The field of datatype ID is a field that stores information indicating the type of an instruction to be output to the control target device, and corresponds to the abovementioned fourth region. The instruction herein may include four instructions of "notification (alarm)", "movement/transportation", "control", and "recommend and the like". In this case, the field of data type ID is a 2-bit fixed-length field that can identify the four instructions.

The "notification" is information that is used when a processing result in the device 200 is notified in the control target device. For example, the "notification" may be an instruction that is used, in a case where a falling-down risk has been detected in a device that determines a falling-down risk, when the fact is notified in the control target device. The "movement/transportation" is an instruction that moves a movable control target device such as the reclining wheelchair 510 and a wheeled walker. For example, in a case where a falling-down risk has been detected, the instruction of movement/transportation may be used for control to move a wheeled walker and the like closer to a target care receiver so as to allow the care receiver to hold thereon. The "control" widely incudes control other than the "movement/transportation" that causes a control target device to operate, and incudes an angle change of a back surface part of the reclining wheelchair 510, a change in a section angle of the care bed 520, and the like. The "recommend and the like" includes, for example, recommendation of a purchase of a product that is used for improvement in the quality of care assistance. For example, the recommendation may indicate, in a case where a determination of recommending a use of a cushion has been made in the bed position detection device 470, an instruction to output the fact to a control target device that is a terminal device or the like of a care giver. Moreover, the "recommend and the like" may include news distribution and the like. For example, the "recommend and the like" may be used for an introduction of the popular device 200.

The field of contents indicates a field that stores information specifying a specific content of an instruction, and corresponds to the abovementioned fifth region. FIG. 28B is a diagram illustrating specific examples of contents in accordance with values of data type ID. For example, data type ID = 0 indicates the abovementioned "notification", and the field of contents in this case includes an alarm contents field. The field of alarm contents indicates a variable-length field that stores information indicating a specific notice content. The field of alarm contents may include information specifying a notification mode (display, light emitting, vibration, or voice output), or may include information specifying a specific notification content (text, light emitting color, vibration pattern, or voice).

Data type ID = 1 indicates the abovementioned "movement/transportation", and the field of contents in this case includes respective fields of current location and destination. Current location is information specifying a current position of the target device 200. Destination is information specifying a target position of the target device 200. How to represent a current position and a target position is arbitrary, and is, for example, a fixed-length field indicating a two-dimensional or three-dimensional coordinate value.

Data type ID = 2 indicates the abovementioned "control", and the field of contents in this case includes respective fields of controlled part and how to control. The field of controlled part stores information specifying a control target location of the target device 200. For example, the care bed 520 illustrated in FIG. 24 includes a plurality of sections, each of which is movable. The field of controlled part may store information specifying any of the plurality of sections. The field of how to control stores information specifying a specific control method of a control target location. In the example of the care bed 520, how to control may be information indicating to which direction and how many times the section specified by the controlled part is driven. Each of the fields of controlled part and how to control may be a fixed-length field. Note that, the "control" herein may include instructions to a plurality of control target locations. Therefore, in a case of data type ID = 2, contents may be configured to repeat a pair of controlled part and how to control for the number of times of control target locations.

Data type ID = 3 indicates the abovementioned "recommend and the like", and the field of contents in this case includes a recommend contents field. The field of recommend contents is a variable-length field in which contents of recommend and news are indicated. Recommend contents herein may include information specifying a product to be recommended, or may include link information to a sale site of the product, a product introduction Webpage by the manufacturer, and the like. Moreover, recommend contents may include text indicating the content of news, link information to a Webpage in which news is displayed, and the like.

Note that, as is in the foregoing, the length of the contents field in the frame body differs in accordance with data type ID and the specific instruction content. Therefore, as illustrated in FIG. 28A, before the contents field, the frame body may include a instruction length field that stores the length of the contents field . It can be considered that the maximum length of the contents field is known, so that the field of instruction length may be a fixed-length field, for example.

Moreover, as mentioned above using FIG. 25, in the embodiment, data including a control signal, ability information, and the like may be transmitted from the device 200 to another device 200 without passing through the server system 100. Further, in a case where format of data to be transmitted from the server system 100 (Step S304 in FIG. 7, and the like) and data to be transmitted from the device 200 (the packet 1 in FIG. 25) are largely different from each other, there is a possibility that interpretation processing of the data in accordance with the transmission source becomes necessary in the device 200 that receives the data. Therefore, in a case where the device 200 becomes a transmission source as well, a frame configuration according to the frame configuration illustrated in FIGs. 28A and 28B may be used.

For example, in a case where the device 200 controls a control target device without passing through the server system 100, in a data link layer of the communication with the control target device, the device 200 may transmit a data frame in which the frame body includes a fixed-length region including a second region and a third region, to the control target device. As mentioned above, the second region corresponds to scene information, and the third region corresponds to device type information. Moreover, the data frame is not prevented from including a first region corresponding to ability information. In this way, it is possible to make portions corresponding to at least ability information, scene information, and device type information common in the frame body of the data frame. For example, the first to third regions may be disposed in a head part excluding a header and the like in the upper-level layer, in the frame body.

Moreover, the device 200 may obtain, based on an operation result of an application in the device 200, a control type and a control content of control with respect to a control target device. Moreover, the device 200 may transmit a data frame including a fourth region and a fifth region in a rear portion from the region including the second region and the third region, in the frame body, without passing through the server system 100 to the control target device. As mentioned above, the fourth region corresponds to a control type and the fifth region corresponds to a control content. In this way, in a case where the serverless control is performed, it is possible to make the configuration of the data frame to be received by the device 200 common.

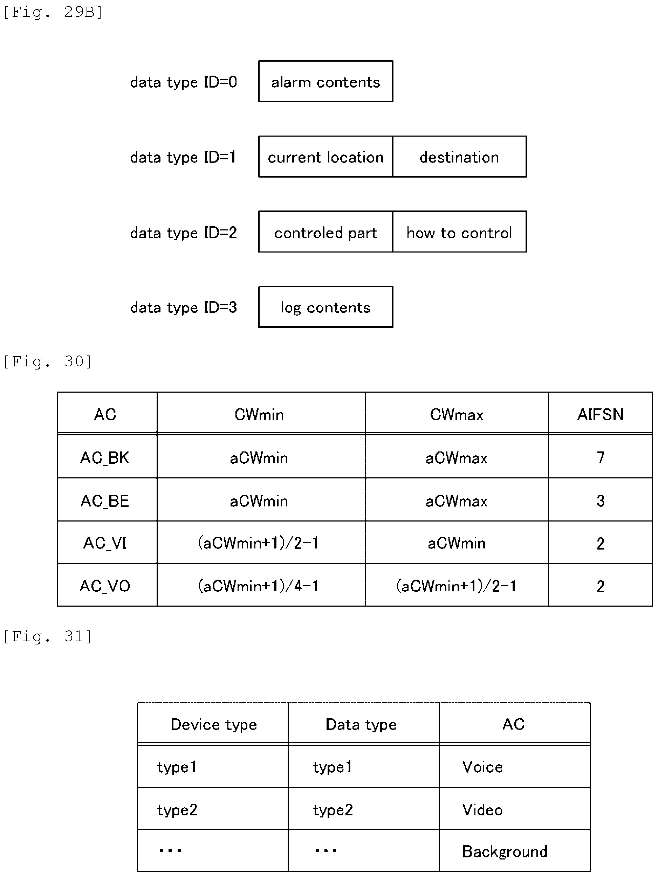

FIGs. 29A and 29B are diagrams illustrating a configuration example of a frame body when the device 200 transmits data including a control signal, ability information, and the like to another device 200. As is understood from a comparison between FIG. 29A and FIG. 28A, the configuration of the frame body is similar to the configuration of the frame body when the server system 100 transmits data, except that the order of device type ID, scene flag, and User ADL is different. The device type ID field herein may store, for example, device type information on the device 200 as a transmission source. FIG. 29B is a diagram illustrating specific examples of contents in accordance with values of data type ID. Except that log data is transmitted instead of recommend and the like in the case of data type ID =3 because the device 200 is a transmission source, the content of contents is similar to that in FIG. 28B. The log data is as mentioned above, and includes information such as a history when serverless control of the control target device is performed, and a notification history and the like such as ability information and the like.

In this way, even in any one of the case where the server system 100 is a transmission source, and the case where the device 200 is a transmission source as illustrated in FIG. 25, it is possible to make the configurations of the data frames similar to each other. As a result, a determination on an operation mode and an operation in accordance with a control signal become possible in the device 200 having received a data frame, based on the similar processing, independent of the transmission source. Therefore, even in a case where both of the processing using the server system 100 as a starting point and the serverless processing are performed, an increase in a processing load can be suppressed.

Note that, various kinds of modifications are executable for the order of the respective fields in FIG. 28A and FIG. 29A, in particular, the order of User ADL, device type ID, and scene flag. Therefore, the configuration of the data frame to be transmitted by the server system 100 and the configuration of the data frame to be transmitted by the device 200 are not prevented from being the same. Moreover, in at least either one of the configuration of the data frame to be transmitted by the server system 100 and the configuration of the data frame to be transmitted by the device 200, a part of User ADL, device type ID, and scene flag may be omitted. For example, in the foregoing, the example in which the device 200 outputs sensing data, and the ability information acquisition unit 111 of the server system 100 calculates ability information based on the sensing data has been described. In this case, ability information is not calculated in the device 200, so that User ADL may be omitted from the data frame to be transmitted by the device 200. Alternatively, a User ADL field may be provided in a data frame to be transmitted by the device 200, and a value of the field may be then set to a fixed value (for example, all 0). Moreover, the device 200 is not prevented from calculating ability information, and in this case, information such as an indicator value of ADL is stored in User ADL of the data frame to be transmitted by the device 200.

Moreover, the data frame mentioned above using FIGs. 29A and 29B may be used for the data transmission from the device 200 to the server system 100. For example, in a case where the device 200 has been determined that a notification is necessary as a processing result, the device 200 transmits a data frame in which a value of data type ID is set to 1, and information indicating the notification content is stored in the field of alarm contents in the field of contents, to the server system 100. In the other cases, the device 200 can appropriately transmit, by changing a value of data type ID and a value of contents in accordance with a processing result, the processing result using the data frame illustrated in FIGs. 29A and 29B to the server system 100. Moreover, in the case of data type ID = 3, log data to be stored in the field of contents is not limited to a log in the serverless communication, but may include a log of the sensing data. In other words, when an log of the sensing data is transmitted to the server system 100, the data frame illustrated in FIGs. 29A and 29B may be used.

A method in the embodiment can be applied to an information processing apparatus. The information processing apparatus corresponds to the server system 100, for example. The information processing apparatus includes a communicator (which corresponds to the communicator 130 in FIG. 3) that transmits a data frame including a media access control (MAC) header, a frame body, and a trailer, in a data link layer of communication with the device 200 including an application that executes processing corresponding to the tacit knowledge of a skilled worker, and a communication processing unit (which corresponds to the communication processing unit 114 in FIG. 3) that controls the communicator. The communicator transmits a data frame in which a frame body includes a fixed-length region including a first region that stores ability information indicating an activity ability of a care receiver, a second region that stores scene information specifying a scene of care assistance for the care receiver, and a third region that stores device type information specifying a type of a combined device to be used with the device, as information determining an active or inactive state of an application, to the device 200. In this way, it is possible to standardize a data format when the server system 100 transmits the ability information and the like to the device 200, independent of a vendor and the like of the device 200.

Moreover, a method in the embodiment can be applied to an information processing method in the information processing system 10 that includes the device 200 including an application that executes processing corresponding to the tacit knowledge of a skilled worker, and the server system 100 that transmits a data frame including a media access control (MAC) header, a frame body, and a trailer, in a data link layer of communication with the device 200. The information processing method includes a step of transmitting a data frame in which a frame body includes a fixed-length region including a first region that stores ability information indicating an activity ability of a care receiver, a second region that stores scene information specifying a scene of care assistance for the care receiver, and a third region that stores device type information specifying a type of a combined device to be used with the device 200, from the server system 100 to the device 200, and a step of determining an active or inactive state of an application, based on at least one of the ability information, the scene information and the device type information.

### 6.3 Degree of Priority

Moreover, in various kinds of communication methods, methods of setting a degree of priority of communication are known. For example, in IEEE802. 11, a method of implementing QoS in IEEE802. 11e is standardized. Specifically, in IEEE802. 11e, the method called Enhanced Distributed Channel Access (EDCA) in which a frame having a high degree of priority is transmitted with priority is used.

For example, a frame body of a management frame in the MAC frame may include an EDCA parameter set element that is used in EDCA. FIG. 30 is a diagram illustrating an example of a default EDCA parameter set element. In EDCA, a packet is classified into four access categories (hereinafter, expressed as AC). The default access categories are four of AC_VO, AC_VI, AC_BE, and AC_BK. AC_VO corresponds to voice, AC_VI corresponds to video, AC_BE corresponds to best effort, and AC_BK corresponds to background. AC_VO has the highest degree of priority, and the degree of priority decreases in the order of AC_VI, AC_BE, and AC_BK.

Moreover, in each AC, parameters such as CWmin, CWmax, and AIFSN are associated. CWmin and CWmax are parameters for determining transmission waiting time, and indicate the minimum value and the maximum value of Contention Window (hereinafter, expressed as CW). The transmission waiting time is set to a value between CWmin and CWmax. The transmission becomes easier as the transmission waiting time is shorter, so that smaller values of CWmin and CWmax are set as the degree of priority is higher.

For example, in a case where CWmin is set as aCWmin and CWmax is set as aCWmax in each of AC_BE and AC_BK with a low degree of priority, CWmin is set as (aCWmin+1)/2 - 1 and CWmax is set as aCWmin in AC_VI with a high degree of priority, so that values of CWmin and CWmax become smaller than those in the abovementioned two ACs. Moreover, in AC_VO with the highest degree of priority, CWmin is set as (aCWmin+1)/4 - 1 further shorter than that in AC_VI, and CWmax is set as (aCWmin+1)/2 - 1 further shorter than that in AC_VI.

Moreover, Arbitration Inter Frame Space Number (AIFSN) is a parameter indicating a transmission interval of a frame, and the degree of priority of a queue becomes higher as the value is smaller. In the example of FIG. 30, AIFSN in each of AC_VO and AC_VI is 2, AIFSN in AC_BE is 3, and AIFSN in AC_BK is 7.

Moreover, parameters to be associated with AC are not limited to those in the above. For example, a Transmission Opportunity (TXOP) limit, which is not illustrated in FIG. 30, may be associated with each AC. TXOP indicates occupied time of the channel. For example, in AC_VI and AC_VO with a high degree of priority, a value that is not 0 is set to the TXOP limit, whereby it is possible to occupy the channel for the transmission of the packets set in these ACs.

It is assumed that the information processing system 10 in the embodiment includes a large number of the devices 200. Moreover, each of the devices 200 executes an operation in accordance with the operation mode, and transmits a processing result to the server system 100 via the gateway 300. As the processing result herein, various kinds of contents such as "notification", "movement/transportation", "control", and "log" mentioned above using FIG. 29B can be considered.

There is a possibility that the devices 200 in the embodiment may include products by various kinds of vendors, and there is also a possibility that setting of the degree of priority varies in accordance with the vendors. For example, there is also a possibility that a higher degree of priority is given to only the communication of the device 200 by a specific vendor than the communication of the devices 200 by the other vendors. Therefore, in the embodiment, the efficiency of the communication may be attained by setting the degree of priority. For example, in the method in the embodiment, the allocation of ACs is updated.

The server system 100 in the embodiment may obtain a degree of priority based on the device type information, and transmit priority degree information specifying the degree of priority to the device. The priority degree information is, for example, information in which device type information is associated with the degree of priority, and more specifically information in which AC is allocated for each device type. Moreover, the device 200 transmits a data frame in accordance with the degree of priority specified based on the type of the device and the priority degree information. In this way, the degree of priority in accordance with the device type can be set, so that the efficiency of the communication in the information processing system 10 including various kinds of the devices 200 can be increased.

In addition, the server system 100 may obtain a degree of priority based on the device type information and the control type, and transmit priority degree information specifying the degree of priority to the device 200. The priority degree information is, for example, information in which AC is allocated for each pair of the information device type and the control type. Moreover, the device 200 transmits a data frame in accordance with the degree of priority specified based on the type of the device, the control type included in the data frame serving as a transmission target, and the priority degree information. In this way, in addition to the device type, the control type can be reflected on the degree of priority, so that even in a case where various kinds of the devices 200 transmit various kinds of information as well, the efficiency of the communication in the information processing system 10 can be increased. Hereinafter, an example in which both of the device type information and the control type are used will be specifically described. Note that, in the following description, either one of the device type information and the control type may be omitted.

FIG. 31 is an example of a table indicating the allocation of AC. In the method in the embodiment, AC may be determined based on the device type and the data type. The device type (Device type) herein corresponds to the abovementioned device type information, and is a device type ID, for example. Moreover, the data type (Data type) herein corresponds to data type ID mentioned above using FIGs. 28A to 29B, for example. In other word, the data type may be information indicating which one of "notification", "movement/transportation", "control", and "log" is transmitted, based on the processing result in the device 200.

For example, the server system 100 performs processing of obtaining a relationship among a device type, a data type, and AC for each of the gateways 300 that performs communication with the server system 100, and transmitting the obtained information to each of the gateways 300. For example, the server system 100 can specify, based on the information from the gateway 300, the number and types of the devices 200 to be connected to the gateway 300, a data type that can be transmitted by each of the devices 200, and the like.

For example, as mentioned above, "log" as the data type indicates a case where a log of the sensing data or a log when the serverless communication has been performed is transmitted, so that the degree of priority of the communication is low, and transmitting data at midnight and the like in which the communication amount decreases hardly causes a problem. On the other hand, "notification", "movement/transportation", and "control" are used for notifying the care giver of the occurrence of a risk, or suppressing an influence by the risk occurred. Therefore, the short time from when a risk has been detected in the device 200 to when data is transmitted is desired. Therefore, in a case where the data type is any one of "notification", "movement/transportation", and "control", the server system 100 sets the degree of priority higher than that in a case of "log". Moreover, the server system 100 may vary the degree of priority in "notification", "movement/transportation", and "control".

Moreover, the output by the swallowing and choking detection device 460 responds to the occurrence of an aspiration risk, so that there is a possibility that the degree of seriousness if a quick response is not provided becomes large. Meanwhile, a bedsore risk to be detected by the bed position detection device 470 has a high degree of importance when care assistance in a comparatively long period is assumed, so that the degree of seriousness is relatively small even if a notification or the like of the bedsore risk is executed with a delay. Therefore, even if the data type is the same "notification", the server system 100 may give a higher degree of priority in a case where the device type is a type corresponding to the swallowing and choking detection device 460 than in a case where the type is corresponding to the bed position detection device 470. In addition, the server system 100 can set the degree of priority in accordance with a device type, with respect to various kinds of the devices mentioned above using FIGs. 8 to 14 and other drawings.

For example, the server system 100 obtains a degree of priority in accordance with the device type and the data type, and allocates AC in accordance with the degree of priority. Note that, in a case where the ratio of packets to be allocated to AC with a high degree of priority becomes excessively high, collision is likely to occur, so that it is known that an effect obtained using EDCA is spoiled. Therefore, the server system 100 may change the AC allocation in accordance with the device type and the data type for each of the gateways 300. For example, such a case may occur that even for the same device type and the same data type, AC_VI with a relatively low degree of priority is allocated in the first gateway, and AC_VO with a relatively high degree of priority is allocated in the second gateway. For example, such a case can be considered that in the network configured by the first gateway, in a case where the number of the devices 200 such as the swallowing and choking detection devices 460 that detect a risk with a high degree of seriousness is large, the degree of priority of a packet, which is allocated to AC_VO in the normal network, is lowered to AC_VI.

The gateway 300 and the device 200 to be connected to the gateway 300 perform communication based on the AC allocation transmitted from the server system 100. Note that, the communication herein is not limited to data transmission from the device 200 to the gateway 300, but includes data transmission from the gateway 300 to the device 200.

Moreover, the server system 100 may perform processing of adjusting a parameter corresponding to each AC, in addition to the processing of determining the allocation of ACs based on the device type and the data type. The parameter herein includes at least one of the abovementioned CWmin, CWmax, AIFSN, and TXOP limit. In this way, it is possible to perform more detailed adjustment in the setting of the degree of priority.

Moreover, the setting processing of the degree of priority in the embodiment is not limited to that using the abovementioned EDCA. For example, RTS/CTS is used as a collision avoidance method in the wireless communication. In this method, STAs that intend to perform data transmission transmit RTS frames to AP, and AP transmits a CTS frame to any one STA to which the transmission is permitted, among the STAs having transmitted the RTS frames. The data transmission is allowed in the only STA having received the CTS frame, so that the data collision can be suppressed.

In the method in the embodiment, the server system 100 may transmit information indicating the priority when transmitting the CTS frame, as the priority degree information, to the gateway 300. For example, the server system 100 transmits information for determining the transmission order of the CTS frame such as ascending order of ADLs of target care receivers, descending order of emergency of data based on the data type, or a combination of these, to the gateway 300. In a case where the collision avoidance based on RTS/CTS is performed, the gateway 300 determines the priority based on a list of the devices 200 to which the RTS frames have been transmitted and the information from the server system 100, and transmits the CTS frame in descending order of the priority. Such a method also allows the appropriate setting of the degree of priority, so that, for example, the degree of priority only in the specific vendor can be prevented from becoming high, for example.

Moreover, in a case where the gateway 300 has received a RTS frame, the gateway 300 may perform processing of presenting a busy state, and completion expected time of the current conducting communication, to the device 200. After that, data indicating that the transmission waiting is not permitted has been transmitted from the device 200, the gateway 300 may accept the interruption by a suspend command, and permit the transmission of data from the target device 200. In a case where the data transmission has ended, the original data transmission is restarted by a resume command. In this way, it is possible to preferentially perform communication of data specially having a high emergency after suspending another data transmission. However, this processing suspends the already started data transmission, and is thus not preferable to be performed frequently or have excessively long suspended time. Therefore, data in which the interruption by the suspend command is allowable may be limited to a part of the data. For example, in a case where conditions of the small amount of transmission data and the short time required for the transmission are satisfied, the interruption may be permitted.

### 7. Application in At-Home Care

Moreover, the method in the embodiment may be applied to at-home care and the like that are performed in a home of a care receiver.

### 7.1 Example of System to Be Used at Home

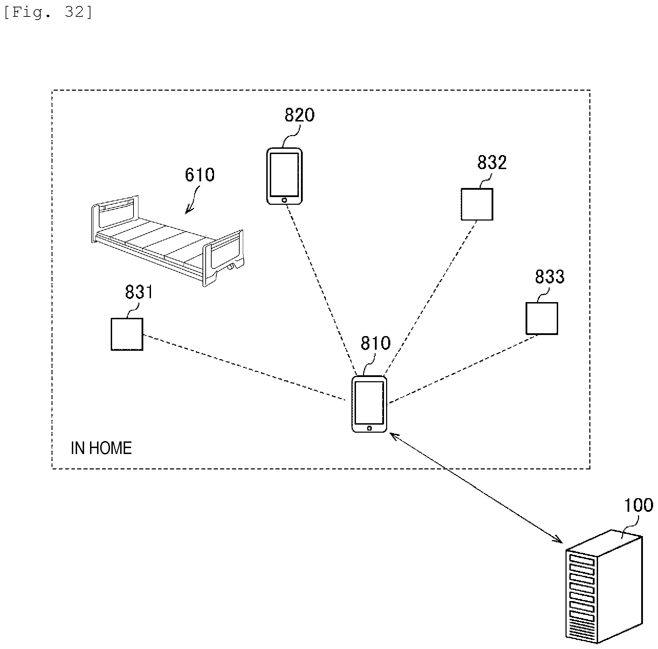

FIG. 32 is a diagram illustrating a configuration example of an at-home information processing system 10. The information processing system 10 in FIG. 32 includes the server system 100, a third terminal device 810, a fourth terminal device 820, and human-presence sensors 831 to 833. For example, in the information processing system 10, the third terminal device 810, the fourth terminal device 820, and the human-presence sensors 831 to 833 are disposed in a home where at-home care is provided. Moreover, in the at-home care, the bed 610, the wheelchair 630, which is not illustrated, and the like may be used.

The third terminal device 810 is, for example, a mobile terminal device, such as a smartphone and a tablet terminal device, that is used by a family who provides care assistance to a care receiver. The fourth terminal device 820 is, for example, a mobile terminal device, such as a smartphone and a tablet terminal device, that is used by the care receiver.

The human-presence sensors 831 to 833 are sensors that are disposed at predetermined positions within the home, and sensors that each detect a motion of a person using infrared rays, for example. Note that, the human-presence sensors 831 to 833 may be a sound sensor that reacts sound or an ultrasonic sensor that detects an article using ultrasound, and various kinds of modifications are executable in a specific mode. The human-presence sensors 831 to 833 are connected to the third terminal device 810 using BLE, for example.

The bed 610 is a bed to be used by a care receiver, and may be the care bed 520 in which an section angle, a height, and the like are adjustable.

For example, a care assistance recording device that is one example of the device 200 according to the embodiment may include the third terminal device 810 and the human-presence sensors 831 to 833 in FIG. 32. The care assistance recording device specifies a place where care assistance has been executed based on detection results by the human-presence sensors 831 to 833, and creates a care assistance record based on the specified place.

For example, each of the human-presence sensors 831 to 833 may be disposed to a place where specific care assistance is executed. For example, the human-presence sensor 831 is disposed in the vicinity the bed 610 to be used by a care receiver. The human-presence sensor 832 is disposed in the vicinity of the toilet. The human-presence sensor 833 is disposed in the vicinity of a dining table at which the meal is eaten. Moreover, the human-presence sensor may be disposed in another place in the home, such as a bathroom in which bath care assistance is provided.

For example, in a case where a motion by a person has been detected by the human-presence sensor 831, it can be considered that the care giver is performing care assistance for the care receiver, such as the bed position adjustment and the diaper changing. Moreover, in a case where a motion by a person has been detected by the human-presence sensor 832, it can be considered that the care giver is performing the excretion care assistance and the like in the toilet. Moreover, in a case where a motion by a person has been detected by the human-presence sensor 833, it can be considered that the care giver is performing the meal care assistance for the care receiver. Therefore, by obtaining the time at which the motion has been detected in each human-presence sensor, it is possible to obtain care assistance time. For example, in one day, by obtaining total time at which the motion has been detected by the human-presence sensor 831, care assistance time performed in the bed 610 in the one day can be obtained. Similarly, by the human-presence sensors 832 and 833, time of excretion care assistance in the toilet and time of the meal care assistance can be obtained. Moreover, more detailed information, such as from what time to what time and in which place the care assistance has been provided, may be obtained.

Note that, even if the human-presence sensors 831 to 833 have detected a motion by a person, in a case of the motion by a care giver alone or a care receiver alone, there is a possibility that care assistance is not executed. In the embodiment, from the viewpoint of making a simple determination, without distinguishing whether the detected motion is by one person or a plurality of persons, a determination simply based on the presence or absence of the motion detection may be executed. Alternatively, with a different method, the presence or absence of care assistance may be determined in more details. For example, instead of the human-presence sensors 831 to 833, RFID readers may be provided in the respective places. Moreover, a care giver and a care receiver carry IC tags or IC tags are incorporated into the third terminal device 810 and the fourth terminal device 820, whereby whether the care giver and the care receiver are present in the vicinity of each of the RFID readers can be determined. The care assistance recording device may determine that care assistance is being performed in a case where both of the care giver and the care receiver are present in the predetermined place. Moreover, instead of the human-presence sensors 831 to 833, cameras may be disposed in respective places in the home. For example, face recognition processing based on a taken image by each of the cameras is performed, whereby whether care assistance is being executed in each place may be determined.

Moreover, in a case where the meal is eaten on the bed, meal care assistance is provided, but there is a possibility that it is not easy to distinguish the meal care assistance from another care assistance such as the bed position adjustment, based on only a motion by a person and the presence or absence of the care assistance. In this case, the care assistance recording device may operate together with a meal amount measurement app, which is described later. For example, a photograph is taken in the meal amount measurement app, so that care assistance in the time including the timing when the photograph has been taken is determined as meal care assistance, and the other time is determined as bed care assistance other than the meal care assistance.

The care assistance recording device is used, whereby time when the care giver needs for care assistance can be easily measured. Therefore, for example, in a case where care assistance burden on a care giver becomes excessive, it is possible to notify the care giver himself/herself or a care manager of the fact, for example.

Moreover, another application may be installed on the third terminal device 810. For example, an application that determines a sleep state of a user using an acceleration sensor and a microphone that are mounted to a smartphone is known. For example, the third terminal device 810 is disposed to a pillow or the like on a bed on which a user sleeps, and determines a sleep state based on the vibration while sleeping . Moreover, the third terminal device 810 may make a determination related to snore and the like using the microphone. In this way, without using a dedicated apparatus such as the detection device 430, it is possible to easily make a determination related to the sleep.

Moreover, the abovementioned MCI determination app that makes an MCI determination may be installed on the third terminal device 810. In other words, the third terminal device 810 may function as an MCI determination device. For example, a care giver carries the third terminal device 810 that is the own smartphone to the vicinity of a care receiver, and causes the care receiver to answer, thereby making an MAC determination.

Moreover, a meal amount measurement app may be installed on the third terminal device 810. The meal amount measurement app is a method in which a photograph of dishes before meals is compared with a photograph of the dishes after meal, thereby estimating the meal amount and the calorie intake, for example. As a method of estimating the meal amount based on a photograph, publicly known methods such as JP2021-086313A are widely applicable.

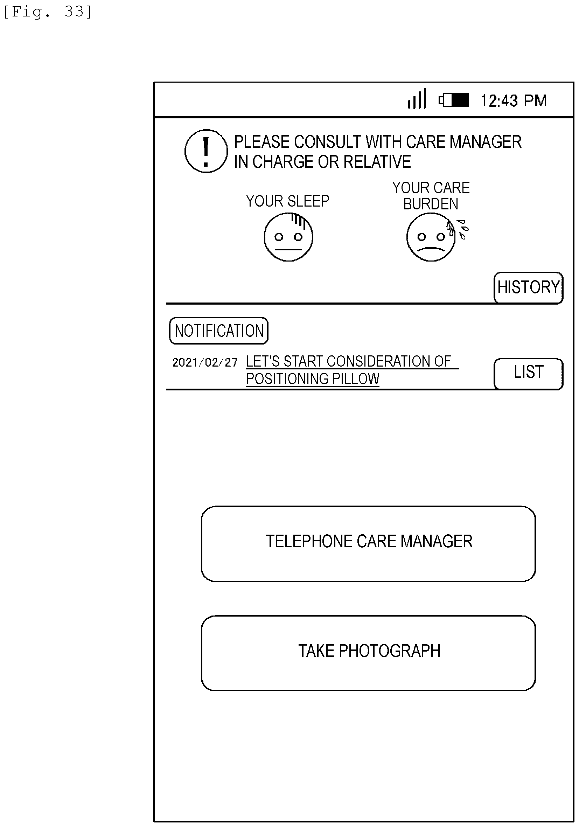

FIGs. 33 to 35 illustrate screen examples to be displayed on a display of the third terminal device 810. These screens may be, for example, user screens that are displayed in a case when an operation of logging in the information processing system 10 in the embodiment using an account of a care giver has been performed in the third terminal device 810. On the user screen, a result by the execution of the abovementioned each processing that is executed in the third terminal device 810 may be displayed. For example, the third terminal device 810 executes the abovementioned each processing, and transmits execution results to the server system 100. The server system 100 executes control of generating a display screen based on the execution results, and displaying the display screen on the display of the third terminal device 810.

FIG. 33 illustrates an example of a home screen to be displayed at the time of the login, for example. As illustrated in FIG. 33, information indicating a state of a care giver, a notification, a contact button, and a photographing button may be displayed on the home screen.

The information indicating a state of a care giver may include, for example, an icon indicating a sleep state of the care giver and an icon indicating care burden on the care giver. In the example of FIG. 33, the sleep state and the degree of care burden are presented using facial expressions of persons so as to be easily understood. Further, each information may be represented using a numerical value and the like, and various kinds of modifications are executable in a specific display mode.

For example, the third terminal device 810 may determine to which stage among a plurality of stages the care burden belongs by obtaining care assistance time that is time when care assistance has been provided based on the processing using the abovementioned human-presence sensors 831 to 833 and the like, and comparing the care assistance time with a threshold. Moreover, the third terminal device 810 may evaluate care burden based on a time-series change in the care assistance time.

Moreover, the third terminal device 810 determines a sleep state using the abovementioned acceleration sensor and microphone. For example, the third terminal device 810 may obtain sleep period per day as a sleep state. The third terminal device 810 may determine to which stage among a plurality of stages the sleep state belongs by comparing the sleep period with a threshold. Moreover, the third terminal device 810 may evaluate the sleep state based on a time-series change in the sleep period.

Moreover, in the column of the notification on the screen in FIG. 33, for example, a recommendation of a care assistance tool may be displayed. In the example of FIG. 33, text that proposes a use of a positioning pillow to be used for the adjustment of a bed position is displayed.

Moreover, the contact button is a button for telephoning a care manager, for example. For example, in a case where a selection operation of the contact button has been performed, the third terminal device 810 executes processing of activating a telephone application, and sending a call to a telephone number of a care manager registered in advance. In this manner, by employing the easy contact mode with a care manager, in a case where burden on a care giver has increased, it is possible to prompt an appropriate response. For example, as illustrated in an upper portion of FIG. 33, in a case where the sleep state or the care burden becomes worse, warning text "PLEASE TELEPHONE CARE MANAGER IN CHARGE OR RELATIVE" may be displayed. In this way, before care assistance fatigue causes a serious bad condition of a care giver, the care giver can be prompted to receive support by another person.

A photographing button is a button serving as a trigger for activating the abovementioned meal amount measurement app. For example, in a case where a selection operation of the photographing button has been performed, the third terminal device 810 performs processing of starting the meal amount measurement app. In the meal amount measurement app, a camera app is activated, dishes before meal and after meal are photographed.

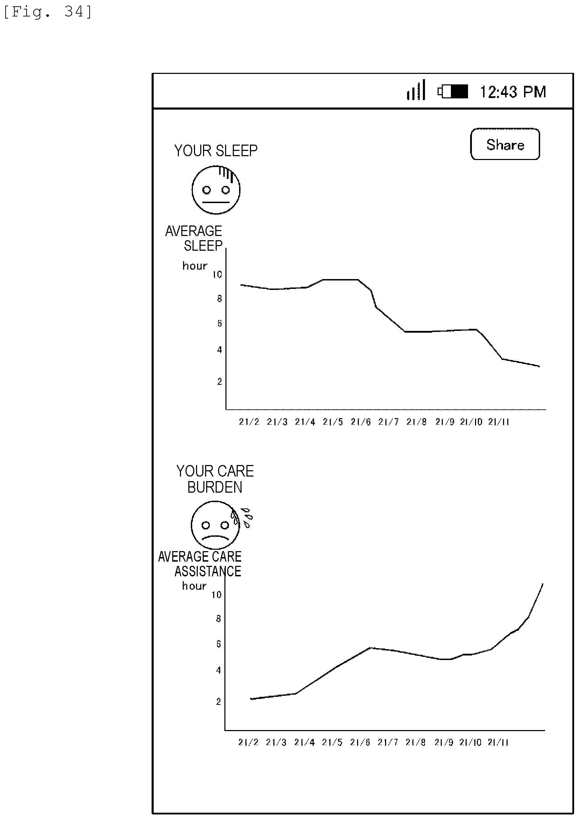

FIG. 34 illustrates an example of a history screen that is displayed in a case where a selection operation of the history button to be displayed in association with information indicating a state of a care giver in FIG. 33 has been performed. On the history screen, a time-series change in the sleep state of the care giver and a time-series change in the care burden on the care giver are displayed. For example, the processing unit 110 of the server system 100 performs processing of obtaining a moving average of sleep period per day for seven days based on information from the third terminal device 810, and displaying a time-series change in the moving average on the history screen. Note that, the calculation unit of a moving average is not limited to seven days, but another period may be used. Moreover, a time-series change in the sleep period itself per day may be displayed.

Moreover, the time-series change in the care burden is, for example, information indicating a change in an average value of the care assistance time per day. Similar to the example of the sleep state, the average value of the care assistance time may be a moving average for seven days or information to be calculated using another period. Moreover, a time-series change in the care assistance time itself per day may be displayed.

In the example of FIG. 34, the sleep period decreases as the time elapses, and the care assistance time increases as the time elapses. The screen illustrated in FIG. 34 is displayed, whereby a change for the worse in the state of the care giver can be presented so as to be easily understood. Moreover, as illustrated in FIG. 34, the history screen may include a share button for sharing information related to a state of a care giver. In a case where a selection operation of the share button has been performed, information including a time-series change in the average sleep period, a time-series change in the average care assistance time, and the like is transmitted to a contact address registered in advance. The contact address herein may be a care manager, a family of the care giver, and the like. Moreover, a share destination of the information related to a state of the care giver is not limited to the contact address registered in advance. For example, in a case where an operation of selecting the share button has been performed, link information to the information related to a state of the care giver may be displayed. The link information herein may be, for example, address information on a Webpage on which the information related to a state of the care giver is displayed. The address information may be Uniform Resource Locator (URL), or may be a two-dimensional barcode that indicates the URL. For example, when a care giver receives a medical examination in a hospital due to poor health, the information related to a state of the care giver is shared with a medical doctor via the link information, whereby it is possible to provide information for making a decision of a diagnosis of the poor health to the medical doctor.

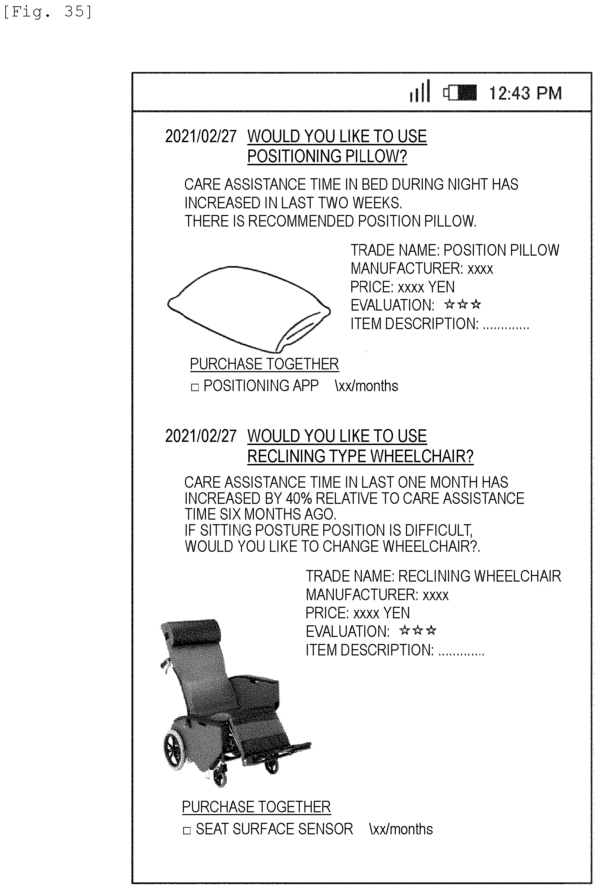

FIG. 35 illustrates an example of a list screen to be displayed in a case where a selection operation of a list button that is displayed associated with "NOTIFICATION" in FIG. 33 has been performed. On the list screen, information such as the recommendation having transmitted to the target care giver is list-displayed in chronological order. In the example of FIG. 35, a recommendation of a positioning pillow and a recommendation of a reclining wheelchair are displayed. Each of the recommendations includes the reason why the item is recommended, an item image, and details information on the item. The details information on the item may include a trade name, a manufacturer, a price, an evaluation, an item explanation, and the like.

Moreover, in the recommendation, a vendor app available with the item and the device 200 according to the embodiment may be presented. In the example of FIG. 35, with the positioning pillow, an introduction of a positioning app is proposed. The positioning app is, for example, an application that executes processing similar to that by the first terminal device 471 of the bed position detection device 470 mentioned above using FIG. 13. The introduction of the positioning app makes it possible to support the bed position adjustment by the care giver. Similarly, a diaper changing support app may be installed on the third terminal device 810. For example, the third terminal device 810 may execute processing similar to that by the second terminal device 472 of the bed position detection device 470 mentioned above using FIG. 13, for example.

Moreover, in the example illustrated in FIG. 35, with the reclining wheelchair 510, an introduction of the seat surface sensor 440 is proposed. The introduction of the seat surface sensor 440 allows a result of the forward displacement/lateral displacement determination and a determination result of a fall possibility to be presented to the care giver, so that it is possible to support the position adjustment in the reclining wheelchair 510.

Moreover, the processing to be executed in the third terminal device 810 is not limited to the above. For example, timing of a meal can be estimated based on the meal amount measurement app, and timing when excretion care assistance has been provided can be estimated based on the processing by the care assistance recording device. Therefore, the third terminal device 810 may perform processing of predicting fecal incontinence based on rhythms of the meal and the excretion. For example, the third terminal device 810 may predict fecal incontinence, based on a trained model or the like that determines the presence or absence of fecal incontinence after predetermined time has elapsed using the type and amount of a cathartic, the type of a diaper, the meal amount, the moisture intake amount, and intake timing of the meal and the moisture as input data. Moreover, as the recommendation, the type of a diaper and the like may be recommended.

Moreover, in the fourth terminal device 820 that is used by a care giver as well, various kinds of processing are executed. For example, the fourth terminal device 820 includes a GPS sensor, and a watching application that uses the GPS sensor may be installed therein. Conventionally, methods of tracking a position of an elderly person and a dementia patient for the purpose of wandering control and the like are known, and are widely applicable in the embodiment.

Moreover, similar to the third terminal device 810, an application that determines a sleep state may be installed on the fourth terminal device 820. The fourth terminal device 820 determines a sleep state of a care receiver. Moreover, similar to the third terminal device 810, an MCI determination app that makes the abovementioned MCI determination may be installed on the fourth terminal device 820.

### 7.2 Cooperation With Care Facility And The Like

Moreover, the information processing system 10 that is used in the at-home care has been exemplified in the foregoing, but the system may cooperate with a system out of the home. For example, information acquired in the at-home care may be transmitted to a terminal device such as a PC that is used by a care manager. For example, processing results in the third terminal device 810 and the fourth terminal device 820 are transmitted to the server system 100, and the server system 100 transmits information based on the acquired processing results to the terminal device of the care manager.

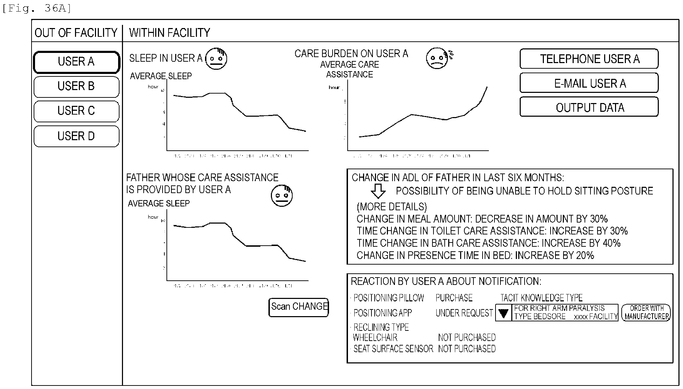

FIGs. 36A to 36C illustrates screen example to be displayed on the terminal device of the care manager. FIG. 36A illustrates an example of a home screen including two tabs of within the facility and out of the facility in the upper portion. The within-facility tab is a tab for displaying information related to a care receiver who occupies the care facility or the like, among care receivers who are taken charge by the care manager. For example, in a case where the within-facility tab has been selected, processing of activating care software introduced into the target facility may be separately performed. The out-of-facility tab is a tab for displaying information related to a care receiver to whom the at-home care is provided, and a care giver who provides care assistance to the care receiver. FIG. 36A illustrates a screen example of a state where the out-of-facility tab is selected.

The screen illustrated in FIG. 36A includes a region in which users are list-displayed, and a region in which detailed information on the user selected in the region is displayed. The user herein indicates a family of the care receiver to whom at-home care is executed. In FIG. 36A, four persons of users A to D are list-displayed, and the user A among them is in a selected state. Note that, the plurality of users to be displayed herein may be sorted in descending order of the degree of importance for the care manager. For example, the plurality of users are sorted in descending order of the degree of a change for the worse of the sleep state and the degree of a change for the worse of the care burden in the user who is a care giver. In this way, in a case where the care manager is in charge of a large number of users, it is possible to present to which user the care manager needs to pay attention so as to be easily understood. Note that, the sort order is not limited thereto, but a newly contact user may be put in the higher level, or a user who has introduced the new device 200 may be put in the higher level, and various kinds of modifications are executable for the specific processing.

In the region in which detailed information is displayed, a sleep state of a user who is a care giver, care burden, and a sleep state of a care receiver are displayed. The information may include, for example, similar to FIG. 34, a graph illustrating a time-series change in the average value, an icon indicating the stage, and the like.

Moreover, in association with the sleep state and the care burden of the user A, a contact button with the user may be displayed. In FIG. 36A, a button for telephoning the user and sending a mail to the user are displayed. In a case where a selection operation of the telephone button has been performed, a telephone call app is activated, call to a telephone number of the user registered in advance is performed. In a case where a selection operation of the mail button has been performed, a mail app is activated, and a mail creation screen in which a mail address of the user is already written is displayed. Moreover, the method in which the care manager makes contact with the user is not limited to the telephone or the mail, but a chatting app and the like may be used. For example, on the screen in FIG. 36A, a talk history between the care manager and the user may be displayed.

Moreover, as illustrated in FIG. 36A, a data output button may be displayed in the region in which the detailed information is displayed. In a case where a selection operation of the data output button has been performed, data related to the sleep state, the care burden, and the like of the user is output. The data herein may be a csv file, or may be data in another format.

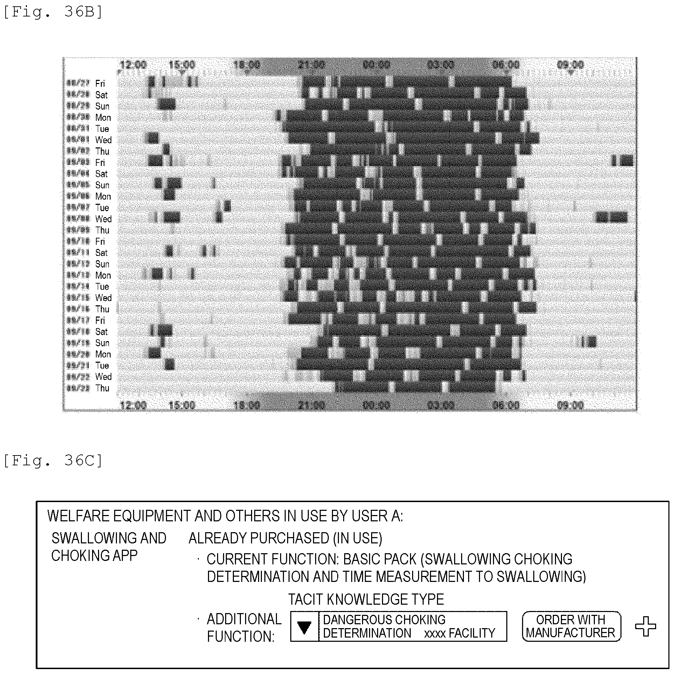

Moreover, a change button in association with the sleep state of the care receiver may be displayed. FIG. 36B illustrates an example of information to be displayed in a case where a selection operation of the change button has been performed. For example, in a case where the change button is selected, instead of the change graph of the average sleep period illustrated in FIG. 36A, information illustrated in FIG. 36B may be displayed. FIG. 36B illustrates a screen on which changes in the awake state, the sleep state, and the bed departure state of the care receiver per day are collectively displayed for a predetermined number of days. The screen illustrated in FIG. 36B is displayed, whereby not only the average sleep period but also more detailed information related to the sleep can be presented. For example, it is possible to present information related to the quality of sleep such as the presence or absence and the number of times of interrupting arousal, and the like, to the care manager.

For example, the screen illustrated in FIG. 36B may be similar to the screen that is used for displaying the sensing data of the detection device 430. In this way, even in a case where the detection device 430 is not introduced, it is possible to perform the display similar to that in a case where the detection device 430 is introduced.

Moreover, as illustrated in FIG. 36A, the region in which the detailed information is displayed may include information related to the ability information on the care receiver. In the example of FIG. 36A, an estimation result of the ADL change is displayed based on a change in each of the meal amount, the time of toilet care assistance, the time of bath care assistance, and the bed presence time. For example, as illustrated in FIG. 36A, based on a decrease in the meal amount and an increase in the care assistance time in the respective places, the lowering of the sitting posture holding ability may be displayed. In this way, in the case where the at-home care is provided as well, it becomes possible to estimate a change in the ability information, and notify the care manager and the care giver of the change.

Note that, in the care facility and the like, various kinds of the devices 200 mentioned above using FIGs. 8 to 14 and other drawings are comparatively easily introduced, and various kinds of data can be acquired as sensing data. For example, as sensing data, data for obtaining the content of the movement starting motion, the sitting posture holding ability, the swallowing ability, the amount of activity on the bed, and the like can be acquired, so that it is possible to increase the estimation accuracy of the ability information. On the other hand, in the at-home care, introducing the devices 200 equivalent to those in the care facility is not easy. Therefore, information that can be acquired is limited information such as the meal amount and the care assistance time, as mentioned above.

Therefore, in the embodiment, information to be acquired in the care facility is associated with information to be acquired in the at-home care, whereby the estimation accuracy of the ability information in the at-home care may be increased. For example, a target is a care receiver who usually receives at-home care and periodically uses day care. A value of ability information estimated using the device 200 in the care facility at the day care has a high reliability as ability information at the timing. Moreover, it is difficult to think a sudden change in the ability information without factors such as development symptoms of the illness and an accident for a short time, so that it can be considered that the ability information before and after the day care in a predetermined period is equivalent to the ability information at the day care. For example, machine learning that uses the meal amount and the care assistance time acquired in the at-home care before and after the day care in a predetermined period as input data, and data to which the ability information estimated relative to the input data at the day care is assigned as correct data, as training data. In this way, it is possible to estimate ability information with accuracy based on the information that can be acquired at the at-home care.

Moreover, ability information may be estimated based on overlapping contents between the at-home care and the day care. For example, it is assumed that the respective devices 200 mentioned above using FIGs. 8 to 14 are introduced into the care facility, and the ability information acquisition unit 111 of the server system 100 obtains ability information based on sensing data from each of the devices 200. For example, the server system 100 may store table data in which data of a sensing data group to be acquired from the respective devices 200 in association with the ability information is used as one record.

Moreover, in the at-home care, it may be not easy to introduce all the devices 200 in FIGs. 8 to 14, but there is a possibility that a part of the devices 200 is introduced. For example, the detection device 430 is introduced into the home, and sensing data corresponding to the sleep, the amount of activity, and the like of a care receiver is obtained. In this case, the server system 100 may perform processing of extracting a record having a high similarity from the abovementioned table data, by comparing the sensing data acquired in the at-home care with the sensing data group included in the abovementioned table data. For example, the sensing data group includes sensing data by the detection device 430, so that the similarity is calculated based on the comparison processing between the sensing data and sensing data acquired in the at-home care. Moreover, the server system 100 outputs ability information included in a record having a high similarity as ability information on a care receiver who receives at-home care. In this way, it is also possible to estimate ability information with accuracy based on the information that can be acquired at the at-home care.

Referring back to FIG. 36A, the description is continued. On the home screen illustrated in FIG. 36A, information related to a reaction to a "notification" presented to a target user, by the user, may be displayed. The notification herein is information illustrated in FIG. 35, for example.

For example, on the screen illustrated in FIG. 36A, whether a user has purchased the device 200 recommended to the user is displayed. In the example of FIG. 36A, the user has an intention of purchasing a positioning pillow, and a recommended accompanying positioning app. For example, when a selection operation of the manufacturer order button illustrated in FIG. 36A is performed, the care manager instead of the user may perform order processing of the positioning pillow.

Note that, as mentioned above, various kinds of applications (vendor apps) can be installed on one device 200, but there is a possibility that a user who is not an expert of care assistance is not easy to determine which application is suitable for care assistance for the care receiver. Therefore, as illustrated in FIG. 36A, the care manager who has knowledge related to the care assistance may determine an application to be installed. The determination of an application corresponds to the determination of tacit knowledge to be used, as mentioned above. In the example of FIG. 36A, among some candidates, tacit knowledge suitable when a bedsore risk is suppressed for a care receiver having a paralysis on his/her right arm as a target is selected. Moreover, tacit knowledge may be created for each care facility, and a facility that has created the tacit knowledge may be selectable by the care manager on the screen illustrated in FIG. 36A. For example, in a case where the selected application is installed on the device 200 to be purchased, based on the selection operation of the manufacturer order button, an item in a state where the target application is preinstalled may be sent.

Moreover, such a case can be also considered that the device 200 (for example, the positioning pillow) to be purchased is different from the device 200 (for example, the third terminal device 810) to which the application is installed. In this case, using the selection operation or the like of the manufacturer order button as a trigger, information specifying the user and the application may be transmitted to the server system 100. The server system 100 executes processing of transmitting the specified application to the device 200 that is used by the target user. In this way, without the procedures by the user himself/herself, the suitable application can be installed on the device 200. Note that, the application selection when the device is purchased has been exemplified herein, but the screen in FIG. 36A may be used for an application change and the like in the middle of use. For example, the device 200 and an application in use by a target user are displayed on a screen on which a care manager browses, and the care manager changes an application to be used in accordance with a situation of a care receiver and the like. For example, as is the example of FIG. 36A, an application (tacit knowledge) to be used may be selected from a pull-down menu. In this case as well, similarly, for example, via the server system 100, the uninstallation and the installation of an application are executed.

FIG. 36C illustrates an example of information that is additionally displayed ed on the screen in FIG. 36A. As illustrated in FIG. 36C, on a screen displayed in a terminal device of a care manager, the device 200 and an application that are used by a target user may be displayed. In the example of FIG. 36C, the user already introduces the swallowing and choking detection device 460, and is using a normal function as a swallowing and choking app. The normal function is a function of performing the detection of swallowing and choking, and the measurement of swallowing time. The installation of an additional function with respect to this function may be executable by the care manager. For example, as illustrated in FIG. 36C, the care manager may add a function of detecting the presence or absence of choking with a high degree of risk. This allows the care manager to add an option function.

As mentioned above, in the method in the embodiment, an operation mode of each of the devices 200 is switchable based on ability information and the like, and specifically, an active/inactive state of each application can be changed. Therefore, in the at-home care as well, the determination as to whether an active/inactive state may be automatically performed. For example, the server system 100 may estimate ability information from sensing data to be acquired in the at-home care, and determine an operation mode of the device 200 based on the ability information. Further, the embodiment is not limited thereto, but an operation of activating the application may be possible on the screen in FIG. 36C and the like. In this way, the care manager can manually change the operation mode in accordance with a situation. As a result, in the at-home care with a small number of types of sensing data, it is possible to appropriately change the operation mode in accordance with a situation.

Although the present embodiment has been described in detail as described above, it will be readily understood by those skilled in the art that many modifications can be made without departing from the novel matters and effects of the present embodiment. Therefore, all such the modifications are intended to fall within the scope of the present disclosure. For example, a term described at least once together with a different term having a broader meaning or the same meaning in the specification or the drawings can be replaced with the different term in any part of the specification or the drawings. Moreover, all the combinations of the present embodiments and the modifications fall within the scope of the present disclosure. Further, the configuration and operation of the server system, the device, the information processing system, and the like are also not limited to those described in the present embodiment, and various modifications can be made.

## Claims

1. An information processing system comprising:
a device including an application that executes processing corresponding to tacit knowledge of a skilled worker; and
a server system that transmits a data frame including a media access control (MAC) header, a frame body, and a trailer, in a data link layer of communication with the device, wherein
the server system transmits the data frame in which the frame body includes a fixed-length region including a first region that stores ability information indicating an activity ability of a care receiver, a second region that stores scene information specifying a care assistance scene with respect to the care receiver, and a third region that stores device type information specifying a type of a combined device to be used with the device, and
the device determines whether the application is active or inactive based on at least one of the ability information, the scene information, and the device type information.

2. The information processing system according to claim 1, wherein the device transmits, when the application has been set active, an operation result of the application to the server system.

3. The information processing system according to claim 2, wherein
the server system
obtains, based on the operation result, a control type and a control content of control with respect to a control target device, and
transmits the data frame in which the frame body includes a fixed-length fourth region that stores the control type, and a fifth region that stores the control content and has a different length in accordance with the control type in a rear portion from the region including the first region, the second region, and the third region, to the control target device.

4. The information processing system according to claim 3, wherein
the device selects whether to
transmit the operation result to the server system, or
transmit, based on the operation result, information for controlling the control target device, without passing through the server system, to the control target device.

5. The information processing system according to claim 4, wherein
the device transmits, when controlling the control target device without passing through the server system, in the data link layer of the communication with the control target device, the data frame in which the frame body includes a fixed-length region including the second region and the third region, to the control target device.

6. The information processing system according to claim 5, wherein
the device
obtains, based on the operation result, the control type and the control content of control with respect to a control target device, and
transmits the data frame in which the frame body includes the fourth region and the fifth region in a rear portion from a region including the second region and the third region, without passing through the server system, to the control target device.

7. The information processing system according to claim 5 or 6, wherein the device transmits log data related to the control with respect to the control target device, to the server system.

8. The information processing system according to any one of claims 4 to 6, further comprising:
an entry/exit management apparatus that detects coming and going of the device into and out of a given space; and
a gateway that relays communication between the device positioned within the space and the server system, wherein
the entry/exit management apparatus acquires, when having detected that a new device enters the space, by performing communication with the new device, address information on the new device that is used in communication via the gateway, and notifies the device positioned within the space of the acquired address information.

9. The information processing system according to claim 8, wherein the device controls the control target device, when the address information on the control target device has been acquired based on information from the entry/exit management apparatus, without passing through the server system.

10. The information processing system according to any one of claims 4 to 6, wherein,
the device
acquires address information on another device positioned within a predetermined distance from the device, from the server system, and
controls the control target device, when the address information on the control target device has been acquired, without passing through the server system.

11. The information processing system according to any one of claims 1 to 6, wherein,
the server system obtains a degree of priority based on the device type information, and transmits priority degree information specifying the degree of priority to the device, and
the device transmits the data frame in accordance with the degree of priority specified based on a type of the device and the priority degree information.

12. The information processing system according to claim 3, wherein
the server system obtains a degree of priority based on the device type information and the control type, and transmits priority degree information specifying the degree of priority to the device, and
the device transmits the data frame in accordance with the degree of priority specified based on the type of the device, the control type included in the data frame serving as a transmission target, and the priority degree information.

13. An information processing apparatus comprising:
a communicator that transmits a data frame including a media access control (MAC) header, a frame body, and a trailer, in a data link layer of communication with a device including an application that executes processing corresponding to tacit knowledge of a skilled worker; and
a communication processing unit that controls the communicator, wherein
the communicator transmits the data frame in which the frame body includes a fixed-length region including a first region that stores ability information indicating an activity ability of a care receiver, a second region that stores scene information specifying a care assistance scene with respect to the care receiver, and a third region that stores device type information specifying a type of a combined device to be used with the device, as information for determining whether the application is active or inactive, to the device.

14. An information processing method in an information processing system that includes a device including an application that executes processing corresponding to tacit knowledge of a skilled worker and a server system that transmits a data frame including a media access control (MAC) header, a frame body, and a trailer, in a data link layer of communication with the device, the information processing method comprising:
transmitting the data frame in which the frame body includes a fixed-length region including a first region that stores ability information indicating an activity ability of a care receiver, a second region that stores scene information specifying a care assistance scene with respect to the care receiver, and a third region that stores device type information specifying a type of a combined device to be used with the device, from the server system to the device; and
determining whether the application is active or inactive based on at least one of the ability information, the scene information, and the device type information.
